# EUROPEAN PATENT SPECIFICATION

(11) **EP 2 773 205 B1**
(45) Date of publication and mention of the grant of the patent: **22.05.2024**
(21) Application number: 12844767.9
(22) Date of filing: 02.11.2012
(51) Int. Cl.: A61K 31/437, A61K 31/545, A61P 1/08, A61P 1/10, A61P 1/14, A61P 7/00, A61P 11/00, A61P 13/02, A61P 29/00, A61P 31/04, A61P 39/02, A61P 43/00, A61P 1/00, A61P 13/12

(54) **RIFAXIMIN FOR RETREATING DIARRHEA-PREDOMINANT IRRITABLE BOWEL SYNDROME**
RIFAXIMIN ZUR BEHANDLUNG VON DURCHFALL-REIZDARMSYNDROM
RIFAXIMINE POUR LE TRAITEMENT DU SYNDROME DU CÔLON IRRITABLE AVEC DIARRHÉE

(30) Priority: 02.11.2011 US 201161554662 P; 14.11.2011 US 201161559686 P; 15.11.2011 US 201161560273 P; 15.11.2011 US 201161560128 P; 15.11.2011 US 201161560133 P; 15.11.2011 US 201161560267 P; 16.11.2011 US 201161560788 P; 22.11.2011 US 201161563033 P; 28.11.2011 US 201161564270 P; 18.02.2012 US 201261600635 P
(43) Date of publication of application: 10.09.2014
(73) Proprietor: Salix Pharmaceuticals, Inc., Raleigh, NC 27615 (US)
(72) Inventor: BORTEY, Enoch, Chapel Hill, NC 27517 (US); FORBES, William, Raleigh, NC 27614 (US); GOLDEN, Pam, Durham, NC 27713 (US); MERCHANT, Kunal, Durham, NC 27705 (US)
(74) Representative: Kraus & Lederer PartGmbB
(86) International application number: PCT/US2012/063380
(87) International publication number: WO 2013/067394

(56) References cited:
- WO-A1-2011/103246
- WO-A2-2011/066458
- US-A1- 2009 305 993
- US-A1- 2011 065 741
- US-A1- 2011 118 295
- US-A1- 2011 118 295
- US-A1- 2011 243 879
- PIMENTEL MARK ET AL: "Rifaximin Therapy for Patients with Irritable Bowel Syndrome without Constipation.", NEW ENGLAND JOURNAL OF MEDICINE, vol. 364, no. 1, January 2011 (2011-01), pages 22-32, XP002735664, ISSN: 0028-4793
- YANG ET AL.: 'Rifaximin versus Other Antibiotics in the Primary Treatment and Retreatment of Bacterial Overgrowth in IBS.' DIGESTIVE DISEASES AND SCIENCES vol. 53, 23 May 2007, pages 169 - 174, XP019550554

## Description

### BACKGROUND

Rifaximin (INN; see The Merck Index, XIII Ed., 8304) is an antibiotic belonging to the rifamycin class of antibiotics, *e*.*g*., a pyrido-imidazo rifamycin. Rifaximin exerts its broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency. It has been reported that rifaximin is characterized by a negligible systemic absorption, due to its chemical and physical characteristics (Descombe J.J. et al. "Pharmacokinetic study of rifaximin after oral administration in healthy volunteers." Int J Clin Pharmacol Res, 14 (2), 51-56, (1994)).

US 2011/065741 discloses a method of treating irritable bowel syndrome comprising administering 550 mg of rifaximin TID to a subject in need thereof.

US 2011/0118295 discloses a method of treating bowel disease (BD) with a durability of antibiotic response, comprising administering a therapeutically effective amount of a rifamycin class antibiotic to a subject in need thereof, selecting subjects who respond to treatment after being treated for between about 1 and about 24 weeks; and removing a responding subject from treatment wherein after removal of treatment there is a durability of response.

### SUMMARY

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy (or for diagnosis).

The invention relates to rifaximin for use in retreating a subject having diarrhea-predominant IBS (d-IBS), being a responder to an initial treatment of d-IBS with rifaximin and having a recurrence of abdominal pain or stool consistency for at least 3 weeks during a 4-week assessment period, comprising
- an initial treatment phase comprising a 2 weeks administration of 550 mg of rifaximin three times daily (TID) and a 2 weeks of treatment-free follow-up,
- a first treatment-free maintenance phase of up to 8 to 12 weeks,
- a first repeat treatment phase comprising a 2 weeks administration of 550 mg of rifaximin TID and a 2 weeks of treatment-free follow-up,
- a second treatment-free maintenance phase of up to 8 weeks,
- a second repeat treatment phase comprising a 2 weeks administration of 550 mg of rifaximin TID and a 2 weeks treatment-free follow-up.

In some embodiments, treating IBS comprises improving IBS-related abdominal pain and stool consistency. In some embodiments, treating IBS comprises improving IBS-related abdominal pain. In some embodiments, treating IBS comprises improving stool consistency. In some embodiments, treating IBS comprises improving IBS-related abdominal pain and stool consistency and having at least a 1 point improvement in weekly average daily IBS symptoms. In some embodiments, treating one or more IBS symptoms is a reduction from baseline symptoms. In some embodiments, the baseline symptoms are established prior to treatment.

In some embodiments, the subject is considered a responder to a repeat treatment if the subject a subject has less IBS-related abdominal pain and better stool consistency after administration of rifaximin. In some embodiments, the subject is considered a responder to a repeat treatment if the subject has less IBS-related abdominal pain after administration of rifaximin. In some embodiments, the subject is considered a responder to a repeat treatment if the subject has better stool consistency after administration of rifaximin. In some embodiments, the subject is considered a responder to a repeat treatment if the subject has less IBS-related bloating after administration of rifaximin. In some embodiments, the response of the responder comprises at least 1 point improvement in weekly average daily IBS symptoms compared to baseline. In some embodiments, the response of the responder comprises a decrease in bloating compared to baseline. In some embodiments, the responder comprises a subject who demonstrates at least 2 weeks of improvement in a 4 week treatment free follow up period in both primary symptoms of IBS.

In some embodiments, the primary symptoms of IBS comprise abdominal pain and stool consistency.

The subject has met recurrence criteria when they experience the recurrence of abdominal pain or stool consistency for at least 3 weeks during a 4-week follow-up period.

The subject has met recurrence criteria when they experience the recurrence of abdominal pain for at least 3 weeks during a 4-week follow-up period.

The subject has met recurrence criteria when they experience the recurrence of stool consistency for at least 3 weeks during a 4-week follow-up period.

In some embodiments, relapse comprises an absence of treatment success for abdominal pain for at least three out of four consecutive weeks or a loss of stool consistency for at least three out of four consecutive weeks.

The use comprises selecting a subject that has relapsed a second time after a second 14 day treatment with rifaximin and administering 550mg rifaximin BID for 14 days.

In some embodiments, the response of the responder comprises at least 1 point improvement in weekly average daily IBS symptoms compared to baseline. In some embodiments, the response of the responder comprises a decrease in bloating compared to baseline. In some embodiments, the responder comprises a subject who demonstrates at least 2 weeks of improvement in a 4 week treatment free follow up period in both primary symptoms of IBS.

In some embodiments, the primary symptoms of IBS comprise abdominal pain and stool consistency.

The subject has met recurrence criteria when they experience the recurrence of abdominal pain or stool consistency for at least 3 weeks during a 4-week follow-up period.

Relapse includes one or more of absence of treatment success for abdominal pain for at least three out of four consecutive weeks and a loss of stool consistency for at least three out of four consecutive weeks.

In some embodiments, IBS-D symptoms comprise one or more of overall IBS-related abdominal pain and stool consistency.

In some embodiments, adequate relief of IBS-D symptoms comprises an improvement in overall IBS-related abdominal pain and stool consistency.

In some embodiments, the adequate relief comprises an improvement in overall IBS-related abdominal pain and stool consistency with at least a 1 point improvement in weekly average daily IBS symptoms as compared to baseline.

In some embodiments, the adequate relief comprises an improvement in bloating.

In some embodiments, baseline symptoms are established prior to treatment.

In some embodiments, adequate relief of bloating symptoms comprises a 'yes' response from a subject when asked the question comprising or similar to "Have you had adequate relief of your IBS-D symptom of bloating over the last 24 hours?" In some embodiments, adequate relief of IBS-D symptoms comprises an affirmative response (*e*.*g*., yes) from a subject if asked whether they have had adequate relief of bloating over the last 24 hours.

In some embodiments, adequate relief of IBS-related abdominal pain comprises a 'yes' response from a subject when asked the question comprising or similar to "Have you had adequate relief of your IBS-related abdominal pain over the last 24 hours?" In one embodiment, adequate relief of IBS-D symptoms comprises an affirmative response (e.g., yes) from a subject if asked whether they have had adequate relief of bloating over the last 24 hours.

In some embodiments, adequate relief of IBS-related abdominal pain comprises an improvement in overall IBS-related abdominal pain and stool consistency with at least a 1 point improvement in weekly average daily IBS symptoms as compared to baseline.

In some embodiments, In one embodiment, bloating symptoms comprise one or more of the symptoms of abdominal fullness, bloating, gas, or swelling.

Described is a method comprising determining, based on clinical data, whether a subject will have a positive response to treatment. The determination may be made based on one or more of a subject's age, a subject's duration of BD, gender, or baseline severity of IBS-D. In some embodiments, the clinical data is presented in a label on a pharmaceutical product.

Described is a method of identifying or defining a subject as a responder to rifaximin treatment for IBS, wherein the method includes identifying a subject that has a positive response during at least 2 out of 4 weeks of rifaximin treatment for IBS based on daily questions for the weekly responses for both abdominal pain and stool consistency, thereby identifying or defining a responder.

The subject may have a decrease in weekly average abdominal pain score. In some embodiments, the decrease in pain score is 30% or greater. In some embodiments, the subject has experienced a reduction in the number of days per week with at least 1 stool with a consistency of greater than or equal to 6 according to per the Bristol stool scale.

Certain aspects are directed to a method for treating bacterial dysbiosis, wherein the method includes administering to the subject an effective amount of rifaximin to treat bacterial dysbiosis, thereby treating bacterial dysbiosis. In some embodiments, the subject has previously been administered rifaximin for treatment of IBS.

In some aspects, the method further includes administering to the subject 550 mg of rifaximin TID. In some embodiments, the subject is administered rifaximin for 14 days.

In some aspects, the subject having IBS or bacterial dysbiosis has been identified using a lactose breath test or a glucose breath test.

In some aspects, treatment with rifaximin results in the acute treatment of symptomatic recurrence of irritable bowel syndrome with diarrhea (IBS-D).

In some aspects, described herein are methods for treating IBS by administering rifaximin, wherein the administration of rifaximin results in alteration of the gut flora.

In some embodiments, the response rate to treatment comprises greater than 50, 55, 60, 65, 70, 75, 80, 85, or 90% of subjects administered rifaximin. In some embodiments, the response rate of relapsed responder comprises between about 30 and 90% of subjects.

In some aspects and embodiments, the methods are provided or other methods further include characterizing the stool of a subject at one or more of the following timepoints: prior to administration of rifaximin, during administration of rifaximin, after administration of rifaximin, prior to administration of rifaximin after relapse has been identified, during administration of rifaximin after relapse has been identified, after administration of rifaximin after relapse has been identified.

In some embodiments, the characterization of stool includes characterizing the stool flora. In some embodiments, the characterization of the stool flora includes analyzing 16sRNA. In some embodiments, the characterization of the stool flora includes culturing the flora.

In some embodiments, the use further includes administering a proton pump inhibitor (PPI) to the subject.

In some embodiments, the subject has been identified with one or more of the Rome II or the Rome III criteria.

A diagnosing step includes one or more of: measuring HPA axis, immune activation markers, or fecal biomarkers, culturing of jejunal contents and identifying small intestinal bacterial overgrowth (SIBO). In some embodiments, the immune activation markers include one or more of cytokines, mucosal lymphocytes, mucosal mast cells or proteases. In some embodiments, the fecal biomarkers include one or more of calprotectin, human β-defensin, or fecal proteases.

The measurement of SIBO includes aspiration and direct culture of jejunal contents, and/or breath testing. In some embodiments, the breath testing includes a breath test, e.g., lactulose hydrogen breath testing and glucose breath testing.

In some embodiments, the subject who is a responder is likely to have one or more of the following predictors of response, abdominal pain ≥ 2.5; bloating ≥ 2.5; average stool consistency score ≥ 3.5; or bothersome urgency, wherein bothersome urgency is defined as ≥ 3.5 days with urgency.

In some embodiments, relapse in a responder includes one or more of: a change in stool consistency, a change in abdominal pain or a change in stool consistency and a change in abdominal pain. In some embodiments, the change in abdominal pain includes increased pain.

Embodiments, not forming part of the present invention, also relate to any of the foregoing methods, wherein the methods further include testing a subject for *C*. *difficile.* In some embodiments, not forming par of the present invention, a subject is identified as having a *C*. *difficile* infection. In some embodiments, not forming part of the present invention, the subject is selected for treatment based on having a *C*. *difficile* infection. In some embodiments, not forming part of the present invention, the subj ect is tested for the presence of a *C.difficile* toxin or for the presence of *C*. *difficile* virulence or resistance mutations.

Further described, but not forming part of the present invention, are methods of treating a subject for *C*. *difficile* infection. In some embodiments, not forming part of the present invention, a subject is identified as having a *C*. *difficile* infection. In some aspects, not forming part of the present invention, the subject is selected for treatment based on having a *C*. *difficile* infection. In some aspects, not forming part of the present invention, the subject is tested for the presence of a *C.difficile* toxin or for the presence of *C*. *difficile* virulence or resistance mutations.

In some embodiments, baseline symptoms are established prior to treatment.

In some embodiments, the subject being treated is white.

In some embodiments, the at least 25% decrease in IBS-related abdominal pain and a stool consistency score of <4 is at a time point of 1 month after the treatment with rifaximin.

In some embodiments, the at least 25% decrease in IBS-related abdominal pain and a stool consistency score of <4 is at a time point of 2 months after the treatment.

In some embodiments, the at least 25% decrease in IBS-related abdominal pain and a stool consistency score of <4 is at a time point of 3 months after the treatment.

In some embodiments, the method further includes determining the gender of a subject and administering the therapeutically effective amount of rifaximin to a female subj ect.

In some embodiments, the method includes administering 550 mg of rifaximin TID to the subject for 14 days.

In some embodiments, administration of 550 mg rifaximin TID results in at least 25% of subjects treated with rifaximin having at least a 30% decrease in IBS-related pain, a stool consistency score of <4 and at least a 1 point decrease in average daily IBS score.

In some embodiments, administration of 550 mg rifaximin TID results in at least 30% of subjects treated with rifaximin having at least a 30% decrease in IBS-related pain, a stool consistency score of <4 and at least a 1 point decrease in average daily IBS score.

In some embodiments, administration of 550 mg rifaximin TID results in at least 35% of subjects treated with rifaximin having at least a 30% decrease in IBS-related pain, a stool consistency score of <4 and at least a 1 point decrease in average daily IBS score.

Described, but not forming part of the present invention, is also a method of reducing the risk of developing an infection in a subject having HE, the method including administering to the subject an effective amount of rifaximin, wherein administration of rifaximin results in a reduction in the risk of developing an infection.

Described, but not forming part of the present invention, is also a method of reducing the risk of hospitalization in a subject having HE, the method including administering to the subject an effective amount of rifaximin, wherein administration of rifaximin results in a reduction in the risk of hospitalization. The hospitalization may be due to the development of an infection in the subject.

Described, but not forming part of the present invention, is also a method of reducing the risk of hospitalization due to infection in a subject having HE, the method including administering to the subject an effective amount of rifaximin, wherein administration of rifaximin results in a reduction in the risk of hospitalization due to infection.

Described, but not forming part of the present invention, is also a method of reducing infection in a population having HE, the method including administering to a subject having HE an effective amount of rifaximin, wherein administration of rifaximin results in a reduction in infection.

Described, but not forming part of the present invention, is also a method of reducing the incidence of hospitalization in a population having HE, the method including administering to a subject having HE an effective amount of rifaximin, wherein administration of rifaximin results in a reduction in the incidence of hospitalization. The hospitalization may be due to the development of an infection in the subject.

A method of reducing the incidence of hospitalization due to infection in a population having HE is described, but not forming part of the present invention, the method including administering to a subject having HE an effective amount of rifaximin, wherein administration of rifaximin results in a reduction in the incidence of hospitalization.

The administration of rifaximin may result in a reduction in infection rate. The administration of rifaximin may result in a reduction in the frequency of developing an infection.

The infection, not forming part of the present invention, may comprise one or more selected from the group of: cellulitis, *C*. *difficile* infection, peritonitis, pneumonia, sepsis, septic shock, urinary tract infection and kidney infection.

Rifaximin may be administered for from three to six months, six months, 12 months, 24 months, 36 months, or until the subject's death. In some embodiments, rifaximin is administered for at least three months, six months, one year, two, three years or until the subject's death.

The administration of rifaximin comprises long-term administration. Long-term rifaximin administration may include an administration duration of from 3 months to 6 months, from 3 months to 12 months, from 3 months to 24 months, or from 3 months until death of the subject.

Long-term administration of rifaximin may result in the decline or stability in the incidence of commonly-occurring infections in cirrhotic subjects.

Long-term rifaximin administration may result in the decline or stability in the use of other antibiotics in the subject.

In some embodiments, the other antibiotics used by the subject include one or more selected from the group of: aminoglycoside, amphenicol, ansamycin, beta-Lactam, carbapenem, cephamycin, monobactam, oxacephem, lincosamide, macrolide, polypeptide, tetracycline, a 2,4-diaminopyrimidine class antibiotic, penicillin, neomycin, metronidazole, vancomycin, paromomycin, timidazole, clarithromycin, amoxicillin, sulfasalazine; olsalazie; mesalamine; prednisone; azathioprine; mercaptopurine; methotrexate, ampicillin, clindamycin, rifampicin, chloramphenicol, spectinomycin, a fluoroquinolone antibiotic, and a cephalosporin antibiotic. The fluoroquinolone antibiotic can be at least one selected from the group of: balofloxacin, ciprofloxacin, difloxacin, enrofloxacin, fleroxacin, gatifloxacin, grepafloxacin, levofloxacin, lomefloxacin, marbofloxacin, moxifloxicin, nadifloxacin, norfloxacin, ofloxacin, orbifloxacin, pazufloxacin, perfloxacin, rufloxacin, sparfloxacin, temafloxacin, and tosufloxacin. The cephalosporin antibiotic can be at least one selected from the group of: cefacetrile, cefaclomezine, cefaclor, cefadroxil, cefalexin, cefaloglycin, cefalonium, cefaloram, cefaloridine, cefalotin, cefaparole, cefapirin, cefatrizine, cefazaflur, cefazedone, cefazolin, cefbuperazone, cefcanel, cefcapene, cefclidine, cefdaloxime, cefdinir, cefditoren, cefedrolor, cefempidone, cefepime, cefetamet, cefetrizole, cefivitril, cefixime, cefluprenam, cefmatilen, cefmenoxime, cefmepidium, cefmetazole, cefminox, cefodizime, cefonicid, cefoperazone, cefoselis, cefotaxime, cefotetan, cefovecin, cefoxazole, cefoxitin, cefozopran, cefpimizole, cefpirome, cefpodoxime, cefprozil, cefquinome, cefradine, cefrotil, cefroxadine, cefsumide, ceftaroline, ceftazidime, cefteram, ceftezole, ceftibuten, ceftiofur, ceftiolene, ceftioxide, ceftizoxime, ceftriaxone, cefuracetime, cefuroxime, cefuzonam, and loracarbef. In some embodiments, the other antibiotics comprises one or more that are administered orally, intravenously, or topically.

In any of the foregoing embodiments, the rifamycin class antibiotic comprises one or more of rifaximin or a Form α, Form β, Form γ, Form δ, Form ε, Form ζ, Form η, Form 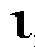 , Form kappa, Form lambda, Form mu, From omicron, Form pi, Form theta, Form xi, mesylate Form or amorphous Forms of rifaximin and a pharmaceutically acceptable carrier. The rifaximin may be formulated as a pharmaceutical composition. In some embodiments, the pharmaceutical composition further comprises excipients.

In any of the foregoing embodiments, rifaximin can include one or more of rifaximin or a Form α, Form β, Form γ, Form δ, Form ε, Form ζ, Form η, Form 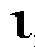 , , Form kappa, Form lambda, Form mu, From omicron, Form pi, Form theta, Form xi, mesylate Form or amorphous Forms of rifaximin and a pharmaceutically acceptable carrier. The rifaximin may be formulated as a pharmaceutical composition. In some embodiments, the pharmaceutical composition further comprises excipients.

In some embodiments, the excipients comprise one or more of a diluting agent, binding agent, lubricating agent, disintegrating agent, coloring agent, flavorings agent or sweetening agent.

In some embodiments, the composition is formulated for selected coated and uncoated tablets, hard and soft gelatin capsules, sugar-coated pills, lozenges, wafer sheets, pellets and powders in sealed packet. In some embodiments, the composition is formulated for topical use.

### DESCRIPTION OF THE DRAWINGS

Figure 1 shows a graph of continuous adequate relief of IBS symptoms during non-treatment follow-up.
Figure 2 shows a graph of continuous adequate relief of bloating symptoms during non-treatment follow-up.
Figure 3 shows proposed study design for treatment with rifaximin to show durability of response.
Figure 4 shows graphical results of adequate relief of IBS symptoms.
Figure 5 shows results of adequate relief of bloating symptoms.
Figure 6 shows results of change from baseline in bloating symptoms after treatment with rifaximin.

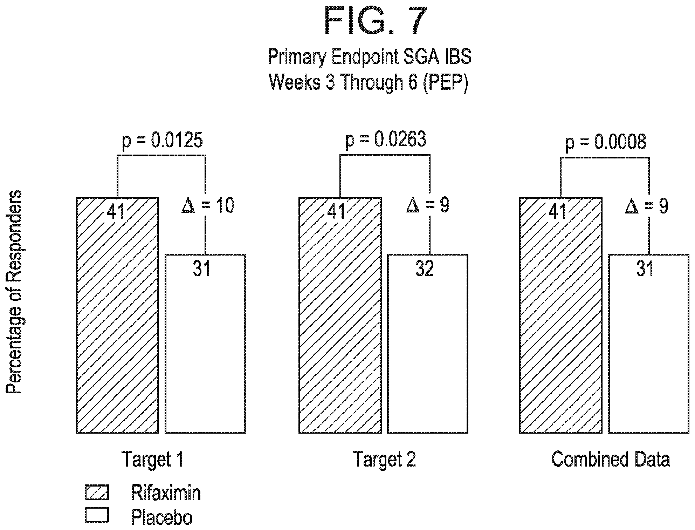

Figure 7 shows an analysis of IBS weeks 3 through 6.

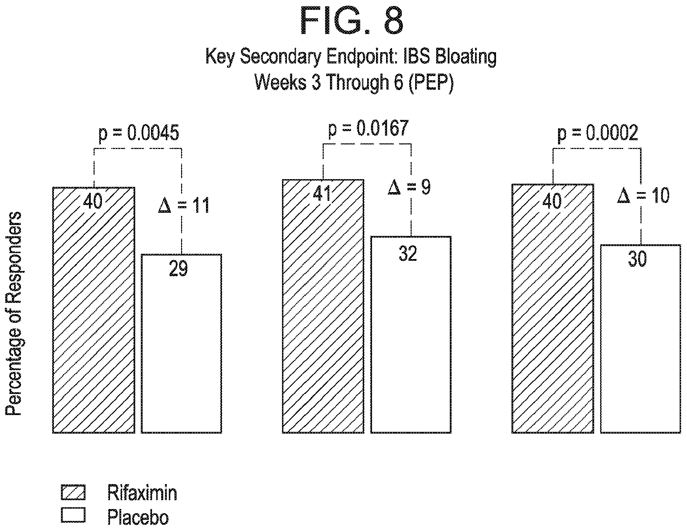

Figure 8 shows IBS bloating data for weeks 3 through 6.

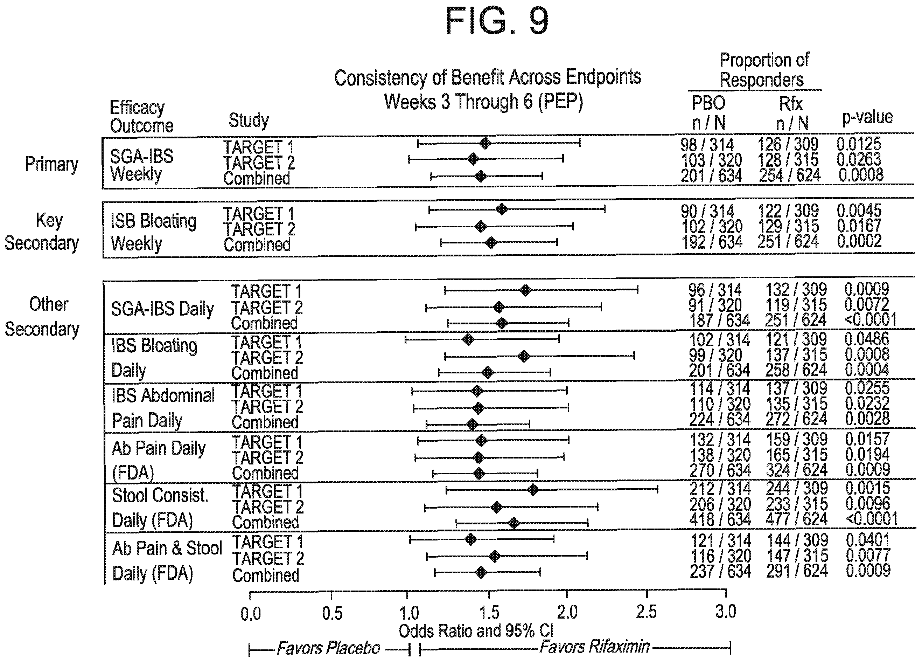

Figure 9 shows IBS consistency data for weeks 3 through 6.

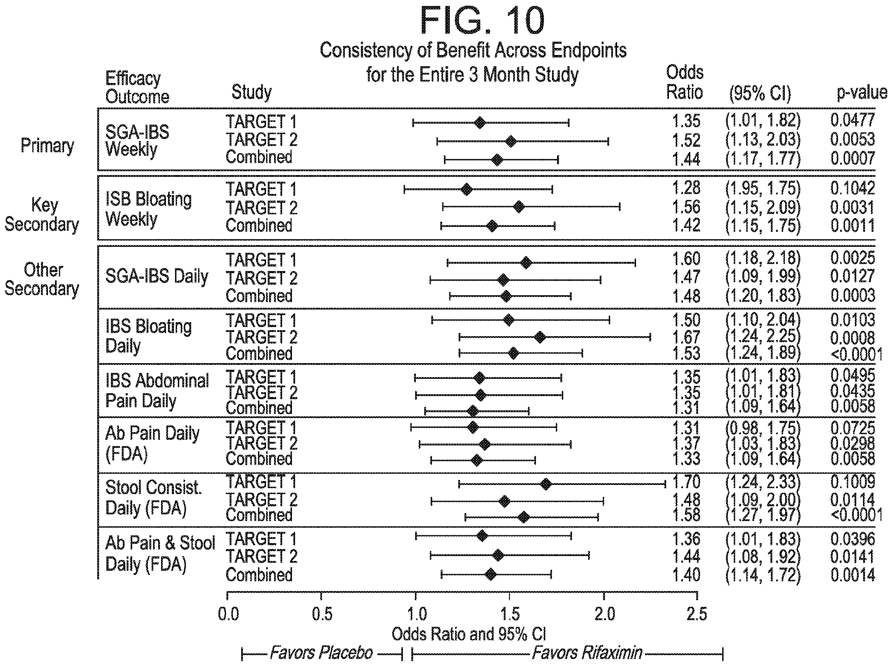

Figure 10 shows IBS data for the entire 3 month study.

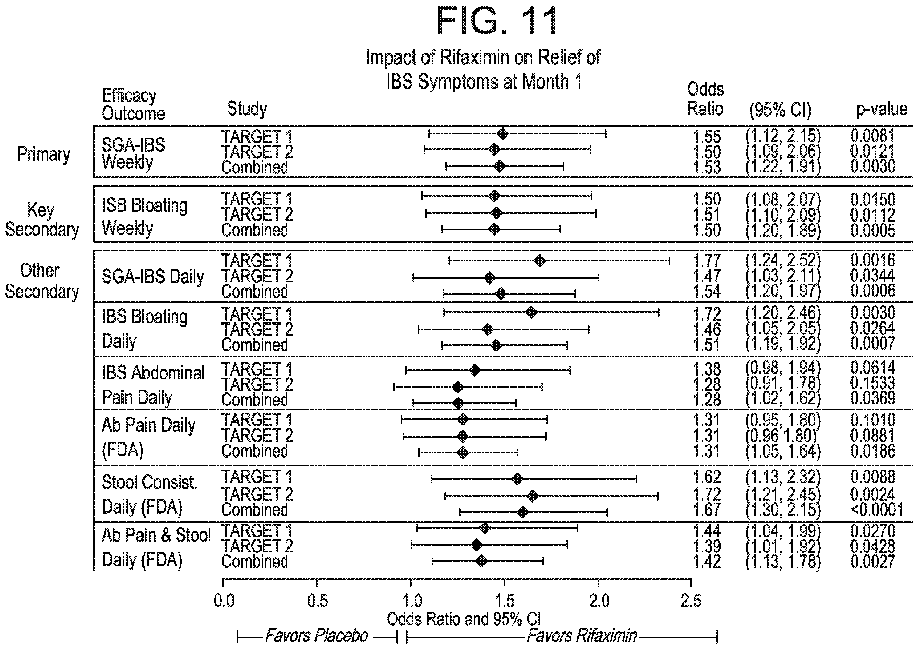

Figure 11 shows relief of IBS symptoms for the first 4 weeks.

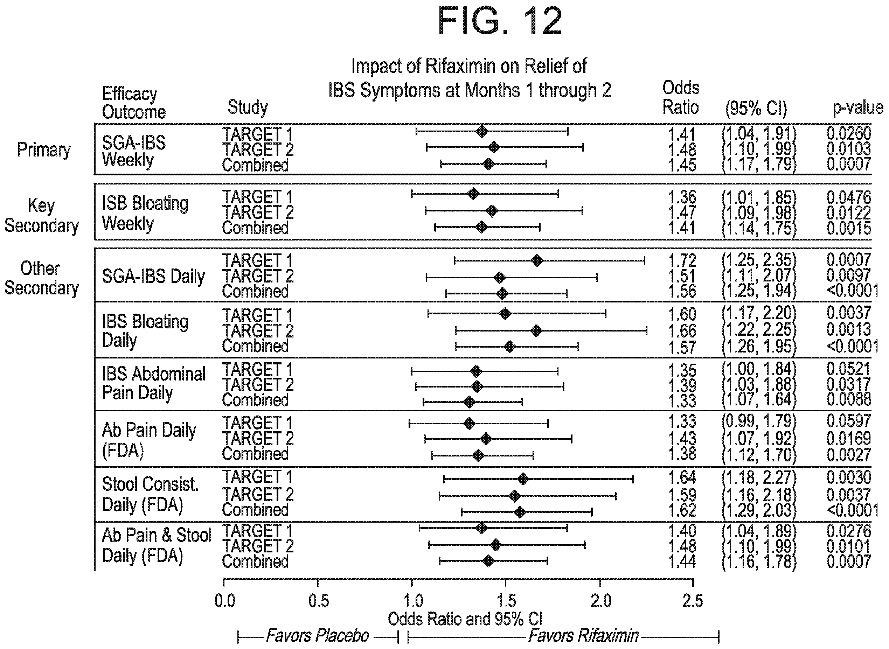

Figure 12 shows relief of IBS symptoms for the first two months.

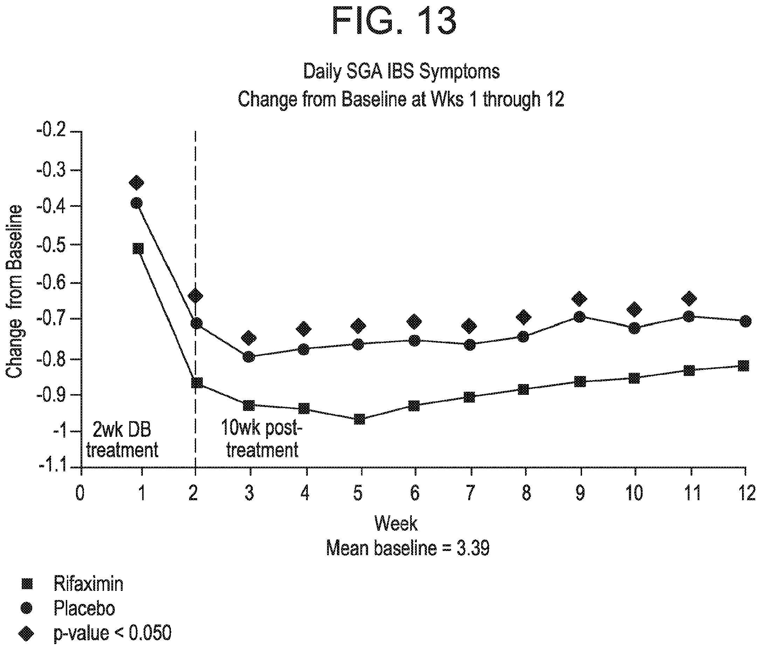

Figure 13 shows daily IBS symptoms weeks 1 through 12.
Figure 14 shows the study design for IBS retreatment.

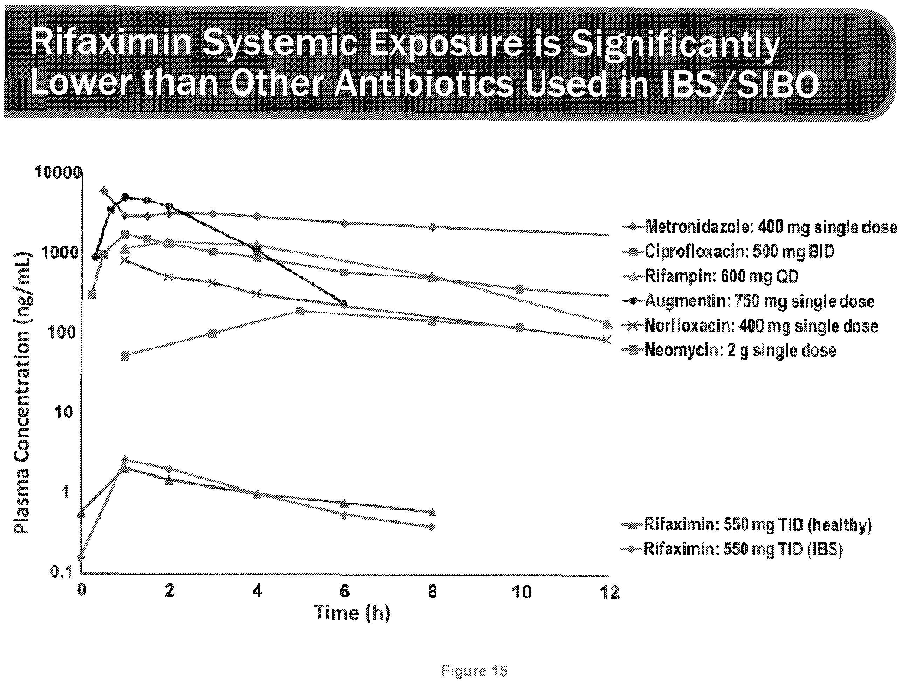

Figure 15 is a graph showing that systemic exposure of rifaximin is significantly lower relative to that of other antibiotics commonly used to treat IBS and/or small intestinal bowel overgrowth (SIBO).
Figure 16 shows a graph of time to last unformed stool analysis in rifaximin treatment of CDI in d-IBS patients.
Figure 17 shows a graph of the time to resolution of diarrhea analysis in rifaximin-treated d-IBS patients.
Figure 18 is a graph showing the average number of unformed stools per day of rifaximin treatment of CDI in d-IBS patients.

### DETAILED DESCRIPTION

Rifaximin (USAN, INN; see The Merck Index, XIII Ed., 8304, CAS No. 80621-81-4), (2S,16Z,18E,20S,21S,22R, 23R,24R,25S,26S,27S,28E)-5,6,21,23,25 Pentahydroxy -27 - methoxy -2,4,11,16,20,22,24,26 - octamethyl-2,7 - (epoxypentadeca-(1,11,13) trienimino) benzofuro (4,5-e) pyrido(1,2,-a) benzimidazole-1,15(2H)-dione,25-acetate), is a semi-synthetic antibiotic produced from rifamycin O. Rifaximin is a molecule belonging to the rifamycin class of antibiotics, e.g., a pyrido-imidazo rifamycin. Rifaximin exerts a broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency.

Rifaximin is also described in Italian Patent IT 1154655 and EP 0161534. EP patent 0161534 discloses a process for rifaximin production using rifamycin O as the starting material (The Merck Index, XIII Ed., 8301). US 7,045,620 B1 discloses polymorphic forms of rifaximin, as do USSN 11/658,702; USSN 61/031,329; USSN 12/119,622; USSN 12/119,630; USSN 12/119,612; USSN 12/119,600; USSN 11/873,841; Publication WO 2006/094662; and USSN 12/393012.

A rifamycin class antibiotic is, for example, a compound having the structure of Formula I: 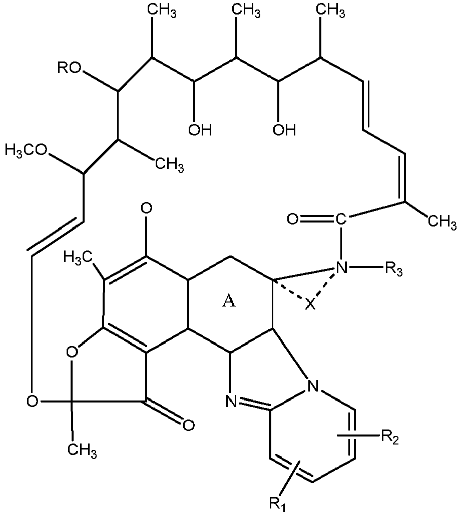 wherein A may be the structure A₁: 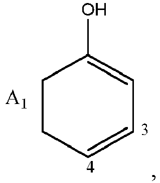 or the structure A₂ 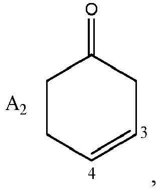 wherein, -x- is a covalent chemical bond or nil; R is hydrogen or acetyl;
R₁ and R₂ independently represent hydrogen, (C₁₋₄) alkyl, benzyloxy, mono- and di-(C₁₋₃) alkylamino-(C₁₋₄) alkyl, (C₁₋₃)alkoxy- (C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is hydrogen or acetyl, Ri and R₂ independently represent hydrogen, (C₁₋₄)alkyl, benzyloxy, hydroxy-(C₂₋₄) alkyl, di-(C₁₋₃) alkylamino-(C₁₋₄) alkyl, nitro or R₁ and R₂ taken together with two consecutive carbon atoms of the pyridine nucleus form a benzene ring and R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is acetyl, R₁ and R₂ independently represent hydrogen, (C₁₋₄) alkyl or R₁ and R₂ taken together with two consecutive carbon atoms of the pyridine nucleus form a benzene ring and R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, which is 4-deoxy-4'-methyl-pyrido[1',2'-1,2]imidazo [5,4-c]rifamycin SV. Also described herein is a compound as defined above, which is 4-deoxy-pyrido [1',2':1,2]imidazo [5,4-c] rifamycin SV.

Also described herein is a compound as defined above, wherein A is as described above,-x- is a covalent chemical bond or nil; R is hydrogen or acetyl; R₁ and R₂ independently represent hydrogen, (C₁₋₄) alkyl, benzyloxy, mono- and di-(C₁₋₃)alkylamino(C₁₋₄)alkyl, (C₁₋₃)alkoxy- (C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Rifaximin is a compound having the structure of formula II:

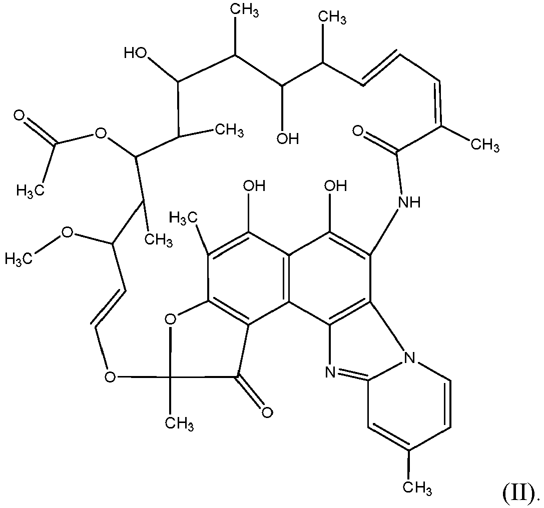

"Rifaximin", as used herein, includes solvates and polymorphous forms of the molecule, including, for example, Form α, Form β, Form γ Form δ, Form ε, Form ζ, Form η, Form 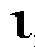 , Form kappa, Form theta, From mu, From omicron, Form pi, Form lambda, Form xi, mesylate Form or amorphous Forms of rifaximin. These forms are described in more detail, for example, in EP 05 004 635.2, filed 03 March 2005; U.S. Patent No. 7,045,620; U.S. Patent No. 7,612,199; U.S. Patent No. 7,709,634; U.S. Patent No. 7,915,275; U.S. Patent No. 8,067,429; U.S. Patent No. 8,193,196; U.S. Patent No. 8,227,482; G. C. Viscomi, et al., CrystEngComm, 2008, 10, 1074-1081 (April 2008), US Patent Publication No. 2010/0174064, US Patent Publication No. 2009/0028940, US Patent Publication No. 2005/0272754 and U.S. Patent Publication No. 2012/0108620.

Medicinal preparations may contain gastrointestinal specific antibiotics together with usual excipients, discussed infra.

"Polymorphs" or "polymorphic forms" as used herein, refer to the occurrence of different crystalline forms of a single compound in distinct hydrate status, e.g., a property of some compounds and complexes. Thus, polymorphs are distinct solids sharing the same molecular formula, yet each polymorph may have distinct physical properties. Therefore, a single compound may give rise to a variety of polymorphic forms where each form has different and distinct physical properties, such as solubility profiles, melting point temperatures, hygroscopicity, particle shape, density, flowability, compatibility and/or x-ray diffraction peaks. The solubility of each polymorph may vary, thus, identifying the existence of pharmaceutical polymorphs is essential for providing pharmaceuticals with predictable solubility profiles. It is desirable to investigate all solid state forms of a drug, including all polymorphic forms, and to determine the stability, dissolution and flow properties of each polymorphic form. Polymorphic forms of a compound can be distinguished in a laboratory by X-ray diffraction spectroscopy and by other methods such as, infrared spectrometry. For a general review of polymorphs and the pharmaceutical applications of polymorphs see G. M. Wall, Pharm Manuf. 3, 33 (1986); J. K. Haleblian and W. McCrone, J Pharm. Sci., 58, 911 (1969); and J. K. Haleblian, J. Pharm. Sci., 64, 1269 (1975). As used herein, the term polymorph is occasionally used as a general term in reference to the forms of rifaximin and include within the context, salt, hydrate, polymorph and amorphous forms of rifaximin disclosed herein. This use depends on context and will be clear to one of skill in the art. Exemplary polymorphic forms of rifaximin useful in the methods and kits described herein are set forth in the published patent applications set forth above.

"GI specific antibiotic," and "GI antibiotic" as used herein include antibiotic known to have an effect on GI disease. For example, a rifamycin class antibiotic (e.g., rifaximin), neomycin, metronidazole, teicoplanin, ciprofloxacin, doxycycline, tetracycline, augmentin, cephalexin, penicillin, ampicillin, kanamycin, rifamycin, vancomycin, and combinations thereof are useful GI specific antibiotics. Even more preferable are GI specific antibiotics with low systemic absorption, for example, rifaximin. Low systemic absorption includes, for example, less than 10% absorption, less than 5% absorption, less than 1% absorption and less than 0.5% absorption. Low systemic absorption also includes, for example, from between about 0.01-1% absorption, from between about 0.05 -1% absorption, from between about 0.1-1% absorption, from between about 1-10% absorption, or from between about 5 - 20% absorption. According to the invention rifaximin is used.

"Ameliorate," "amelioration," "improvement" or the like refers to, for example, a detectable improvement or a detectable change consistent with improvement that occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range between about any two of these values. Such improvement or change may be observed in treated subjects as compared to subjects not treated with rifaximin, where the untreated subjects have, or are subject to developing, the same or similar disease, condition, symptom or the like. Amelioration of a disease, condition, symptom or assay parameter may be determined subjectively or objectively, e.g., self assessment by a subject(s), by a clinician's assessment or by conducting an appropriate assay or measurement, including, e.g., a quality of life assessment, a slowed progression of a disease(s) or condition(s), a reduced severity of a disease(s) or condition(s), or a suitable assay(s) for the level or activity(ies) of a biomolecule(s), cell(s) or by detection of BD episodes or infection in a subject. Amelioration may be transient, prolonged or permanent or it may be variable at relevant times during or after a GI specific antibiotic is administered to a subject or is used in an assay or other method described herein or a cited reference, e.g., within timeframes described infra, or about 1 hour after the administration or use of a GI specific antibiotic to about 7 days, 2 weeks, 28 days, or 1, 3, 6, 9 months or more after a subject(s) has received such treatment.

The "modulation" of, e.g., a symptom, level or biological activity of a molecule, or the like, refers, for example, that the symptom or activity, or the like is detectably increased or decreased. Such increase or decrease may be observed in treated subjects as compared to subjects not treated with a GI specific antibiotic, where the untreated subjects have, or are subject to developing, the same or similar disease, condition, symptom or the like. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500%, 1000% or more or within any range between any two of these values. Modulation may be determined subjectively or objectively, *e*.*g*., by the subject's self assessment, by a clinician's assessment or by conducting an appropriate assay or measurement, including, *e*.*g*., quality of life assessments or suitable assays for the level or activity of molecules within a subject. Modulation may be transient, prolonged or permanent or it may be variable at relevant times during or after a GI specific antibiotic is administered to a subject or is used in an assay or other method described herein or a cited reference, e.g., within times descried infra, or about 1 hour of the administration or use of a GI specific antibiotic to about 2 weeks, 28 days, 3, 6, 9 months or more after a subject(s) has received a GI specific antibiotic.

The term "modulate" may also refer to increases or decreases in the activity of a cell in response to exposure to a GI specific antibiotic, *e*.*g*., the inhibition of proliferation and/or induction of differentiation of at least a sub-population of cells in an animal such that a desired end result is achieved, *e*.*g*., a therapeutic result of GI specific antibiotic used for treatment may increase or decrease over the course of a particular treatment.

The term "effective amount" includes an amount effective, at dosages and for periods of time necessary, to achieve the desired result, *e*.*g*., sufficient to treat or prevent a disease, disorder, or infection as described herein. An effective amount of a GI specific antibiotic may vary according to factors such as the disease state, age, and weight of the subject, and the ability of a GI specific antibiotic to elicit a desired response in the subject. Dosage regimens may be adjusted to provide the optimum therapeutic response. An effective amount is also one in which any toxic or detrimental effects (e.g., side effects) of a GI specific antibiotic are outweighed by the therapeutically beneficial effects.

Similarly, the language "a prophylactically effective amount" of a compound refers to an amount of a compound of formula I, formula II, or otherwise described herein which is effective, upon single or multiple dose administration to the subject, in preventing or treating a disease, disorder or infection as described herein. In the present invention the disease is diarrhea-predominant irritable bowel syndrome (d-IBS, also referred to as IBS-D).

As used herein, "subject" includes organisms which are capable of suffering from a disease, disorder, or infection treatable by a rifamycin class antibiotic (*e*.*g*., rifaximin) as described herein or who could otherwise benefit from the administration of a rifamycin class antibiotic (*e*.*g*., rifaximin) as described herein, such as human and non-human animals. Preferred human animals include human subjects. The term "non-human animals" includes all vertebrates, *e*.*g*., mammals, *e*.*g*., rodents, *e*.*g*., mice, and non-mammals, such as non-human primates, *e*.*g*., sheep, dog, cow, chickens, amphibians, reptiles, etc. Susceptible to a bowel disorder is meant to include a subject at risk of developing a bowel disorder or a person who is in remission from a BD or a person who may relapse from a BD, i.e. a subject with d-IBS.

The term "administration" or "administering" includes routes of introducing a GI specific antibiotic to a subject to perform their intended function. Examples of routes of administration that may be used include injection, oral, inhalation, vaginal, rectal and transdermal. The pharmaceutical preparations may be given by forms suitable for each administration route. For example, these preparations are administered in tablets or capsule form, by injection, inhalation, eye lotion, eye drops, ointment, suppository, etc. administration by injection, infusion or inhalation; topical by lotion or ointment; and rectal by suppositories. Oral administration is preferred. The injection can be bolus or can be continuous infusion. Depending on the route of administration, a GI specific antibiotic can be coated with or disposed in a selected material to protect it from natural conditions that may detrimentally affect its ability to perform its intended function. A GI specific antibiotic can be administered alone, or in conjunction with either another agent or agents as described above or with a pharmaceutically-acceptable carrier, or both. A GI specific antibiotic can be administered prior to the administration of the other agent, simultaneously with the agent, or after the administration of the agent. Furthermore, a GI specific antibiotic can also be administered in a pro-form, which is converted into its active metabolite, or more active metabolite *in vivo.*

Administration "in combination with" one or more further therapeutic agents includes simultaneous (concurrent) and consecutive administration in any order.

As will be readily apparent to one skilled in the art, the useful *in vivo* dosage to be administered and the particular mode of administration will vary depending upon the age, weight and mammalian species treated, the particular compounds employed, and/or the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved.

The term "obtaining" as in "obtaining a GI specific antibiotic" is intended to include purchasing, synthesizing or otherwise acquiring a GI specific antibiotic. For example, obtaining rifaximin can include purchasing, synthesizing or otherwise acquiring rifaximin.

The term "pharmaceutical agent composition" (or agent or drug) as used herein refers to a chemical compound, composition, agent or drug capable of inducing a desired therapeutic effect when properly administered to a patient. It does not necessarily require more than one type of ingredient.

As used herein, "durability of response" includes for example, adequate relief of symptoms after removal of treatment, continuous adequate relief of symptoms after removal of treatment, or response that is greater than or superior to placebo response. A response by a subject may be considered durable, for example, if they have a response to the rifamycin class antibiotic (*e*.*g*. rifaximin) after removal from treatment. The duration of response, may be, for example, 2 days, 7 days, two weeks, 3 weeks, 4 weeks, 12 weeks, between about 1 week and about 24 weeks or longer. In some embodiments, durability of response is a therapeutic effect that is observed for at least two months out of a three-month period. The response may be measured, for example using one or more of the methods outlined below, including, for example, a subject's subjective assessment of their symptoms or a healthcare provider's or caretaker's assessment of a subject's symptoms.

As used herein, "selecting subjects who respond," "selection of subjects who respond" or the like, include, for example, determining that a subject has responded to treatment based on a decrease of bowel disease (BD) or IBS symptoms and/or following label instructions to administer a product (*e.g.*, a rifamycin class antibiotic) for a certain period of time or the like. The determination or selection may be based on the label (*e.g.*, package or package insert) instructions or on the subject's subjective assessment of their symptoms or a healthcare provider's or caretaker's assessment of a subject's symptoms.

As used herein, a "responder" is a subject administered rifaximin for treatment of a disease, disorder or infection as described herein who response to treatment by experiencing relief of symptoms, alleviation of discomfort or pain, or a general improvement in health relative to baseline. For example, a responder can be a subject administered rifaximin for treating IBS that has a positive response during at least 2 out of 4 weeks based on daily questions for the weekly responses for both abdominal pain and stool consistency. In one embodiment, a responder has a decrease in weekly average abdominal pain score and a reduction in the # of days per week with at least 1 stool with a consistency of greater than or equal to 6 (per the Bristol stool scale) as defined by the Rome III criteria.

In some embodiments, a responder can be identified as an IBS-D subject having one or more of the following: Subjects with moderate bloating and abdominal pain, loose stools and/or bothersome urgency. For example, any one of the following criteria can be used to identify subject that are likely to respond to treatment with rifaximin: abdominal pain greater than or equal to, for example, 2, 2.5, 3, or 3.5; bloating greater than, for example, 2, 2.5, 3, or 3.5; loose stools with an average stool consistency score greater than or equal to 3, 3.5, 4, 4.5; or bothersome urgency for example greater than or equal to 3.0, 3.5, 4.0 or 4.5 days with urgency. Alternatively, two or more of the above-identified criteria can be used to identify subjects that are likely to respond to treatment with rifaximin. For example, abdominal pain and bloating; abdominal pain and loose stools, abdominal pain and bothersome urgency; abdominal pain, bloating and loose stools, etc.

A responder can also be defined as: 1) ≥ 30% improvement in abdominal pain, < 4 in stool consistency, and ≥ 1 point decrease in daily IBS symptoms; 2) ≥ 30% improvement in abdominal pain, and ≥ 50% decrease in number of loose/watery stools within a given week comparing to the baseline; 3) ≥ 30% decrease in mean abdominal pain score from baseline using_the worst 3 daily entries in a given week; 4) ≥ 30% decrease in the number of days with urgency within a given week comparing to the baseline; 5) ≥ 30% improvement in the selected worst baseline symptom; or 6) daily responder scores of 0 (not at all) or 1(hardly) at least 50% of the days in a given week; OR 0 (not at all), 1 (hardly) or 2 (somewhat) 100% of days in a given week in the selected worst baseline symptom.

In a specific embodiment, a subject is defined as "a one month responder" if the subject has been administered rifaximin and is considered a responder at 2 weeks post treatment, wherein treatment comprises administering rifaximin for 14 days.

As used herein, a subject is considered to have a "recurrence" when criteria for a response is absent for at least 3 weeks during a 4 week period. Alternatively, "recurrence" can be defined as a worsening of stool or abdominal pain.

### Methods of Treatment

The invention does not relate to methods of treatment carried out on the human or animal body.

Provided herein are methods, which do not form part of the present inveniton, of treating, preventing, or alleviating disease, disorder or an infection comprising administering to a subject in need thereof an effective amount of rifaximin. The infection, which does not form part of the present invention, can be, for example, an infection caused by *C*. *difficile.* The disease or disorder can be, for example, a bowel-related disorder. Bowel related disorders (*e*.*g*., bowel diseases) include one or more of irritable bowel syndrome (IBS), alternating predominant IBS, Crohn's disease, traveler's diarrhea, ulcerative colitis, enteritis, small intestinal bacterial overgrowth, chronic pancreatitis, pancreatic insufficiency, colitis, diverticular disease, hepatic encephalopathy, abdominal pain associated with IBS and/or pouchitis. In some embodiments, not forming part of the present invention, the bowel-related disorder is hepatic encephalopathy. According to the invention, the bowel-related disorder is IBS-D.

### C. difficile Infection (referential)

*Clostridium difficile* is a gram-positive anaerobic bacterium, and is deemed a significant human pathogen causing a spectrum of diseases ranging from mild diarrhea to fulminant pseudomembranous colitis (PMC). The bacterium is endemic in hospitals, and studies have shown that approximately one third of patients receiving antibiotic treatment in acute-care medical wards were colonized by *C*. *difficile* while in hospital (Kyne, L., et al., 2002, Clin. Infect. Dis. 34(3), pp346-53, PMID: 11774082). Patients suffering from CDI respond well to treatment with vancomycin. However, the use of vancomycin is one of last resort since it is associated with several problems. Not only may it cause nephrotoxicity, ototoxicity, bone marrow toxicity and the red man syndrome, but vancomycin treatment often is not effective for treatment of CDI. Additionally, there is evidence that *C*. *difficile* is becoming at least partially resistant to vancomycin, demonstrating the need for new alternatives in the treatment of CDI.

Described are methods of treating, preventing, or alleviating *C*. *difficile* infection (CDI) in a subject, wherein the method includes administering to the subject an effective amount of rifaximin. The subject may be one who failed to respond to other therapies or treatment by antibiotics other than rifaximin. The subject may be one who failed to respond to treatment with vancomycin.

Also described are methods of treating, preventing, or alleviating an antibioticresistant *C*. *difficile* infection, comprising administering rifaximin to a subject in need thereof, wherein administration of rifaximin is effective in treating the antibioticresistant CDI. In embodiments of the invention, a method of preventing CDI is provided, wherein the method comprises administering a non-systemic antibiotic to a subject in need of antibiotic treatment for a condition. In some embodiments, the condition is one selected from the group of: Crohn's disease, travelers' diarrhea, hepatic encephalopathy, minimal hepatic encephalopathy, irritable bowel syndrome, restless leg syndrome, dermal infections, small intestinal bacterial overgrowth, chronic pancreatitis, pancreatic insufficiency, diverticulitis, enteritis and colitis, skin infections, mucous membrane disorders, pouchitis, vaginal infections, anal fissures, ear infections, lung infections, periodontal conditions, rosacea, and other infections of the skin and/or other related conditions. In some embodiments, the non-systemic antibiotic is a rifaximin.

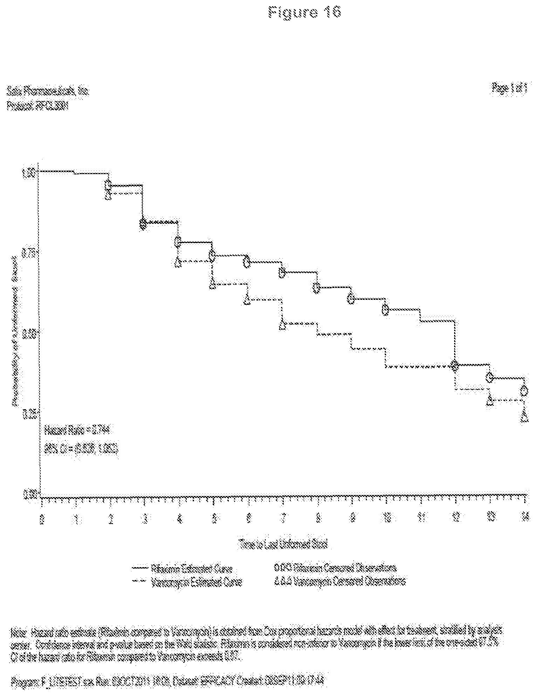

Figure 16 and Tables 40-41 demonstrate the efficacy of rifaximin for treating CDI. It was surprisingly shown that a rifamycin class antibiotic (*e*.*g*., rifaximin) is particularly efficacious for treating CDI, *e*.*g*., diarrhea associated with CDI.

In addition, treatment with all antibiotics can predispose a subject to CDI. Rifaximin administration preserves the colonic flora and is less likely to cause CDI. In situations where CDI develops during rifaximin use, it was found that the CDI continued to respond to treatment with rifaximin.

In some embodiments, methods for treating CDI by reducing CDI-related abdominal pain and discomfort by, for example, at least 20%, 25%, 30%, 35%, 40%, 50%, 60%, 70%, 80%, 90% or more, are described. Additionally the methods provided methods of treating CDI in a subject by improving stool consistency.

Rifaximin may be used in various treatment regimes. These regimes may vary depending upon the subject and the type of treatment. For example, rifaximin may be administered, for example, twice a day, three times a day, or four times or more often as necessary per day. Rifaximin may be administered in doses, for example of from about between 25 mg once daily to about 3000 mg TID. For example, rifaximin can be administered in daily doses of about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, In some embodiments, rifaximin can be administered in daily doses of about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, or about 500 mg. In some embodiments, rifaximin can be administered in daily doses of about 550 mg, about 600 mg, about 650 mg, about 700 mg, about 750 mg, about 800 mg, about 850 mg, about 900 mg, about 950 mg, or about 1000 mg. In some embodiments, rifaximin can be administered in daily doses of about 1100 mg about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, about 2800 mg, about 2900 mg, or about 3000 mg, In some embodiments, rifaximin can be administered in doses of about 25 mg BID, about 30 mg BID, about 35 mg BID, about 40 mg BID, about 45 mg BID, about 50 mg BID, about 55 mg BID, about 60 mg BID, about 65 mg BID, about 70 mg BID, about 75 mg BID, about 80 mg BID, about 85 mg BID, about 90 mg BID, about 95 mg BID, or about 100 mg BID. In some embodiments, rifaximin can be administered in doses of about 125 mg BID, about 150 mg BID, about 175 mg BID, about 200 mg BID, about 225 mg BID, about 250 mg BID, about 275 mg BID, about 300 mg BID, about 325 mg BID, about 350 mg BID, about 375 mg BID, about 400 mg BID, about 425 mg BID, about 450 mg BID, about 475 mg BID, or about 500 mg BID. In some embodiments, rifaximin can be administered in doses of about 550 mg BID, about 600 mg BID, about 650 mg BID, about 700 mg BID, about 750 mg BID, about 800 mg BID, about 850 mg BID, about 900 mg BID, about 950 mg BID, or about 1000 mg BID. In some embodiments, rifaximin can be administered in doses of about 1100 mg BID, about 1200 mg BID, about 1300 mg BID, about 1400 mg BID, about 1500 mg BID, about 1600 mg BID, about 1700 mg BID, about 1800 mg BID, about 1900 mg BID, about 2000 mg BID, about 2100 mg BID, about 2200 mg BID, about 2300 mg BID, about 2400 mg BID, about 2500 mg BID, about 2600 mg BID, about 2700 mg BID, about 2800 mg BID, about 2900 mg BID or about 3000 mg BID, In some embodiments, rifaximin can be administered in doses of about 25 mg TID, about 30 mg TID, about 35 mg TID, about 40 mg TID, about 45 mg TID, about 50 mg TID, about 55 mg TID, about 60 mg TID, about 65 mg TID, about 70 mg TID, about 75 mg TID, about 80 mg TID, about 85 mg TID, about 90 mg TID, about 95 mg TID, or about 100 mg TID. In some embodiments, rifaximin can be administered in doses of about 125 mg TID, about 150 mg TID, about 175 mg TID, about 200 mg TID, about 225 mg TID, about 250 mg TID, about 275 mg TID, about 300 mg TID, about 325 mg TID, about 350 mg TID, about 375 mg TID, about 400 mg TID, about 425 mg TID, about 450 mg TID, about 475 mg TID, or about 500 mg TID, In some embodiments, rifaximin can be administered in doses of about 550 mg TID, about 600 mg TID, about 650 mg TID, about 700 mg TID, about 750 mg TID, about 800 mg TID, about 850 mg TID, about 900 mg TID, about 950 mg TID, or about 1000 mg TID. In some embodiments, rifaximin can be administered in doses of about 1100 mg TID, about 1200 mg TID, about 1300 mg TID, about 1400 mg TID, about 1500 mg TID, about 1600 mg TID, about 1700 mg TID, about 1800 mg TID, about 1900 mg TID, about 2000 mg TID, about 2100 mg TID, about 2200 mg TID, about 2300 mg TID, about 2400 mg TID, about 2500 mg TID, about 2600 mg TID, about 2700 mg TID, about 2800 mg TID, about 2900 mg TID or about 3000 mg TID. The rifaximin may be administered, for example, in tablet form, powdered form, liquid form or in capsules. In some embodiments, rifaximin can be administered in a time-released formulation.

In some embodiments, rifaximin is administered as a soluble solid dispersion. For example, rifaximin can be administered at between about 25 - 550 mg of soluble solid dispersion of rifaximin.

In some embodiments, the rifaximin is administered to a subject from between about 1 week to about 6 weeks in duration, from between about 8 weeks to about 12 weeks in duration, or from between about 1 day to about 21 days in duration. In one embodiment, rifaximin is administered for 10 days. The rifaximin may be administered from between about 1 day and about 1 year, or from 1 week to about 52 weeks. The rifaximin may be administered intermittently or continuously during the course of treatment. Length of treatment may vary depending on the type and length of disease and the proper length of treatment may be easily determined by one of skill in the art having the benefit of this disclosure.

For any of the embodiments, rifaximin may be administered, for example, once daily, twice daily, three times daily, or four times daily (or more often as necessary for a particular subject) to a subject. In some embodiments, the methods comprise administering the rifaximin once daily to the subject because it may, for example, minimize the side effects and increase patient compliance. In some embodiments, rifaximin is administered twice and/or three times daily.

Dosages, according to certain preferred embodiments, range from between about 50 to about 6000 mg of rifaximin administered daily. For example, a dose of 400 mg may be administered to a subject three times daily, or a dose of 550 mg may be administered to a subject twice daily. Other appropriate dosages for the methods as disclosed herein may be determined by health care professionals or by the subject. The amount of rifaximin administered daily may be increased or decreased based on the weight, age, health, sex or medical condition of the subject. One of skill in the art would be able to determine the proper dose for a subject based on this disclosure.

In some embodiments, rifaximin may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. For example, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine, and/or loperamide.

The treatment regime for retreating a subject is as claimed.

### Hepatic Encephalopathy (referential)

Hepatic encephalopathy (HE), also known as hepatic coma or portal-systemic encephalopathy (PSE), is a serious, rare, complex, episodic, neuropsychiatric syndrome associated with advanced liver disease. Hepatic encephalopathy is a formidable burden on the patient, his/her family, and the healthcare system; and the current standard of care is inadequate. Overt, episodic HE is common among patients with liver cirrhosis. The condition is rare among individuals in the overall, general population. Overt HE episodes are debilitating, can present without warning, render the patient incapable of self-care, and frequently result in hospitalization. The frequency of hospitalizations due to HE increased since 1993 to over 40,000 patients in 2003; and in 2004, 50,962 patients were hospitalized with a principal diagnosis of HE. HE, as used herein, comprises, for example, episodic, persistent and minimal HE.

HE is manifested as a continuum of psychomotor dysfunction, impaired memory, increased reaction time, sensory abnormalities, poor concentration and in severe forms, as coma. Changes may be observed in personality, consciousness, behavior and neuromuscular function. Neurologic signs may include hyperreflexia, rigidity, myoclonus and asterixis (coarse "flapping" muscle tremor). Cognitive tasks such as connecting numbers with lines can be abnormal. Fetor hepaticus (sweet breath odor) may be present. Electroencephalogram (EEG) tracings show nonspecific slow, triphasic wave activity mainly over the frontal areas. Prothrombin time may be prolonged and not correctable with Vitamin K. A computed tomography scan of the head may be normal or show general atrophy. Finally, signs of liver disease such as jaundice and ascites may be noted.

Rifaximin was found to be advantageous in treatment of HE relative to previously used antibiotics; e.g., negligible systemic absorption (<0.4%) regardless of food intake or presence of GI disease and exhibits no plasma accumulation with high or repeat doses. The lack of systemic absorption makes rifaximin safe and well tolerated, thus improving patient compliance and reducing side effects associated with currently known treatments. Results describing the efficacy of rifaximin in treating HE compared to other antibiotics are described, for example, in WO 2010/040020, and in WO 2011/005388.

Accordingly, in some embodiments, described herein is a method of treating, preventing or maintaining remission from hepatic encephalopathy (HE) in a subject, wherein the method includes administering a therapeutically effective amount of a gastrointestinal (GI) specific antibiotic to the subject. Examples of gastrointestinal antibiotics as used herein include rifamycin class antibiotics, such as rifaximin. In some embodiments, treatment with the GI specific antibiotic maintains remission of HE in the subj ect.

In some embodiments, the therapeutically effective amount of a gastrointestinal (GI) specific antibiotic comprises from between about 1000 mg to about 1200 mg/day.

In some embodiments, the therapeutically effective amount of a GI specific antibiotic comprises from between about 1100 mg to about 1200 mg/day.

In some embodiments, the therapeutically effective amount of a GI specific antibiotic comprises about 1150 mg /day. In some embodiments, the therapeutically effective amount of a GI specific antibiotic comprises 550 mg twice a day. For example, the therapeutically effective amount can include 550 mg rifaximin BID (twice a day).

In some embodiments, the therapeutically effective amount is a dosage regimen of one capsule or tablet of the formulation two times each day, wherein each tablet comprises about 550 mg of the GI specific antibiotic, such as rifaximin.

In some embodiments, the therapeutically effective amount is a dosage regimen of two capsules or tablets three times each day, wherein each capsule comprises about 200 mg of the GI specific antibiotic.

In some embodiments, the therapeutically effective amount is a dosage of 275 mg of a GI specific antibiotic administered four times per day. In another embodiment, 275 mg of a GI specific antibiotic is administered as two dosage forms two times per day.

In some embodiments, the GI specific antibiotic is administered to the subject daily for at least about six months, one year, two, three years or until the subject's death.

In some embodiments, a subject suffering from, susceptible to or in remission from hepatic encephalopathy (HE) can be administered a rifamycin class antibiotic for between about 24 weeks and 24 months. In treating HE, the rifamycin class antibiotic may be administered to the subject for 12 months and longer, for example for a subject's entire life span. In some embodiments, the antibiotic is administered daily until the death of the subject.

Presented herein is a method of treating or preventing HE in a subject, wherein the method includes administering 1100 mg of rifaximin per day to the subject for more than 28 days. In some embodiments, presented herein is a method of maintaining remission of HE in a subject, wherein the method includes administering 550 mg of rifaximin twice a day (BID) to the subject.

In some embodiments, the GI specific antibiotic is administered to the subject with lactulose, prior to treatment with lactulose, or following treatment with lactulose. In some embodiments, the subject or a health care worker is advised to administer the GI specific antibiotic with lactulose. In some embodiments, the subject or a health care worker is advised by a pharmaceutical label or insert to administer the GI specific antibiotic with lactulose in order to maintain remission of HE, or to decrease the risk for episodes of overt HE. In some embodiments, the subject or health care worker is advised to administer two 550 mg tablets of rifaximin twice daily with lactulose. Lactulose use may be titrated over time so that the subject maintains 2-3 soft stool bowel movements per day. In some embodiments, lactulose is administered in 15 ml dosages, wherein each 15 ml dosage contains 10 mg of lactulose. In a typical titration, the subject may start on one dosage, or a partial dosage, per day and then move up in 15 ml dosages over time until they reach an end point of 2-3 soft stool bowel movements per day.

In some embodiments, the method includes decreasing lactulose use in a subject. This method includes: administering rifaximin to a subject daily that is being treated with lactulose, and tapering lactulose consumption. For example, the lactulose consumption may be reduced by 1, 2, 3, 4, 5, 6 or more unit dose cups of lactulose from a baseline level. In some embodiments, the lactulose use may be reduced by 5, 10, 15, 20, 25, 30, 34, 40, 45, 50, 55, 60, 65, or 70 g lactulose from a baseline level. In some embodiments, the baseline use of lactulose is no use.

In some embodiments, the GI specific antibiotic is administered with one or more of align, alinia, Lactulose, pentasa, cholestyramine, sandostatin, vancomycin, lactose, amitiza, flagyl, zegerid, prevacid, or miralax.

Also described herein is a method of reducing the risk of developing an infection in a subject having HE, the method including administering to the subject an effective amount of rifaximin, wherein administration of rifaximin results in a reduction in the risk of developing an infection. The administration of rifaximin can result in a reduction the infection rate itself.

In some embodiments, a method of reducing infection in a population having HE is provided, the method including administering to a subject having HE an effective amount of rifaximin, wherein administration of rifaximin results in a reduction in infection.

Embodiments also relate to a method of reducing the risk of hospitalization in a subject having HE, the method including administering to the subject an effective amount of rifaximin, wherein administration of rifaximin results in a reduction in the risk of hospitalization. In some embodiments, the hospitalization is due to or caused by development of an infection in the subject.

In some embodiments, a method of reducing the incidence of hospitalization in a population having HE is provided, the method including administering to a subject having HE an effective amount of rifaximin, wherein administration of rifaximin results in a reduction in the incidence of hospitalization. In some embodiments, the hospitalization is due to or caused by development of an infection in the subject.

In some embodiments, the infection comprises one or more selected from the group of: cellulitis, *C*. *difficile* infection, peritonitis, pneumonia, sepsis, septic shock, urinary tract infection and kidney infection.

In some embodiments, administration of rifaximin remains the same or declines with time.

In some embodiments, administration of rifaximin comprises long-term administration. Long-term rifaximin administration can include an administration duration of from about 3 months to about 6 months, about 6 months, about 12 months, about 24 months, or about 36 months. In some embodiments, long-term rifaximin administration can include an administration duration of from about 3 months until death of the subject. In some embodiments, long-term rifaximin administration can include an administration duration of at least about three months, six months, one year, two, three years or until the subject's death. Long-term administration of rifaximin can result in the decline or stability in the incidence of commonly-occurring infections in cirrhotic subjects.

In some embodiments, long-term rifaximin administration results in the decline or stability in the use of other antibiotics in the subject. Other antibiotics used by the subject can include one or more selected from: aminoglycoside, fluoroquinolone antibiotics, cephalosporin antibiotics, aminoglycosides, amphenicols, ansamycins, β-Lactams, carbapenems, cephalosporins, cephamycins, monobactams, oxacephems, lincosamides, macrolides polypeptides, tetracyclines, such as spicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, senociclin and tetracycline, nitrofurans, quinolones, sulfonamides, sulfones, lipopeptides, ketolides, and miscellaneous antibiotics such as clofoctol, hexedine, magainins, methenamine, methenamine anhydromethylene-citrate, methenamine hippurate, methenamine mandelate, methenamine sulfosalicylate, nitroxoline, squalamine, xibomol, cycloserine, mupirocin, and tuberin.

Antibiotics can include newly developed antimicrobial and antibiotic agents. In some embodiments, the other antibiotics comprise one or more that are administered orally, intravenously, or topically.

### Irritable Bowel Syndrome

Provided herein is rifaximin for use in retreating a subject having diarrhea-predominant IBS (d-IBS), being a responder to an initial treatment of d-IBS with rifaximin and having a recurrence of abdominal pain or stool consistency for at least 3 weeks during a 4-week assessment period, comprising
- an initial treatment phase comprising a 2 weeks administration of 550 mg of rifaximin three times daily (TID) and a 2 weeks of treatment-free follow-up,
- a first treatment-free maintenance phase of up to 8 to 12 weeks,
- a first repeat treatment phase comprising a 2 weeks administration of 550 mg of rifaximin TID and a 2 weeks of treatment-free follow-up,
- a second treatment-free maintenance phase of up to 8 weeks,
- a second repeat treatment phase comprising a 2 weeks administration of 550 mg of rifaximin TID and a 2 weeks treatment-free follow-up.

The methods presented herein allow for retreatment of a subject having IBS-D after the subject has been treated one or more time for IBS-D, e.g., wherein the subject has previously been treated for IBS-D with rifaximin. In one embodiment, the subject is retreated with rifaximin if they previously responded to treatment with rifaximin.

Table 1 below demonstrates differential response to treatment with rifaximin based on gender, age and IBS type. Table 2 demonstrates response to the treatment is correlated with the duration of disease.

**Table 1**

| | Thresholds | Treatment effect (IBS Sx, Bloating) |
|---|---|---|
| Gender | M versus. | 21%*, 13.6% |
| | F | 3.5%, 3.9% |
| Age | <65 versus. | 6.9%, 6.8% |
| | ≥65 | 19.1%, 3.2% |
| dIBS Type | dIBS only versus. | 6.3%, 4.5% |
| | alBS | 31.4%*, 31.4% |

| | | |
|---|---|---|
| * p-value <0.05 | | |

**Table 2**

| | Thresholds | Treatment effect (IBS Sx, Bloating) |
|---|---|---|
| Diabetes History | Y versus. | -12.7%, -5.6% |
| | N | 9.5%, 7.4% |
| Disease Duration: | ≤10 y | 1.8%, 2.8% |
| | 10-20 y | 20.1%, 11.7% |
| | >20 y | 46.6%*, 35.1% |

| | | |
|---|---|---|
| * p-value <0.05 | | |

It was surprisingly shown that rifaximin is particularly efficacious in males for the treatment of IBS.

Accordingly, described herein are methods for treating IBS by reducing IBS-related abdominal pain and discomfort by, for example, at least 20%, 25%, 30%, 35% or more. Additionally the methods provided methods of treating IBS in a subject by improving stool consistency, for example, a stool consistency score of <4, and improving the average daily IBS score by at least 1.

In related embodiments, at least 25%, 30%, 35%, 40% or more of subjects administered rifaximin to treat IBS have at least a 30% reduction in IBS-related abdominal discomfort, a stool consistency score of <4, and a average daily IBS score that is improved by at least 1.

New endpoints for IBS drug development are set forth below. For IBS with diarrhea, the new endpoint uses co-primary endpoints that include two of the major symptoms, abdominal pain and stool consistency (Table 3). These endpoints are designed to be more symptom-specific than global IBS construct endpoints and to address the common definition of IBS from Rome III as abdominal pain or discomfort that is improved by defecation.

**Table 3. Endpoints for IBS with Diarrhea**

| **Co-Primary endpoint** | **Entry criteria** | **Responder Definition** |
|---|---|---|
| **Pain Intensity** AND **Stool Consistency** | **Pain Intensity** | **Pain Intensity** |
| | Weekly average of worst abdominal pain in past 24 hours score of ≥ 3.0 in a 0 to 10 point score | Decrease in weekly average of worst abdominal pain in past 24 hours score of ≥ 30% compared with baseline |
| | **Stool Consistency** | **Stool Consistency** |
| | Weekly average ≥ Type 6 by the Bristol stool score. | Weekly average ≤ Type 5 by the Bristol stool score. |
| | | 'Classification as a responder involves achieving a prespecified improvement in symptoms at least 50 percent of the time.' |

| | | |
|---|---|---|
| Source: Guidance for Industry. Irritable bowel syndrome: Clinical evaluation of products for treatment. FDA Center for Drug Evaluation and Research (CDER) and Center for Biologies Evaluation and Research (CBER); March 2010. | | |

The endpoints set forth above can be used to determine the efficacy of treatment.

Also provided herein are methods of treating bacterial dysbiosis. Bacterial dysbiosis may be best viewed as a quantitative or qualitative imbalance which results in the symptoms of IBS, and not an infection per se. Epidemiologic, physiologic, and clinical evidence has emerged suggesting that dysbiosis of the GI microbiota occurs in the pathogenesis of IBS and may be a target for therapy. The GI microbiota in IBS patients have been shown to have less diversity and stability than in healthy subjects.

Epidemiological studies have strongly linked the development of IBS to previous experience with infectious GI events, such as TD or gastroenteritis; infectious diarrhea caused by Salmonella, Shigella, or campylobacter precedes IBS onset in up to 30% of patients that experience an acute event of infectious diarrhea. In these cases, the initial pathogen may result in lingering dysbiosis and a resulting low-grade inflammatory response. Additionally, IBS symptoms have been correlated to the presence of bacteria in the small intestine in quantities greater than those observed in healthy controls. Eradication or modulation of this bacterial overgrowth has also been shown to correlate with improvement in IBS symptoms.

Specific to the microbiome of the small intestine, there is evidence pointing to a role for SIBO in IBS. Increases in bacterial counts in the small intestine can lead to increased fermentation, gas production, and altered gut motility. The presence of SIBO has been shown to be prevalent in a large number of IBS patients and the symptoms of IBS are similar to the symptoms of SIBO, including bloating, abdominal discomfort, and diarrhea.

Evidence suggests that IBS may be linked to subtle qualitative changes in the gut microbiota. These changes may include the proliferation of species that produce more gas and short chain fatty acids, and are more active in the deconjugation of bile acids. The deconjugation of bile acids could profoundly affect colonic motility by changing water and electrolyte transport in the gut.

The interaction between altered gut flora and the gut mucosa in IBS patients may also be of importance. Evidence suggests that altered gut microbiota may lead to immune activation and inflammation in the colonic mucosa, which may promote or exacerbate the symptoms of IBS.

Sub-inhibitory concentrations of rifaximin are beneficial to the mucosa of the gut and therefore, treatment with rifaximin may be useful for treating subjects having IBS, wherein there is exacerbation of the IBS symptoms due to altered gut microbiota.

Accordingly, also provided herein are methods for treating a subject having altered microbiota in the gut, thereby treating IBS.

Subjects may be selected for treatment or retreatment of IBS with, for example, rifaximin based on the presence of one or more biomarkers that are indicative of IBS. For example, stress response biomarkers such as HPA axis; immune activation markers such as cytokines, mucosal lymphocytes, mucosal mast cells or proteases; fecal biomarkers such as calprotectin, human β-defensin, or fecal proteases can be used to select subjects for treatment or retreatment of IBS.

Additionally, identification of small intestinal bacterial overgrowth (SIBO) can be used as a marker for IBS. Techniques to identify SIBO include aspiration and direct culture of jejunal contents, and breath testing, *e*.*g*., lactulose hydrogen breath tests and glucose breath tests.

### Durability of Response (referential)

Embodiments relate to the discovery that the dosing regimens described herein of rifaximin results in a durability of response and amelioration of IBS symptoms in subjects in need thereof. Described is a method of treating bowel disease (BD) with a durability of antibiotic response, by administering a therapeutically effective amount of a rifamycin class antibiotic to a subject in need thereof, selecting subjects who respond to treatment after being treated for between about 1 and about 24 weeks, and removing a responding subject from treatment wherein after removal of treatment there is a durability of response. The selecting may be by a healthcare professional, by self-selection or by selection of one in a position to decide or discern symptoms or to diagnose a response to the antibiotic. Removal of treatment comprises, for example, ceasing to administer, ceasing to recommend administration of the antibiotic, and/or advising responding subjects to stop taking the antibiotic.

The recommendation (*e*.*g*., selection) may be made on a label of a pharmaceutical product, which indicates that the product should be administered for 14 days (*e*.*g*., two weeks). For example, a subject in need of treatment is administered rifaximin 550mg TID for two weeks and instructed by a label. In one embodiment, the recommendation (*e*.*g*., selection) is made on a label of a pharmaceutical product, which indicates that the product should be administered for two weeks. For example, a subject in need of treatment is administered rifaximin 550mg TID for two weeks as instructed by a label. In one embodiment, selecting is following dosing instructions on a package insert of a pharmaceutical product.

Also described herein are methods for maintenance of remission of bowel disease in a subject comprising administering a therapeutically effective amount of rifaximin for at least 25 weeks to a subject in need thereof.

Yet another aspect relates to a method of treating a subject (*e*.*g*., mammal, human, horse, dog, cat) with rifaximin who is in need thereof. Identifying a subject in need of such treatment can be in the judgment of a subject or a health care professional and can be subjective (*e*.*g*., opinion) or objective (*e*.*g*., measurable by a test or diagnostic method).

Rifaximin may be used in various treatment regimes. These regimes may vary depending upon the subject and the type of treatment.

Rifaximin may be administered, for example, twice a day, three times a day, or four times or more often as necessary per day. Rifaximin may be administered in doses, for example of from about between 25 mg BID to about 3000 mg TID. Rifaximin can be administered one, two, three, or four times a day in order to achieve the desired treatment. In exemplary embodiments, 15, 20, 25, 50, 100, 150, 200, 250, 300, 350, 400, 450, 500, 500, 550, 600, 650, 700 or 750 mg of rifaximin is administered one, two, three, or four times a day in order to achieve the desired treatment. Another example is administering rifaximin from between about 4.0 g/day to about 7.25 g/day. The rifaximin may be administered, for example, in tablet form, powered form, liquid for or in capsules.

Subjects in need thereof include subjects having or that are susceptible to BD, are in remission from BD, males and/or older subjects with long duration of disease, as disclosed further below.

As used herein, a therapeutically effective amount means an amount effective, when administered to a human or non-human subject, to provide a therapeutic benefit such as an amelioration of symptoms, *e*.*g*., an amount effective to decrease the symptoms of IBS, or maintenance of remission of a IBS.

In certain aspects, the rifaximin is administered to a subject from between about 1 week to about 6 weeks in duration, from between about 8 weeks to about 12 weeks in duration, or from between 1 day to about 7 days. The rifaximin may be administered from between about 1 day and about 1 year, or from 1 week to about 24 weeks. In specific embodiments, rifaximin is administered from 1, 2, 3, 4, 5, 6, 7, 8, 9, 10, 11, 12, 13, 14, 15, 16, 17, 18, 19, 20, 21 or more days. In exemplary embodiments, rifaximin is administered for 7 days, 10 days, or 14 days. The rifaximin may be administered, for example, for the remainder of a subject's life. The rifaximin may be administered intermittently or continuously during the course of treatment. Length of treatment may vary depending on the type and length of disease and the proper length of treatment may be easily determined by one of skill in the art having the benefit of this disclosure. In one aspect, the subject is administered rifaximin for 14 days.

For any of the aspects, rifaximin may be administered, for example, once daily, twice daily, three times daily, or four times daily (or more often as necessary for a particular subject) to a subject. In some embodiments, the methods comprise administering the rifaximin once daily to the subject because it may, for example, minimize the side effects and increase patient compliance. Also preferred, are twice and three times daily administration of rifaximin.

Dosages, according to certain preferred aspects, range from between about 50 to about 6000 mg of rifaximin administered daily. For example, a dose of 550 mg may be administered to a subject twice daily. Other appropriate dosages for methods described herein may be determined by health care professionals or by the subject. The amount of rifaximin administered daily may be increased or decreased based on the weight, age, health, sex or medical condition of the subject. One of skill in the art would be able to determine the proper dose for a subject based on this disclosure.

According to certain aspects, rifaximin may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. For example, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine, and/or loperamide.

In one aspect, subjects administered rifaximin for treatment of IBS have a sustained response. Specifically, for any four week period, a subject has a sustained response. For example, subjects with no recurrence are defined as having a stool consistency score of less than 4, abdominal pain reduced by at least 30 percent or both.

In specific aspects, at least 70, 75, 80, or 85% subjects being administered rifaximin have met the stool consistency endpoint for any rolling 4 week window of the trial and have no recurrence. Alternatively, less than 5, 4, 3, 2, or 1% of subjects have recurrence (*e.g.*, a stool consistency of greater than 4) during a 4 weeks period. In a further aspect, at least 65% of the subjects have a sustained durable response.

In another specific aspect, at least 35, 40, 45, 50, 55, 60, 65 or 70% subjects being administered rifaximin have met the abdominal pain endpoint for any rolling 4 week window of the trial. Alternatively, less than 5, 4, 3, 2, or 1% of subjects have recurrence (*e.g.*, a abdominal pain not reduced or reduced by less than 30%) during a 4 weeks period. In a further aspect, at least 35% of the subjects have a sustained durable response.

In another specific aspect, at least 30, 35, 40, 45, 50, 55, 60, 65 or 70% subjects being administered rifaximin have met the abdominal pain and stool consistency endpoint for any rolling 4 week window of the trial. In a further aspect, at least 30% of the subjects have a sustained durable response.

In other embodiments, provided herein are methods for treating small intestine bacteria overgrowth (SIBO) by treating a subject with rifaximin. In another embodiment, provided herein are methods for treating SIBO by altering the microbiome of the gut.

### Risk Selection Methods (referential)

Described are methods for genetically profiling for genetic risk of BD and selecting to treat an at risk subject. For example, an at-risk subject may be determined to be at risk of a bowel disease by genetic screening, family history, lifestyle, travel plans and the like. Genetic screening may, for example, be for genes and expression profiles or epigenetic modifiers shown to affect or predict bowel disease or susceptibility for bowel diseases. Mutations which may be screened for include mutations or polymorphisms in, for example, Nod2, CFTR, or CARD15. Nod2, a gene involved in the immune systems initial response to bacterial infection, significantly increases the risk of Crohn's disease. The CFTR protein resides in the surface of cells lining the digestive system, lungs and sweat glands. In normal cells, it acts as an ion channel that transports chloride into and out of cells. It also controls the regulation of other transport pathways regulating the passage of fluid and bicarbonate across cell membranes. DNA sequence variations (or mutations) alone do not explain CFTR-related gastrointestinal disease patterns; rather, epigenetic modifiers, or changes that leave the gene's sequence of DNA intact, influence CFTR expression.

For example, a subject may be typed for rs6822844 and/or rs2305767 to indicate risk of celiac disease. One study examined 778 individuals with celiac disease and 1,422 healthy controls. The authors found that each T at rs6822844 lowered subjects' risk of celiac disease by about 1.6 times. See Zhernakova A et al. (2007) "Novel association in chromosome 4q27 region with rheumatoid arthritis and confirmation of type 1 diabetes point to a general risk locus for autoimmune diseases." Am J Hum Genet 81(6): 1284-8; and van Heel DA et al. (2007) "A genome-wide association study for celiac disease identifies risk variants in the region harboring IL2 and IL21." Nat Genet 39(7):827-9. Another study examined 463 individuals with celiac disease and 686 healthy controls. The authors found that people with a C at both copies had 2.3 times lower odds for celiac disease than those with the TT genotype. See Hunt KA et al. (2006) "Lack of association of MYO9B genetic variants with coeliac disease in a British cohort." Gut 55(7):969-72; Núñez C et al. (2006) "No evidence of association of the MYO9B polymorphisms with celiac disease in the Spanish population." Tissue Antigens 68(6):489-92; Cirillo G et al. (2007) "Do MYO9B genetic variants predispose to coeliac disease? An association study in a cohort of South Italian children." Dig Liver Dis 39(3):228-31; Cirillo G et al. (2007) "Do MYO9B genetic variants predispose to coeliac disease? An association study in a cohort of South Italian children." Dig Liver Dis 39(3):228-31; Cirillo G et al. (2007) "Do MYO9B genetic variants predispose to coeliac disease? An association study in a cohort of South Italian children." Dig Liver Dis 39(3):228-31.

For example, a subject may be typed for 8q24 region; Marker:rs6983267 to determine risk for colon cancer. This SNP occurs in a hypothetical gene called LOC727677. It has been suggested that the riskier version of this SNP is associated not only with an increased risk of colorectal cancer, but also with formation of the precancerous adenomatous polyps. The riskier version of this SNP has also been linked to prostate cancer in some studies, though more research is needed to confirm this association. See Haiman et al. (2007) "A common genetic risk factor colorectal and prostate cancer." Nat Genet 39(8):954-6; and Tomlinson et al. (2007) "A genome-wide association scan of tag SNPs identifies a susceptibility variant for colorectal cancer at 8q24.21." Nat Genet 39(8):984-988; and Zanke et al. (2007) "Genome-wide association scan identifies a colorectal cancer susceptibility locus on chromosome 8q24." Nat Genet 39(8):989-994.

For example, a subject may be typed for NOD2(1) SNP: rs2066844; NOD2(2) SNP: rs2066845; NOD2(3) SNP: rs2066847; IL23R(1) SNP: rs11209026; NKX2-3 SNP: rs11190140; 5p13 region SNP: rs17234657; PTPN2 SNP: rs1893217; MST1 SNP: rs3197999; IRGM SNP: rs7714584; IL23R(2) SNP: rs11805303; and/or 10q21 region SNP: rs10761659 to determine risk of Crohn's disease. See Hugot et al. (2001) "Association of NOD2 leucine-rich repeat variants with susceptibility to Crohn's disease." Nature 411(6837):599-603; Ogura et al. (2001) "A frameshift mutation in NOD2 associated with susceptibility to Crohn's disease." Nature 411(6837):603-6; Rioux et al. (2007) "Genome-wide association study identifies new susceptibility loci for Crohn disease and implicates autophagy in disease pathogenesis." Nat Genet 39(5):596-604; Libioulle et al. (2007) "Novel Crohn's disease locus identified by genome-wide association maps to a gene desert on 5p13.1 and modulates expression of PTGER4." PLoS Genet 3(4):e58; Hampe et al. (2007) "A genome-wide association scan of non-synonymous SNPs identifies a susceptibility variant for Crohn disease in ATG16L1." Nat Genet 39(2):207-11; Duerr et al. (2006) "A genome-wide association study identifies IL23R as an inflammatory bowel disease gene." Science 314(5804):1461-1463; van Limbergen et al. (2007) "IL23R Arg381Gln is associated with childhood onset inflammatory bowel disease in Scotland." Gut 56(8):1173-4; Wellcome Trust Case Control Consortium (2007) "Genome-wide association study of 14,000 cases of seven common diseases and 3,000 shared controls." Nature 447(7145):661-78; Parkes et al. (2007) "Sequence variants in the autophagy gene IRGM and multiple other replicating loci contribute to Crohn's disease susceptibility." Nat Genet 39(7):830-2; Sheibanie et al. (2007) "The proinflammatory effect of prostaglandin E2 in experimental inflammatory bowel disease is mediated through the IL-23-->IL-17 axis." J Immunol 178(12):8138-47; Simoncic et al. (2007) "The T cell protein tyrosine phosphatase is a negative regulator of janus family kinases 1 and 3." Curr Biol 12(6):446-53; You-Ten et al. (1997) "Impaired bone marrow microenvironment and immune function in T cell protein tyrosine phosphatase-deficient mice." J Exp Med 22(16):5662-8; Barrett et al. (2008) "Genome-wide association defines more than 30 distinct susceptibility loci for Crohn's disease." Nat Genet 40(8):955-62; Goyette er al. (2008) "Gene-centric association mapping of chromosome 3p implicates MST1 in IBD pathogenesis" Mucosal Immunology 1:131-138; Barrett et al. (2008). "Genome-wide association defines more than 30 distinct susceptibility loci for Crohn's disease." Nat Genet 40(8):955-62; McCarroll et al (2008) "Deletion polymorphism upstream of IRGM associated with altered IRGM expression and Crohn's disease." Nat Genet 40(9):1107-1112; and Singh et al. (2006) "Human IRGM induces autophagy to eliminate intracellular mycobacteria." Science 313(5792):1438-41.

### Retreatment

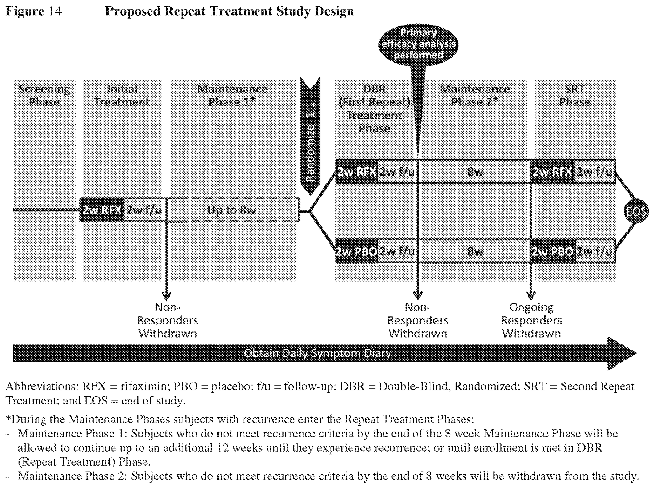

The inventors have developed a repeat treatment method based on a study that assesses the benefit of repeat treatment with rifaximin in IBS patients, as well as add to the existing evidence for rifaximin's efficacy and safety in this indication. The study is a multi-center, randomized, double-blind, placebo-controlled trial in adult subjects with non-C IBS confirmed using Rome III diagnostic criteria. The efficacy of repeat treatment with rifaximin 550 mg TID in subjects who responded to initial treatment with rifaximin is assessed. A study design is illustrated in Figure 14.

Accordingly, the invention relates to rifaximin for use in retreating a subject having diarrhea-predominant IBS (d-IBS), being a responder to an initial treatment of d-IBS with rifaximin and having a recurrence of abdominal pain or stool consistency for at least 3 weeks during a 4-week assessment period, comprising
- an initial treatment phase comprising a 2 weeks administration of 550 mg of rifaximin three times daily (TID) and a 2 weeks of treatment-free follow-up,
- a first treatment-free maintenance phase of up to 8 to 12 weeks,
- a first repeat treatment phase comprising a 2 weeks administration of 550 mg of rifaximin TID and a 2 weeks of treatment-free follow-up,
- a second treatment-free maintenance phase of up to 8 weeks,
- a second repeat treatment phase comprising a 2 weeks administration of 550 mg of rifaximin TID and a 2 weeks treatment-free follow-up.

An efficacy measure is treatment success for abdominal pain AND stool consistency. Response is defined as subjects who experience treatment success for IBS-related abdominal pain AND stool consistency for at least 2 out of 4 weeks during a 4-week assessment period, for at least 3 weeks during a 4-week assessment period, for at least 3 weeks during a 5-week assessment period, for at least 2 weeks during a 6-week assessment period, for at least 3 weeks during a 6-week assessment period, for at least 4 weeks during a 6-week assessment period, for at least 3 weeks during a 8-week assessment period, for at least 4 weeks during a 8-week assessment period, for at least 5 weeks during a 8-week assessment period, or for at least 6 weeks during a 8-week assessment period. A subject will be considered to have met recurrence criteria when treatment success of abdominal pain AND stool consistency is absent for at least 2 weeks during a 4-week assessment period, for at least 3 weeks during a 4-week assessment period, for at least 3 weeks during a 5-week assessment period, for at least 2 weeks during a 6-week assessment period, for at least 3 weeks during a 6-week assessment period, for at least 4 weeks during a 6-week assessment period, for at least 3 weeks during a 8-week assessment period, for at least 4 weeks during a 8-week assessment period, for at least 5 weeks during a 8-week assessment period, or for at least 6 weeks during a 8-week assessment period (an alternate possibility for the definition of recurrence will be the absence of treatment success of abdominal pain AND stool consistency for any 2 consecutive weeks, any 3 consecutive weeks, any 4 consecutive weeks, or any 5 consecutive weeks).

The total study duration can be approximately 20, 24, 28, 30, 32, 36, 40, 44, or 48 weeks, depending on whether a colonoscopy is required.

The study consists of the following phases:
- Screening Phase (up to 30 days) - Potential subjects undergo screening assessments including a colonoscopy, if necessary, and complete a Diary Eligibility Period of at least 7 days. During the Diary Eligibility Period, subjects are required to respond to daily IBS symptom related questions.
- Initial Treatment Phase (4 weeks) - Eligible subjects receive a 2 week course of rifaximin 550 mg TID, with 2 weeks of treatment-free follow-up. At the end of this initial treatment and follow-up phase, subjects are assessed for response. Subjects who are responders are entered into a treatment- free maintenance phase (i.e., Maintenance Phase 1) whereas non-responders are withdrawn from the study.
- Maintenance Phase 1 - This phase is variable in duration for subjects, depending on whether or not there is a recurrence of IBS symptoms. Subjects are continually assessed for ongoing response as well as recurrence of IBS symptoms starting after 2 weeks in Maintenance Phase 1. Subjects who meet the criteria for recurrence are entered into the Double-Blind, Randomized (first repeat) Treatment) Phase. Subjects who do not meet recurrence criteria by the end of Maintenance Phase 1 are allowed to continue up to an additional 12, 14, 16, 18, 20, 22, 24, 26, 28, 30, 32, 34, 36 or 38 weeks until they either experience recurrence; or until enrollment is met in the Double-Blind, Randomized (first repeat) Treatment Phase.
- Double-Blind, Randomized (first repeat) Treatment Phase and Interim Analysis - In this phase, subjects who experienced recurrence during Maintenance Phase 1 are randomized 1:1 to receive rifaximin 550 mg TID or placebo TID for 2 weeks with a 2 week treatment-free follow-up.
- Maintenance Phase 2 - Responders in the Double-Blind, Randomized (first repeat) Treatment Phase are eligible for Maintenance Phase 2 and continue with an additional treatment-free follow-up period of up to 8, 10, 12, 14, 16, 18, 20, 22, or 24 weeks. Subjects who experience recurrence are immediately transitioned into the Second Repeat Treatment Phase. Subjects who do not meet recurrence criteria by the end of a 8, 10, 12, 14, 16, 18 or 20-week Maintenance Phase 2 are withdrawn from the study.
- Second Repeat Treatment Phase and End of Study - Subjects with recurrence in Maintenance Phase 2 are eligible to enter the Second Repeat Treatment Phase, and receive a second repeat treatment of rifaximin 550 mg TID or placebo TID for 2, 3, or 4 weeks with a 2, 3, or 4 week treatment-free follow up. The treatment assignment from the Double-Blind, Randomized (first repeat) Treatment Phase is maintained in this phase. At the end of this phase, subjects undergo end of study assessments.

Patients selected for inclusion meet the Rome III diagnostic criteria for IBS-D. The Rome III criteria are the accepted current standard for diagnosing IBS in the clinical setting and are consistent with FDA guidance. Table 4 outlines the criteria for diagnosing and subtyping IBS using Rome III.

Additionally, during the diary eligibility period:
- An average score ≥ 3 for abdominal pain (Scale: 0-10, with 0 indicating no pain, and 10 indicating the worst imaginable pain).
- An average score ≥ 3 for bloating (Scale: 0-6, ranking how bothersome IBS-related bloating was in the last 24 hours, 0 = not at all; 1 = hardly; 2 = somewhat; 3 = moderately; 4 = a good deal; 5 = a great deal; 6 = a very great deal.")
- A score of 6 or greater for stool consistency using the Bristol Stool form Scale for at least 2 out of 7 days

Subjects record IBS symptoms in an IVRS during screening to confirm eligibility and will have had a colonoscopy within the last 2 years to rule out inflammatory bowel diseases or other causes of IBS symptoms. Other confounding medical conditions and medications are excluded by qualified healthcare professionals.

**Table 4. Rome III: IBS Diagnosis and Subtyping**

| **Rome III Criteria** |
|---|
| 1. Recurrent abdominal pain or discomfort at least 3 days per month in the last 3 months, with symptom onset at least 6 months prior to diagnosis associated with 2 or more of the following: improvement with defecation; onset associated with a change in frequency of stool; and/or onset associated with a change in form (appearance) of stool. |

| **2. Rome III Subtyping** |
|---|
| 1. IBS with constipation (IBS-C) - hard or lumpy stools^{a} ≥ 25% and loose (mushy) or watery stools^{b} < 25% of bowel movements, in the absence of use of antidiarrheals or laxatives. |
| 2. IBS with diarrhea (IBS-D) - loose (mushy) or watery stools^{b} ≥ 25% and hard or lumpy stool^{a} < 25% of bowel movements, in the absence of use of antidiarrheals or laxatives. |
| 3. Mixed IBS (IBS-M) - hard or lumpy stools^{a} ≥ 25% and loose (mushy) or watery stools^{b} ≥ 25% of bowel movements, in the absence of use of antidiarrheals or laxatives. |
| 4. Unsubtyped IBS (IBS-U) - insufficient abnormality of stool consistency to meet criteria for IBS-C, -D or -M. |

| |
|---|
| References: Ersryd et al., Corazziari et al.,and Thompson et al a. Bristol Stool Form Scale 1-2 [separate hard lumps like nuts (difficult to pass) or sausage shaped but lumpy]. b. Bristol Stool Form Scale 6-7 (fluffy pieces with ragged edges, a mushy stool or watery, no solid pieces, entirely liquid). |

### Exemplary Repeat Treatment Study

An objective of this study is to evaluate the efficacy of repeat treatment with rifaximin 550 mg TID for 2 weeks in subjects with non-C IBS who responded to an initial course of rifaximin treatment and subsequently experienced recurrence.

An endpoint is the proportion of subjects who are responders to repeat treatment in both IBS-related abdominal pain AND stool consistency during the, for example, 2 weeks treatment; 2-week treatment-free follow-up during the Double-Blind, Randomized (first repeat) Treatment Phase.

Weekly response for the primary endpoint is defined based on IBS-symptom related questions, as follows:
- Weekly treatment success in IBS-related abdominal pain is defined as a 30% or greater improvement from baseline in the weekly average abdominal pain score, based on subject response to a daily question, for example:
   "In regards to your specific IBS symptom of abdominal pain, on a scale of 0-10, what was your worst IBS-related abdominal pain in the last 24 hours? 'Zero' means you have no pain at all; `Ten' means the worst possible pain you can imagine."
- Weekly treatment success in stool consistency is achieved when a subject has 50% reduction in the number of stools scored at ≥ 6 over 7 days as compared to baseline based on subject response to the following daily question based on the Bristol Stool Form Scale:
   "On a scale of 1-7, what was the overall form of your bowel movements in the last 24 hours? 1 = Separate hard lumps, like nuts (hard to pass); 2 = Sausage-shaped but lumpy; 3 = Like a sausage but with cracks on its surface; 4 = Like a sausage or snake, smooth and soft; 5 = Soft blobs with clear cut edges (passed easily); 6 = Fluffy pieces with ragged edges, a mushy stool; 7 = Watery stool, no solid pieces; entirely liquid."

Secondary Endpoints for the study, during the Double-blind, Randomized (first repeat) Treatment phase, are as follows:
- The proportion of subjects who are responders during the 2-weeks treatment; 2-weeks treatment-free follow-up periods for the following: IBS-related abdominal pain; stool consistency; IBS-related bloating; and IBS symptoms (daily reported).
- Change from baseline to each week for the following: abdominal pain (11 point scoring system, see above); stool consistency (7-point scoring system, Bristol Stool form Scale, see above); Bloating (7-point scoring system); IBS symptoms (7-point scoring system); sense of urgency (based on a Yes/No diary question).
- The number of recurrent events relative to person-time on study in IBS-related abdominal pain and stool consistency during the Double-Blind, Randomized (first repeat) Treatment Phase and through the follow-up 8-week Maintenance phase.

Weekly treatment success for IBS-related bloating is assessed using a question similar to: "In regards to your specific IBS symptom of bloating, on a scale of 0-6, how bothersome was your IBS-related bloating in the last 24 hours? 0 = not at all; 1 = hardly; 2 = somewhat; 3 = moderately; 4 = a good deal; 5 = a great deal; 6 = a very great deal." Treatment success for bloating is achieved when a subject rates his/her daily IBS-related bloating as either: 0 (not at all) or 1(hardly) at least 50% of the days in a given week; OR 0 (not at all), 1 (hardly) or 2 (somewhat) 100% of the days in a given week

Weekly treatment success for IBS symptoms (daily reported) is assessed using the following question: "In regards to all of your symptoms of IBS, on a scale of 0-6, how bothersome were your symptoms of IBS in the last 24 hours? 0 = not at all; 1 = hardly; 2 = somewhat; 3 = moderately; 4 = a good deal; 5 = a great deal; 6 = a very great deal." Treatment success for IBS symptoms is achieved when a subject rates his/her daily IBS symptoms as either: 0 (not at all) or 1(hardly) at least 50% of the days in a given week; OR 0 (not at all), 1 (hardly) or 2 (somewhat) 100% of the days in a given week.

Planned Exploratory Endpoints for the study include the following:
- Descriptive characterization of the proportion of responders (yes/no) on rifaximin after the Double-Blind, Randomized (first repeat) Treatment Phase versus their response profile (yes/no) in the Second Repeat Treatment Phase.
- Biomarker assessments will be performed during the study.

### Pharmaceutical Preparations (referential)

Described are pharmaceutical compositions, comprising an effective amount of a rifamycin class antibiotic (e.g., rifaximin or a rifaximin polymorph) described herein and a pharmaceutically acceptable carrier. In a further embodiment, the effective amount is effective to treat a bacterial infection, e.g., small intestinal bacterial overgrowth, Crohn's disease, hepatic encephalopathy, antibiotic associated colitis, and/or diverticular disease.

For examples of the use of rifaximin and formulations thereof to treat Travelers' diarrhea, see Infante RM, Ericsson CD, Zhi-Dong J, Ke S, Steffen R, Riopel L, Sack DA, DuPont, HL. Enteroaggregative Escherichia coli Diarrhea in Travelers: Response to Rifaximin Therapy. Clinical Gastroenterology and Hepatology. 2004;2:135-138; and Steffen R, M.D., Sack DA, M.D., Riopel L, Ph.D., Zhi-Dong J, Ph.D., Sturchler M, M.D., Ericsson CD, M.D., Lowe B, M.Phil., Waiyaki P, Ph.D., White M, Ph.D., DuPont HL, M.D. Therapy of Travelers' Diarrhea With Rifaximin on Various Continents. The American Journal of Gastroenterology. May 2003, Volume 98, Number 5.

One exemplary pharmaceutical compositions comprising rifaximin or any polymorphic form thereof and a pharmaceutically acceptable carrier. That is, formulations may contain only one polymorph or may contain a mixture of more than one polymorph. Polymorph, in this context, refers to any physical form, hydrate, acid, salt or the like of rifaximin. Mixtures may be selected, for example on the basis of desired amounts of systemic adsorption, dissolution profile, desired location in the digestive tract to be treated, and the like. The pharmaceutical composition further comprises excipients, for example, one or more of a diluting agent, binding agent, lubricating agent, disintegrating agent, coloring agent, flavoring agent or sweetening agent. Compositions may be formulated for selected coated and uncoated tablets, hard and soft gelatin capsules, sugar-coated pills, lozenges, wafer sheets, pellets and powders in sealed packet. For example, compositions may be formulated for topical use, for example, ointments, pomades, creams, gels and lotions.

In an aspect, the rifaximin is administered to the subject using a pharmaceutically-acceptable formulation, e.g., a pharmaceutically-acceptable formulation that provides sustained delivery of the rifaximin to a subject for at least 12 hours, 24 hours, 36 hours, 48 hours, one week, two weeks, three weeks, or four weeks after the pharmaceutically-acceptable formulation is administered to the subject.

In certain aspects, these pharmaceutical compositions are suitable for topical or oral administration to a subject. In other aspects, as described in detail below, the pharmaceutical compositions described herein may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "pharmaceutically acceptable" refers to rifaximin described herein, compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" includes pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxy toluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Compositions containing rifaximin include those suitable for oral, nasal, topical (including buccal and sublingual), rectal, vaginal, aerosol and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 1% to about 99 % of active ingredient, preferably from about 5 % to about 70 %, most preferably from about 10 % to about 30 %.

Liquid dosage forms for oral or rectal administration of the rifaximin include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

In addition to inert diluents, the oral compositions can include adjuvants such as wetting agents, emulsifying and suspending agents, sweetening, flavoring, coloring, perfuming and preservative agents.

Suspensions, in addition to the active rifaximin may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions for rectal or vaginal administration may be presented as a suppository, which may be prepared by mixing one or more rifaximin with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent. Compositions which are suitable for vaginal administration can include pessaries, tampons, creams, gels, pastes, foams or spray formulations containing such carriers as are known in the art to be appropriate.

Dosage forms for the topical or transdermal administration of rifaximin can include powders, sprays, ointments, pastes, creams, lotions, gels, solutions, patches and inhalants. The active rifaximin may be mixed under sterile conditions with a pharmaceutically-acceptable carrier, and with any preservatives, buffers, or propellants which may be required.

The ointments, pastes, creams and gels may contain, in addition to rifaximin, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to rifaximin, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The rifaximin can be alternatively administered by aerosol. This is accomplished, for example, by preparing an aqueous aerosol, liposomal preparation or solid particles containing the compound. A non-aqueous (e.g., fluorocarbon propellant) suspension could be used. Sonic nebulizers are preferred because they minimize exposing the agent to shear, which can result in degradation of the compound.

Examples of suitable aqueous and non-aqueous carriers which may be employed in the pharmaceutical compositions can include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, to prolong the effect of a drug, it is desirable to alter the absorption of the drug. This may be accomplished by the use of a liquid suspension of crystalline, salt oramorphous material having poor water solubility. The rate of absorption of the drug may then depend on its rate of dissolution which, in turn, may depend on crystal size and crystalline form. Alternatively, delayed absorption of a drug form is accomplished by dissolving or suspending the drug in an oil vehicle.

When the rifaximin is administered as pharmaceuticals, to humans and animals, they can be given per se or as a pharmaceutical composition containing, for example, 0.1 to 99.5% (more preferably, 0.5 to 90%) of active ingredient in combination with a pharmaceutically-acceptable carrier.

Regardless of the route of administration selected, the rifaximin, which may be used in a suitable hydrated form, and/or the pharmaceutical compositions, are formulated into pharmaceutically-acceptable dosage forms by conventional methods known to those of skill in the art.

Actual dosage levels and time course of administration of the active ingredients in the pharmaceutical compositions may be varied so as to obtain an amount of the active ingredient which is effective to achieve the desired therapeutic response for a particular patient, composition, and mode of administration, without being toxic to the patient. An exemplary dose range is from 25 to 3000 mg per day.

In combination therapy treatment, both the compounds and the other drug agent(s) are administered to mammals (e.g., humans, male or female) by conventional methods. The agents may be administered in a single dosage form or in separate dosage forms. Effective amounts of the other therapeutic agents are well known to those skilled in the art. However, it is well within the skilled artisan's purview to determine the other therapeutic agent's optimal effective-amount range. In one embodiment in which another therapeutic agent is administered to an animal, the effective amount of the compound is less than its effective amount in case the other therapeutic agent is not administered. In another embodiment, the effective amount of the conventional agent is less than its effective amount in case the compound is not administered. In this way, undesired side effects associated with high doses of either agent may be minimized. Other potential advantages (including without limitation improved dosing regimens and/or reduced drug cost) will be apparent to those skilled in the art.

In various embodiments, the therapies (*e.g*., prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more therapies are administered within the same patient's visit.

In certain aspects, one or more of the rifamycin class antibiotic (*e.g*., rifaximin) and one or more other therapies (*e.g*., prophylactic or therapeutic agents) are cyclically administered. Cycling therapy involves the administration of a first therapy (*e.g.,* a first prophylactic or therapeutic agent) for a period of time, followed by the administration of a second therapy (*e.g.,* a second prophylactic or therapeutic agent) for a period of time, optionally, followed by the administration of a third therapy (*e.g*., prophylactic or therapeutic agent) for a period of time and so forth, and repeating this sequential administration, *i.e.*, the cycle in order to reduce the development of resistance to one of the therapies, to avoid or reduce the side effects of one of the therapies, and/or to improve the efficacy of the therapies.

In certain aspects, the administration of the same compounds may be repeated and the administrations may be separated by at least about 1 day, 2 days, 3 days, 5 days, 10 days, 15 days, 30 days, 45 days, 3 weeks, 4 weeks, 5 weeks, 6 weeks, 12 weeks, 2 months, 75 days, 3 months, or at least 6 months. In other embodiments, the administration of the same therapy (*e.g*., prophylactic or therapeutic agent) other than rifaximin may be repeated and the administration may be separated by at least at least 1 day, 2 days, 3 days, 5 days, 10 days, 15 days, 30 days, 45 days, 2 months, 75 days, 3 months, or at least 6 months. In one embodiment, a label on a rifamycin class antibiotic may instruct, for example, do not repeat more often than every 6 weeks. In another embodiment, a label on a rifamycin class antibiotic may instruct, for example, do not repeat more often than every 3 weeks. In another embodiment, a label on a rifamycin class antibiotic may instruct, for example, do not repeat more often than every 3 - 12 weeks. Included within ranges given herein for dosage or administration are any value within the range.

In certain aspects, retreatment is efficacious in combination with the methods disclosed herein. See for example, Rifaximin versus Other Antibiotics in the Primary Treatment and Retreatment of Bacterial Overgrowth in IBS, Janet Yang, Hyo-Rang Lee, Kimberly Low, Soumya Chatterjee, and Mark Pimentel, Dig Dis Sci (2008) 53:169-174. For example, methods as described herein may further comprise determining symptom relief in a subject and administering a second course of rifaximin treatment if symptoms remain unresolved. Methods may also further comprise, for example, determining the gender of a subject and administering the therapeutically effective amount to a male subject.

Certain indications may require longer treatment times. For example, travelers' diarrhea treatment may only last from between about 12 hours to about 72 hours, while a treatment for Crohn's disease may be from between about 1 day to about 3 months and a treatment for hepatic encephalopathy may be from between 1 day and 12 months. For example, HE may require chronic therapy for the remainder of a subject's life. Crohn's disease subjects may also require chronic therapy.

### Kits (referential)

Kits are also described herein, for example, kits for treating a bowel disorder in a subject treating bowel disease (BD) with a durability of antibiotic response; methods of treating bowel disease (BD) in females methods of treating bowel disease (BD) in males; methods of treating bloating due to BD in males; methods of treating bloating due to BD; methods of treating non-white subjects having BD; and/or methods of treating BD in older subjects; methods of treating BD in older subjects with long duration of disease; and/or methods of predicting response to rifaximin treatment for BD. The kits may contain, for example, a polymorph or amorphous form of rifaximin and instructions for use. The instructions for use may contain prescribing information, dosage information, storage information, and the like.

Kits are also described herein, for example, kits for treating IBS-D in a subject; methods of treating IBS-D in females; methods of treating IBS-D in males; methods of treating bloating due to IBS-D in males; methods of treating bloating due to IBS-D. The kits may contain, for example, a polymorph or amorphous form of rifaximin and instructions for use. The instructions for use may contain prescribing information, dosage information, storage information, and the like.

In addition, described herein are kits for retreatment of IBS in a subject who has previously suffered from IBS. In some aspects, the subject has responded well to treatment of previously-suffered IBS with administration of rifaximin. The kits may contain, for example, a polymorph or amorphous form of rifaximin and instructions for use. The instructions for use may contain prescribing information, dosage information, storage information, and the like.

Also described herein are kits for reducing the incidence of commonly-occurring infections in a subject having HE. The kits may contain, for example, a polymorph or amorphous form of rifaximin and instructions for use. The instructions for use may contain prescribing information, dosage information, storage information, and the like.

Also described herein are kits for treating a *C*. *difficile* infection or for treating CDI associated symptoms in a subject. The kits may contain, for example, a polymorph or amorphous form of rifaximin and instructions for use. The instructions for use may contain prescribing information, dosage information, storage information, and the like.

In one aspect, the label describes adverse events comprising one or more of infections and infestations, gastrointestinal disorders, nervous system disorders, and musculoskeletal and connective tissue disorders.

In one aspect, the label describes a length of treatment with the rifamycin class antibiotic, whereby a subject is selected as responding to treatment if a healthcare professional prescribes the rifamycin class antibiotic according to the label instructions.

In one aspect, the label describes a length of treatment with the rifamycin class antibiotic, whereby a subject is removed from treatment if a healthcare professional prescribes the rifamycin class antibiotic according to the label instructions.

Label instructions can include, for example, instructions to take the rifamycin class antibiotic for 14 days for the treatment of IBS. The instructions could also read, for example, take for 1650 mg/day of rifaximin for 14 days for acute treatment of Irritable Bowel Syndrome (IBS).

Label instructions may also include instructions that a higher percentage of non-white subjects, female subjects and subjects 65 years of age or older have an adequate relief of IBS symptoms and/or adequate relief of IBS symptom of bloating.

Packaged compositions are also described, and may comprise a therapeutically effective amount of one or more of a one or more of an amorphous form, Form α, Form β, Form γ, Form 6, Form ε, Form ζ, Form mu, Form omicron, Form xi, Form kappa, Form iota, Form lambda or Form η polymorph of rifaximin of rifaximin and a pharmaceutically acceptable carrier or diluent, wherein the composition is formulated for treating a subject suffering from or susceptible to a bowel disorder, and packaged with instructions to treat a subject suffering from or susceptible to a bowel disorder.

In some aspects, rifaximin is administered as a soluble solid dispersion. For example, rifaximin can be administered at between about 25 - 550 mg of soluble solid dispersion of rifaxmin.

### EXAMPLES

It should be appreciated that embodiments should not be construed to be limited to the example, which is now described.

### EXAMPLE 1

### (reference example)

This example relates to a study of rifaximin in subjects with d-IBS. Subjects received daily one of BID doses of placebo, rifaximin 275 mg, 550 mg, or 1100 mg for 14 days. A fifth group of subjects received rifaximin 550 mg BID for a period of 28 days. There were two measures of efficacy assessed. Subjects were questioned on the relief of overall IBS symptoms and bloating. Adequate relief of IBS related symptoms (SGA) and IBS-related bloating (IBS-B) were assessed, and a dose of 550 mg BID for 2 weeks demonstrated statistically significant relief. The analyses defined success as a "yes" response to questions regarding adequate relief.

Predictors of response analyses showed that the response was similar across some subgroups however, there were differences. Analyses on predictors of response demonstrated that age (older subjects and those with a longer IBS duration); sex (males) and baseline severity (mild to moderate symptoms) were predictors of response. All subpopulations in the study responded to therapy. Baseline severity was determined using 7-point Lickert scales during screening for Abdominal Pain/Discomfort and Bloating, and the number, type (normal, hard, loose) and urgency of bowel movements.

Duration of effect was assessed in a follow-up period. Subjects that responded in the 4 week treatment period were followed for an additional 3 months. The subjects in the placebo group had a greater rate of decline in response than the 550 mg BID 2 week group, demonstrating that subjects treated with rifaximin had a better chance of maintaining symptom relief than their placebo treated counterparts.

### Percentage of Subjects with Adequate Relief of IBS and Bloating Symptoms

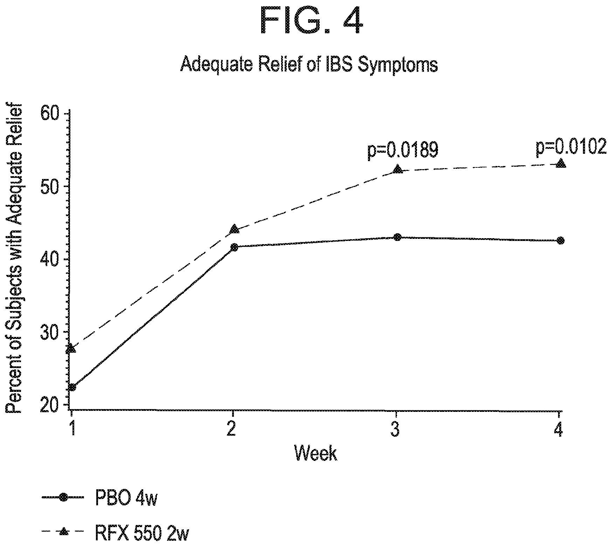

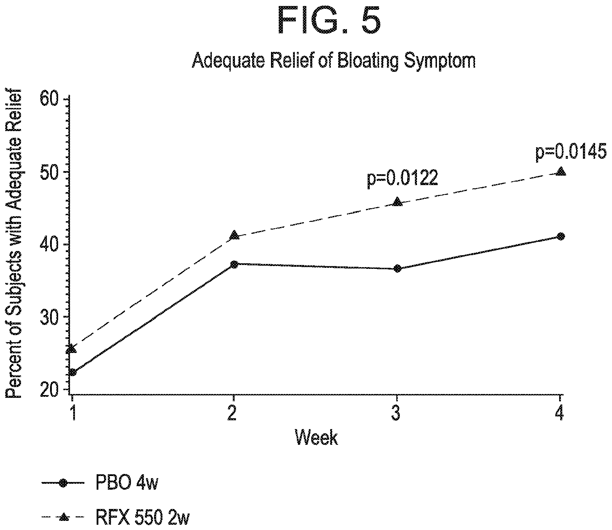

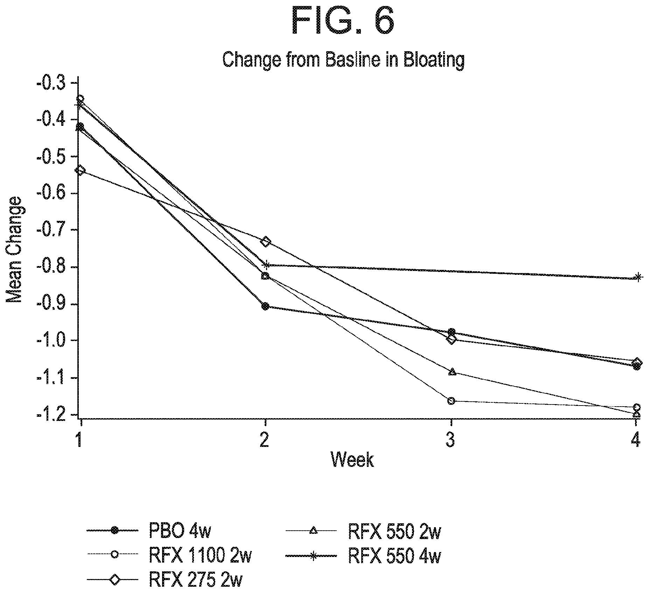

The effect of treatment on the percentage of subjects who reported adequate relief of IBS and bloating symptoms for at least two of the final three weeks during the treatment phase (Weeks 1 to 4) is shown in Tables 5-7 and Figures 4-6 below.

During the treatment phase, 52.4 % of subjects on RFX 550 mg BID met the IBS symptoms responder criterion, compared with 44.2% of the placebo subjects (odds ratio of 1.6 and p value = 0.0314). Similarly, 46.1% of subjects in the 550 mg BID group met the bloating symptom responder criterion, compared with 39.6% of the placebo group (odds ratio of 1.6 and p value = 0.0402).

**Table 5: Percentage of Subjects with Adequate Relief of IBS and Bloating Symptoms - ITT Population**

| **Measure** | **Placebo [N=197]** | **RFX 550 mg BID [N=191]** | **Odds Ratio Estimate (95% CI)** | **P-Value** |
|---|---|---|---|---|
| IBS Symptoms | 44.2% | 52.4% | 1.60 (1.04, 2.45) | 0.0314 |
| Bloating Symptom | 39.6% | 46.1% | 1.58 (1.02, 2.45) | 0.0402 |

**Table 6: Percentage of Subjects with Number of Weeks with Adequate Relief of IBS Symptoms - ITT Population**

| | **Placebo [N=197]** | **RFX 550 mg BID [N=191]** | **Odds Ratio Estimate** | **P-Value** |
|---|---|---|---|---|
| Number of Weeks - IBS Symptoms | | | 1.54 (1.07, 2.24) | 0.0216 |
| 0 | 45% | 33% | | |
| 1 | 7% | 11% | | |
| 2 | 15% | 15% | | |
| 3 | 16% | 23% | | |
| 4 | 15% | 16% | | |

**Table 7: Percentage of Subjects with Number of Weeks with Adequate Relief of Bloating Symptoms - ITT Population**

| | **Placeb o [N=19 7]** | **RFX 550 mg BID [N=191]** | **Odds Ratio Estimate** | **P-Value** |
|---|---|---|---|---|
| Number of Weeks -Bloating Symptom | | | 1.57 (1.08, 2.29) | 0.0182 |
| 0 | 47% | 35% | | |
| 1 | 10% | 14% | | |
| 2 | 12% | 15% | | |
| 3 | 15% | 20% | | |
| 4 | 13% | 14% | | |

### Daily Symptom Score

Subjects recorded the following information on d-IBS symptoms daily throughout the duration of the study:
- Number of normal stools/day;
- Number of hard and lumpy stools/day;
- Number of loose or watery stools/day;
- Number of loose or watery stools/day with the symptom of urgency;
- How bothersome is abdominal pain and discomfort? [7-point response scale: 0 (not at all) to 6 (a very great deal)];
- How bothersome is bloating? [(7-point response scale: 0 (not at all) to 6 (a very great deal)].

Changes from baseline variables were computed for each weekly summary score.

### Long Term Follow-up of Adequate Relief

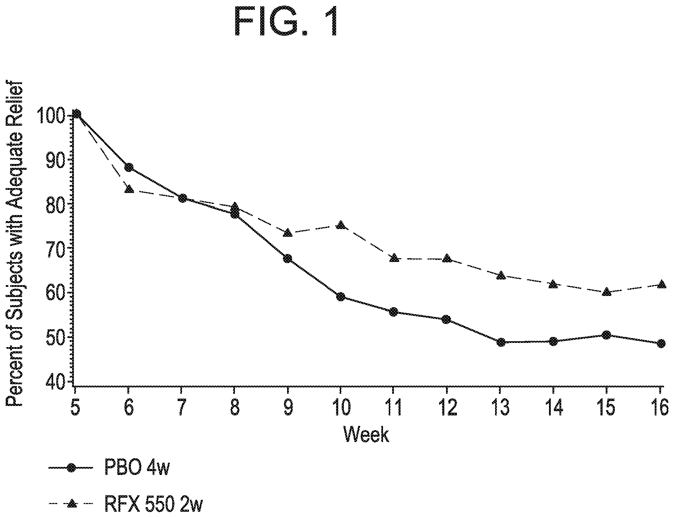

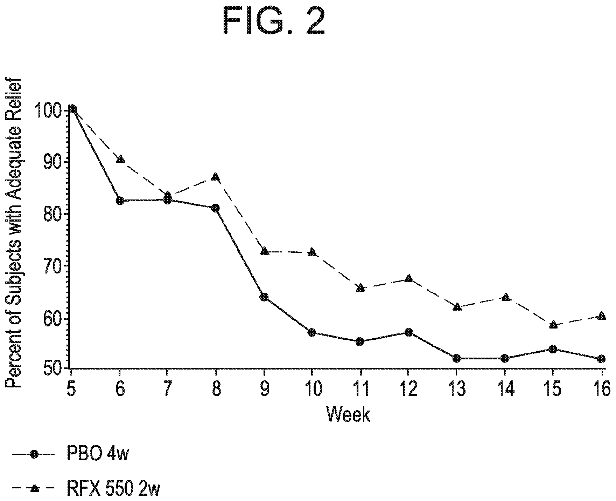

The study assessed the effect over 12 weeks of follow-up on long-term adequate relief. Subjects who had adequate relief by Week 4 and remained symptom-free at Week 5 were followed during the post-treatment phase and shown in Figures 1 and 2. Superiority to placebo was maintained during the 12 weeks post-treatment follow-up. Results for IBS symptoms were RFX 550 mg BID 62.3% versus placebo 49.2%, and RFX 550 mg BID 59.3% versus 50.9% for placebo for the symptom of bloating through week 16. In assessing the follow-up data, there was statistical significance (p<0.05) of bloating and IBS symptoms for RFX 550 mg BID versus placebo.

**Table 8.**

| | Adequate Relief of Bloating, Post Treatment | | PBO4w | RFX 550 2w |
|---|---|---|---|---|
| | | | (N=57) | (N=54) |
| | | | n (%) | n (%) |
| | Week 6 | Success | 47 (82.5%) | 49 (90.7%) |
| | | Failures | 10 (17.5%) | 5 (9.3%) |
| Comparison of RFX 550 2w vs. PBO p-value: 0.1303, odds ratio: 3.840 (0.672, 21.95) | | | | |
| | Week 7 | Success | 47 (82.5%) | 45 (83.3%) |
| | | Failures | 10 (17.5%) | 9 (16.7%) |
| Comparison of RFX 550 2w vs. PBO p-value: 0.1311, odds ratio: 2.931 (0.726, 11.84) | | | | |
| | Week 8 | Success | 46 (80.7%) | 47 (87.0%) |
| | | Failures | 11 (19.3%) | 7 (13.0%) |
| Comparison of RFX 550 2w vs. PBO p-value: 0.2858, odds ratio: 2.107 (0.536, 8.276) | | | | |
| | Week 9 | Success | 36 (63.2%) | 39 (72.2%) |
| | | Failures | 21 (36.8%) | 15 (27.8%) |
| Comparison of RFX 550 2w vs. PBO p-value: 0.0814, odds ratio: 2.737 (0.882, 8.492) | | | | |
| | Week 10 | Success | 32 (56.1%) | 39 (72.2%) |
| | | Failures | 25 (43.9%) | 15 (27.8%) |
| Comparison of RFX 550 2w vs. PBO p-value: 0.0217, odds ratio: 3.828 (1.217, 12.04) | | | | |
| | Week 11 | Success | 31 (54.4%) | 35 (64.8%) |
| | | Failures | 26 (45.6%) | 19 (35.2%) |
| Comparison of RFX 550 2w vs. PBO p-value: 0.0398, odds ratio: 3.115 (1.054, 9.205) | | | | |

**Table 9.**

| Adequate Relief of Bloating, Post Treatment | | PBO 4w | RFX 550 2w |
|---|---|---|---|
| | | (N=57) | (N=54) |
| | | n (%) | n (%) |
| Week 12 | Success | 32 (56.1%) | 36 (66.7%) |
| | Failure | 25 (43.9%) | 18 (33.3%) |
| Comparison of RFX 550 2w vs. PBO p- | | | |
| value: 0.0596, odds ratio: 2.891 (0.958, 8.726) | | | |
| Week 13 | Success | 29 (50.9%) | 33 (61.1%) |
| | Failure | 28 (49.1%) | 21 (38.9%) |
| Comparison of RFX 550 2w vs. PBO p- | | | |
| value: 0.0142, odds ratio: 4.187 (1.333, 13.15) | | | |
| Week 14 | Success | 29 (50.9%) | 34 (63.0%) |
| | Failure | 28 (49.1%) | 20 (37.0%) |
| Comparison of RFX 550 2w vs. PBO p- | | | |
| value: 0.0121, odds ratio: 4.230 (1.372, 13.05) | | | |
| Week 15 | Success | 30 (52.6%) | 31 (57.4%) |
| | Failure | 27 (47.4%) | 23 (42.6%) |
| Comparison of RFX 550 2w vs. PBO p- | | | |
| value: 0.0391, odds ratio: 3.323 (1.062, 10.40) | | | |
| Week 16 | Success | 29 (50.9%) | 32 (59.3%) |
| | Failure | 28 (49.1%) | 22 (40.7%) |
| Comparison of RFX 550 2w vs. PBO p- | | | |
| value: 0.0212, odds ratio: 3.700 (1.216, 11.25) | | | |

**Table 10. Baseline Disease Characteristics Across All Treatment Groups**

| Daily Symptom | Median | Min, Max |
|---|---|---|
| Total No. bowel movements/day | 3.0 | 1, 15 |
| No. loose/watery bowel movements/day | 2.0 | 0, 10 |
| No. loose/waterv with urgency | 1.6 | 0, 10 |
| Abdominal pain/discomfort* | 3.4 | 0, 6 |
| Bloating* | 3.4 | 0, 6 |

| | | |
|---|---|---|
| * 7 pt. scale asking "How bothersome..." [0= not at all to 6= a very great deal] | | |

Two measures of efficacy were assessed independently. The first was the proportion of subjects who provided a 'yes' response to the weekly SGA question: "*In the past 7 days, have you had adequate relief of your IBS symptoms? (yes*/*no)* ". The second endpoint was the proportion of subjects who provide a 'yes' response to the weekly individual symptom question: *"In the past 7 days, have you had adequate relief of your symptom of bloating? (yes*/*no)*". Durability was based on the proportion of subjects that had adequate relief over the entire treatment phase.

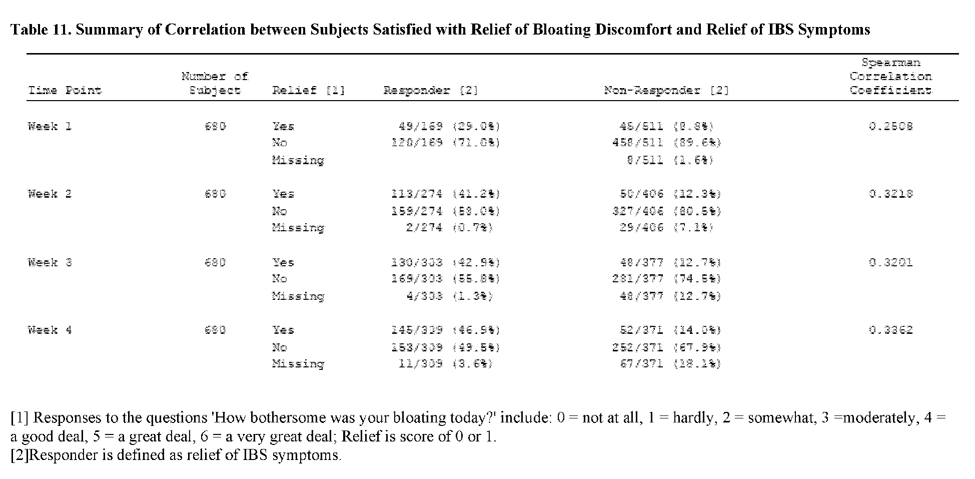

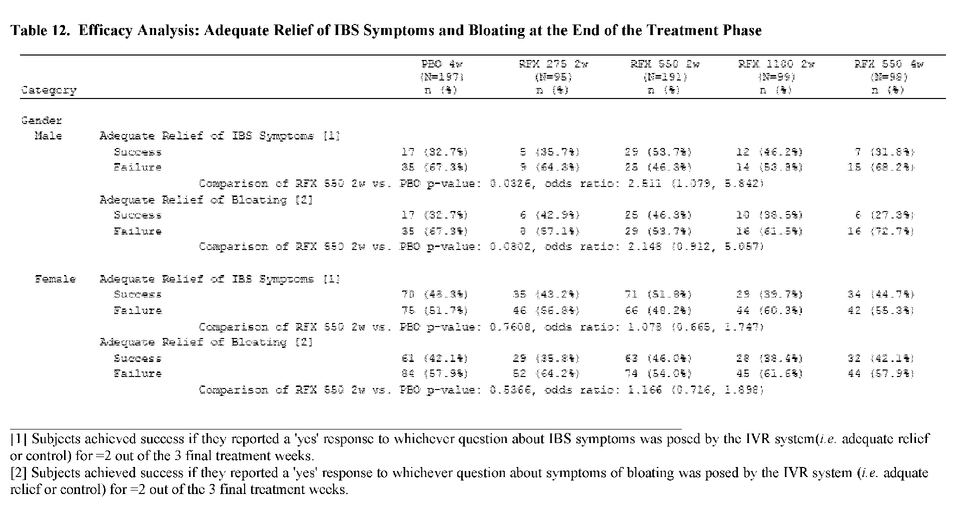

The treatment effect is more pronounced when accounting for milder disease severity, *e.g*., bloating, abdominal pain/discomfort and bowel movements.

### EXAMPLE 2 (reference example)

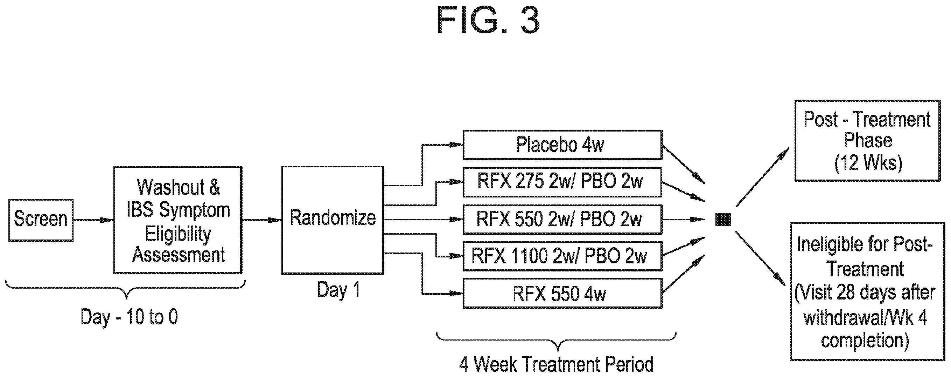

A study (Figure 3) is designed to evaluate the efficacy of a 14-day course of oral rifaximin at 550 mg TID in providing adequate relief from diarrhea-associated IBS (d-IBS) symptoms over four weeks. A measure of efficacy is based on subjects' answers to the Weekly Subject Global Assessment (SGA) questions over the 4 week study duration in relation to their IBS symptoms. The SGA question is asked weekly as follows: *"In the past 7 days, have you had adequate relief of your IBS symptoms?" (Yes*/*No.)* Subjects in the treatment group taking oral rifaximin respond "Yes" more often than Subjects who are not taking oral rifaximin. Another measure of efficacy is based on subjects' answers to the Weekly Subject Global Assessment (SGA) question over the 4 week study duration in relation to their IBS symptom of bloating. The SGA question is asked weekly as follows: "*In the past 7 days, have you had adequate relief of your IBS symptom of bloating?*" *(Yes*/*No.* Subjects in the treatment group taking oral rifaximin respond "Yes" more often than subjects who are not taking oral rifaximin. Other measures of efficacy include the changes in d-IBS symptoms from baseline to each week of the 4 weeks in the study (*e.g*., abdominal pain and discomfort, bloating, number of stools per day, stool consistency, urgency with loose or watery stools).

### EXAMPLE 3 (reference example)

### Improvements in Quality of Life

A study showed the rifaximin 550 mg twice daily (BID) significantly improved IBS symptoms versus placebo in patients with diarrhea-predominant IBS (d-IBS, or IBS-D). Analyses from that study evaluated the efficacy of rifaximin for improving quality of life (QOL) measures in patients with dIBS.

Adults diagnosed with d-IBS (Rome II criteria) received rifaximin 550 mg BID or placebo for 14 days. Both groups received placebo for an additional 14 days after the initial 2-week treatment. Quality of life was assessed with the 34-item IBS-QOL questionnaire at baseline and 4 weeks after initiating treatment. Each item was scored on a 5-point scale (1=not at all; 2=slightly; 3=moderately; 4=quite a bit; and 5=extremely or a great deal). Results for composite and subscale scores were converted to a scale ranging from 0 to 100, with higher scores indicating better QOL.

A total of 388 patients were treated; 191 patients received rifaximin and 197 patients received placebo during the 2-week initial treatment period. The mean improvement from baseline in overall QOL scores at week 4 was significantly greater with rifaximin compared with placebo (Table 11). Patients in the rifaximin group reported significantly greater mean improvement from baseline in QOL scores for dysphoria, body image, health worry, social reaction, and relationship subscales compared with placebo (Table 11). Rifaximin was well tolerated, with similar incidence of adverse events compared with placebo.

In patients with IBS-D, rifaximin 1100 mg/d for 14 days significantly improved QOL measures compared with placebo. These findings suggest a potential therapeutic role for rifaximin 550 mg BID for improving symptoms and QOL in patients with IBS-D and are summarized in Table 13.

**Table 13. Mean Change From Baseline in IBS-QOL Scores at Week 4**

| Domain | Rifaximin 1100 mg/d (n=191) | Placebo (n=197) | Improvement with rifaximin over placebo, % | P value |
|---|---|---|---|---|
| Overall score | 20.4 | 15.8 | 28.7 | 0.020 |
| Dysphoria | 24.8 | 19.8 | 25.3 | 0.027 |
| Interference with activity | 22.2 | 18.1 | 22.2 | 0.083 |
| Body image | 20.1 | 14.6 | 37.4 | 0.012 |
| Health worry | 16.0 | 12.2 | 30.6 | 0.047 |
| Food avoidance | 25.0 | 20.5 | 22.1 | 0.088 |
| Social reaction | 17.3 | 13.2 | 31.6 | 0.047 |
| Sexual | 13.6 | 10.9 | 24.9 | 0.199 |
| Relationship | 14.9 | 10.7 | 39.5 | 0.030 |

A 2-week course of rifaximin (1100 mg/day) significantly improved quality of life (QOL) measures, compared with placebo.

In a study, 191 adult patients diagnosed with diarrhea-predominant IBS (d-IBS) by Rome II criteria were randomized to receive rifaximin 550 mg twice daily (BID) and 197 patients were randomized to placebo. Following a 2-week initial treatment period, both groups of patients received placebo for an additional 14 days. Quality of life was assessed via the 34-item IBS-QOL questionnaire at baseline and 4 weeks after initiating treatment. Each item was scored on a 5-point scale (1=not at all; 2=slightly; 3=moderately; 4=quite a bit; 5=extremely or a great deal); results for composite and subscale scores were converted to a scale ranging from 0 to 100, with higher scores indicating better QOL.

At Week 4, the mean improvement from baseline in the overall QOL score was significantly greater with rifaximin compared with placebo (20.4 vs. 15.8, respectively; p=0.020). Patients in the rifaximin group also reported significantly greater mean improvement from baseline in QOL subscale scores for dysphoria (restlessness or agitation, 24.8 vs. 19.8; p=0.027), body image (20.1 vs. 14.6; p=0.012), health worry (16.0 vs. 12.2; p=0.047), social reaction (17.3 vs. 13.2; p=0.047), and relationships (14.9 vs. 10.7; p=0.030), compared with placebo. Rifaximin was well tolerated in the study, with a similar incidence of adverse events compared with placebo.

### EXAMPLE 4 (reference example)

### Severity of Baseline Symptoms as Predictor of Clinical Response

It is reported herein that the severity of baseline symptoms of abdominal pain and bloating influenced the response to rifaximin treatment. The co-primary endpoints in this analysis assessed weekly yes/no responses to questions regarding adequate relief of global IBS symptoms and IBS-associated bloating. Severity of baseline IBS symptoms was evaluated as a potential confounder of clinical response and was categorized as mild/moderate or severe based on a mean score of ≤ 4 vs. > 4 (on a 7-point scale) for bloating and abdominal pain.

A significantly larger percentage of patients treated with rifaximin reported adequate relief of global IBS symptoms (52% vs. 44% for placebo; p=0.03) and bloating (46% vs. 40%; p=0.04), compared with placebo-treated patients. In patients with mild/moderate abdominal pain, rifaximin produced a greater degree of improvement, compared with placebo, in global symptoms of IBS (50% vs. 39%, respectively; p=0.04) and bloating (44% vs. 35%; p=0.09). Similarly, in patients with mild/moderate bloating, rifaximin treatment was associated with greater improvement, compared with placebo, in global IBS symptoms (56% vs. 41%, respectively; p=0.006) and bloating (47% vs. 36%; p=0.03). This demonstrates that patients with mild/moderate IBS symptoms are more likely than those with severe disease to achieve symptomatic relief with rifaximin.

These results show that rifaximin improves gastrointestinal (GI) symptoms associated with IBS. In this study of rifaximin versus placebo, patients with diarrhea-predominant IBS (IBS-D) were studied, a supplemental analysis examined the association between severity of baseline IBS symptoms and clinical response to rifaximin.

A comparison involved 2 groups of adult patients with IBS-D (Rome II) who received rifaximin 550 mg twice daily or placebo for 14 days, followed by an additional 14 days of placebo in both groups. The Weekly yes/no responses to questions regarding adequate relief of global IBS symptoms and IBS-associated bloating were assessed. Clinical response was defined as adequate relief for 2 of the final 3 treatment weeks (wk 2, 3, or 4). Severity of baseline IBS symptoms was evaluated as a potential confounder of clinical response and was categorized as mild/moderate or severe based on a mean score of ≤ 4 versus > 4 (on a 7-point scale (0=not bothersome; 6=very bothersome)) for bloating and abdominal pain.

A significantly larger percentage of patients who received rifaximin versus placebo reported adequate relief of global IBS symptoms (52% versus 44%, respectively; P=0.03) and bloating (46% versus 40%, respectively; P=0.04). In patients with mild/moderate abdominal pain, rifaximin produced a greater degree of improvement versus placebo in symptoms of IBS (50% versus 39%, respectively; P=0.04) and bloating (44% versus 35%, respectively; P=0.09). In patients with mild/moderate bloating, rifaximin also achieved greater improvement versus placebo in global symptoms of IBS (56% versus 41%, respectively; P=0.006) and bloating (47% versus 36%, respectively; P=0.03). Severity of baseline symptoms of abdominal pain and bloating influenced the response to rifaximin 1100 mg/d for 14 days. Patients with mild/moderate IBS symptoms had a greater likelihood of relief of global IBS-related symptoms with rifaximin treatment versus individuals with severe IBS symptoms.

### EXAMPLE 5 (reference example)

In a study carried out to evaluate the efficacy of a 14-day course of oral rifaximin at 550 mg TID, it was demonstrated that the administered dosage provided adequate relief from diarrhea-predominant IBS (d-IBS) symptoms over four weeks. A measure of efficacy was based on subjects' answers to the Weekly Subject Global Assessment (SGA) questions over the 4 week study duration in relation to their IBS symptoms. The SGA question was asked weekly as follows: *"In the past 7 days, have you had adequate relief of your IBS symptoms?" (Yes*/*No.)* Subjects in the treatment group taking oral rifaximin responded "Yes" more often than Subjects who were not taking oral rifaximin. Another measure of efficacy was based on subjects' answers to the Weekly Subject Global Assessment (SGA) question over the 4 week study duration in relation to their IBS symptom of bloating. The SGA question was asked weekly as follows: "*In the past 7 days, have you had adequate relief of your IBS symptom of bloating?" (Yes*/*No)..* Subjects in the treatment group taking oral rifaximin responded "Yes" more often than subjects who were not taking oral rifaximin. Other measures of efficacy included the changes in d-IBS symptoms from baseline to each week of the 4 weeks in the study (e.g., abdominal pain and discomfort, bloating, number of stools per day, stool consistency, urgency with loose or watery stools).

The randomized, double-blind, placebo-controlled, multicenter trial was designed to evaluate the efficacy and safety of rifaximin 550 mg TID in the treatment of patients with nonconstipation irritable bowel syndrome (non-C IBS). In the trial, rifaximin versus placebo treated patients demonstrated a statistically significant improvement for the primary endpoint of the adequate relief of IBS symptoms as assessed over one month (weeks 3, 4, 5 and 6) following completion of a 14-day course of therapy (weeks 1 and 2). Consistent with the primary endpoint in each trial, the key secondary endpoint of relief of IBS-related bloating also demonstrated statistical significance of rifaximin versus placebo in each trial.

The assessment of the clinical efficacy and safety of a 550 mg TID dosing regimen of rifaximin (1650 mg/day) compared with placebo in a broad population comprised of males and females 18 years of age and older who were diagnosed with non-constipation IBS, *e.g*., diarrhea-predominant IBS or alternating IBS. The primary efficacy endpoint of the study was the proportion of subjects who achieved adequate relief of IBS symptoms for at least 2 weeks during the first 4 weeks of the 10-week follow-up phase.

Subjects received 550 mg of rifaximin three times daily (TID) for 14 days and then were followed for 10 weeks for study duration of 12 weeks. Two measures of efficacy were assessed.

Subjects were questioned on the relief of overall IBS symptoms and bloating. Adequate relief of IBS related symptoms (SGA) and IBS-related bloating (IBS-B) were assessed, and a dose of 550 mg TID 15 for 2 weeks demonstrated statistically significant relief. The analyses defined success as a "yes" response to questions regarding adequate relief.

A measure of efficacy was based on subjects' answers to the Weekly Subject Global Assessment (SGA) questions over the 4 week study duration in relation to their IBS symptoms. The SGA question was asked weekly as follows: "In the past 7 days, have you had adequate relief of your 20 IBS symptoms?" (Yes/No.) Subjects in the treatment group taking oral rifaximin responded "Yes" more often than Subjects who are not taking oral rifaximin. Another measure of efficacy was based on subjects' answers to the Weekly Subject Global Assessment (SGA) question over the 4 week study duration in relation to their IBS symptom of bloating. The SGA question was asked weekly as follows: "In the past 7 days, have you had adequate relief of your IBS symptom of bloating?" (Yes/No). Subjects in the treatment group taking oral rifaximin responded "Yes" more often than subjects who are not taking oral rifaximin.

All subpopulations in the study responded to therapy. Baseline severity was determined during screening for Abdominal Pain/Discomfort and Bloating, and the number, type (normal, hard, loose) and urgency of bowel movements. Duration of effect was assessed in a ten week follow-up period.

Subjects enrolled in the study are detailed in Table 13. Demographics of the population are set forth in Tables 14, 15, and 16.

It was observed that rifaximin exposure in subjects having IBS is similar to the levels of exposure in healthy subjects and more than 520-fold lower than rifampin exposure, and more than 66-fold lower than neomycin exposure. As previously disclosed by Applicants, the rifaximin exposure in healthy subjects is significantly lower than the level of exposure in subjects having hepatic encephalopathy.

### Percentage of Subjects with Adequate Relief of IBS and Bloating Symptoms

The primary and secondary endpoints evaluated in this study were the effect of treatment on the percentage of subjects who reported adequate relief of IBS and the adequate relief of IBS symptom of bloating. These results are shown in Tables 17, 18, 19 and 20. The data demonstrates that more subjects taking rifaximin had adequate relief of IBS symptoms and of bloating.

To further evaluate the study results, efficacy of rifaximin treatment on subpopulations of study participants was evaluated.

Primary and secondary endpoints were evaluated for male and female populations independently. This analysis indicated that higher percentage of female subjects taking rifaximin had adequate relief of IBS symptoms and the IBS symptom of bloating. See Table 23.

Primary and secondary endpoints were evaluated for subpopulations of study participants based on age. Analysis of subjects less than 65 and those 65 years old and older demonstrated that a higher percentage of subjects 65 years old or older that were administered rifaximin had adequate relief of IBS symptom of bloating. See Table 24.

The efficacy of rifaximin treatment of white and non-white study participants demonstrated that a higher percentage of non-white participants administered rifaximin had adequate relief of IBS symptoms. See Table 25.

The efficacy of rifaximin treatment was also evaluated for subjects having diarrhea-predominant IBS and alternating-predominant IBS. The data indicate that a higher percentage of subjects having alternating-predominant IBS had adequate relief of IBS symptoms and adequate relief of IBS symptom of bloating than subjects with diarrhea-predominant. See Table 26.

The study also evaluated the effect of rifaximin administration on the average number of stools per day from the baseline value for each subject. The data indicate that rifaximin effectively decreased the weekly average of stool frequency by at least one for subjects in the study. In particular, the last four weeks of the study show significant decrease in the stool frequency for subject administered rifaximin when compared to those administered a placebo. See Table 14.

**Table 14. Stool frequency (SF).**

| Week | Placebo % with decrease in SF | Rifaximin (550 mg TID)% with decrease in SF | P-value |
|---|---|---|---|
| 1 | 16.9 | 14.4 | .3853 |
| 2 | 21.6 | 20.6 | .7562 |
| 3 | 23.1 | 22.2 | .7677 |
| 4 | 24.1 | 21.6 | .4464 |
| 5 | 21.3 | 25.7 | .1901 |
| 6 | 23.4 | 22.9 | .8402 |
| 7 | 23.8 | 23.8 | .9945 |
| 8 | 20.9 | 24.4 | .3029 |
| 9 | 24.7 | 25.4 | .8581 |
| 10 | 21.6 | 26.7 | .1360 |
| 11 | 24.1 | 25.4 | .6994 |
| 12 | 25.3 | 27.0 | .6390 |

Interestingly, subjects administered 550 mg rifaximin TID showed a decrease in skin and subcutaneous tissue disorders as compared to the placebo group. 3.8% of the placebo group had skin or subcutaneous tissue disorders as compared to 1.3% of the rifaximin treated group.

**Table 15. Subject Disposition by Treatment Group Population: Randomized Subjects**

| | Placebo n (%) | Rifaximin 550mgTID n (%) | Total n | (%) |
|---|---|---|---|---|
| Subjects Randomized | 321 | 316 | 637 | |
| Intent-to-Treat Subjects [ | 320 (99.7%) | 315 (99.7%) | 635 | (99.70%) |
| Subjects Completed the Treatment Phase | 313 (97.5%) | 310 (98.1%) | 623 | (97.80%) |
| Subjects Completed through Week 6 | 307 (95.6%) | 308 (97.5%) | 615 | (96.50%) |
| Subjects Completed the Study | 302 (94.1%) | 301 (95.3%) | 603 | (94.70%) |
| Subjects Discontinued | 19 (5.9%) | 15 (4.7%) | 34 | (5.30%) |
| Study Early | | | | |
| Primary Reason For Early | | | | |
| Discontinuation of Study | | | | |
| Adverse Event/Serious | 2 (0.6%) | 0 | 2 | (0.30%) |
| Adverse Event | | | | |
| Subject Request | 8 (2.5%) | 6 (1.9%) | 14 | (2.20%) |
| Lost to Follow-Up | 6 (1.9%) | 6 (1.9%) | 12 | (1.90%) |
| Noncompliance | 2 (0.6%) | 1 (0.3%) | 3 | (0.50%) |
| Pregnancy | 0 | 0 | 0 | |
| Other | 1 (0.3%) | 2 (0.6%) | 3 | (0.50%) |

| | | | | |
|---|---|---|---|---|
| Note: Percentage calculation is based on the number of subjects randomized. [1] Intent-to-Treat population includes all randomized subjects who ingested at least one dose of the study drug. | | | | |

**Table 16. Summary of Demographic by Treatment Group: Population: ITT**

| | mg TID (N = 320) | | Total (N = 315) | (N = 635) |
|---|---|---|---|---|
| Age (years) | | | | |
| N | 320 | | 315 | 635 |
| Mean | 46.3 | | 45.9 | 46.1 |
| SD | 14.57 | | 13.87 | 14.22 |
| Median | 46 | | 45 | 46 |
| Min | 18 | | 19 | 18 |
| Max | 82 | | 88 | 88 |

| Age group - n (%) | | | | |
|---|---|---|---|---|
| <65 | 283 (88.4%) | | 285 (90.5%) | 568 (89.4%) |
| >=65 | 37 (11.6%) | | 30 (9.5%) | 67 (10.6%) |

| Gender - n (%) | | | | |
|---|---|---|---|---|
| Male | 95 (29.7%) | | 88 (27.9%) | 183 (28.8%) |
| Female | 225 (70.3%) | | 227 (72.1%) | 452 (71.2%) |

| Race [ 1] - n (%) | | | | |
|---|---|---|---|---|
| American Indian or Alaskan Native | 2 (0.6%) | | 1 (0.3%) | 3 (0.5%) |
| Asian | 2 (0.6%) | | 6 (1.9%) | 8 (1.3%) |
| Black or African American | 14 (4.4%) | | 21 (6.7%) | 35 (5.5%) |
| Native Hawaiian or Other Pacific Islander | | 0 | 3 (1.0%) | 3 (0.5%) |
| White | 302 (94.4%) | | 282 (89.5%) | 584 (92.0%) |
| Other | | 0 | 2 (0.6%) | 2 (0.3%) |

**Table 17. Summary of Demographic by Treatment Group Population ITT**

| | | Placebo (N= 320) | | Rifaximin 550 mg TID (N = 315) | Total (N = 635) |
|---|---|---|---|---|---|
| Ethnicity - n (%) | | | | | |
| | Hispanic or Latino | | 29 (9.1%) | 29 (9.2%) | 58 (9.1%) |
| | Not Hispanic or Latino | | 291 (90.9%) | 286 (90.8%) | 577 (90.9%) |
| Height (cm) | | | | | |
| | n | | 320 | 315 | 635 |
| | Mean | | 167.85 | 16732 | 167.59 |
| | SD | | 9.684 | 10.342 | 10.011 |
| | Median | | 167.60 | 165.50 | 167.60 |
| | Min | | 147.3 | 104.8 | 104.8 |
| | Max | | 193.0 | 198.1 | 198.1 |
| | Weight - n | 320 | | 315 | 635 |
| | Mean | 81.30 | | 80.91 | 81.11 |
| | SD | 19.715 | | 20.233 | 19.959 |
| | Median | 7895 | | 7890 | 7890 |
| | Min | 40.8 | | 46.7 | 40.8 |
| | Max | 161.5 | | 166.9 | 166.9 |

Note: Percentages are based on the number of subjects in the ITT population in each treatment group. [1] If more than one race are checked, the subject is only included in the 'Other' category.

**Table 18. Summary of Demographic by Treatment Group Population ITT**

| | | | |
|---|---|---|---|
| | Placebo N=320 | Rifaximin 550 mgTID N=315 | Total N=635 |

| BMI(kg/m^2) | | | |
|---|---|---|---|
| n | 320 | 315 | 635 |
| Mean | 28.8 | 28.92 | 28.86 |
| SD | 6.546 | 6.872 | 6.705 |
| Median | 27.6 | 27.8 | 27.7 |
| Min | 15.7 | 17.3 | 15.7 |
| Max | 55.7 | 55.8 | 55.8 |

| BMI (kg/m^2) - Male | | | |
|---|---|---|---|
| n | 95 | 88 | 183 |
| Mean | 28.09 | 28.45 | 28.27 |
| SD | 4.903 | 5.526 | 5.2 |
| Median | 27.7 | 27.55 | 27.7 |
| Min | 18.9 | 19.4 | 18.9 |
| Max | 46.6 | 54.3 | 54.3 |

| BMI (kg/m^2) - Female | | | |
|---|---|---|---|
| n | 225 | 227 | 452 |
| Mean | 29.1 | 29.11 | 29.1 |
| SD | 7.116 | 7.33 | 7.216 |
| Median | 27.5 | 27.9 | 27.75 |
| Min | 15.7 | 17.3 | 15.7 |
| Max | 55.7 | 55.8 | 55.8 |

**Table 19. Adequate Relief of IBS Symptoms and IBS Symptom of Bloating by Treatment Group**

| | Placebo | Rifaximin 550mg TID | |
|---|---|---|---|
| | (N=320) | (N=315) | p-value |
| Adequate Relief of IBS symptoms [ 2] | n (%) | n (%) | 0.0256 |
| Success | 100 (31.3%) | 125 (39.7%) | |
| Failure | 220 (68.8%) | 190 (60.3%) | |
| Adequate Relief of IBS symptom of Bloating [ 3] | 0.0198 | | |
| Success | 99 (30.9%) | 125 (39.7%) | |
| Failure | 221 (69.1%) | 190 (60.3%) | |

| | | | |
|---|---|---|---|
| Note: Last observation carried forward method (LOCF) was used to handle missing responses. Baseline responses were not carried forward. [1] p-value is obtained from a Logistic regression model with fixed effects treatment arm and analysis center. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 20. Adequate Relief of IBS Symptoms and IBS Symptom of Bloating by Treatment Group**

| Population: PP | | | |
|---|---|---|---|
| | Placebo | Rifaximin 550mg TID | |
| | (N=310) | (N=311) | p-value |
| | N (%) | N (%) | 0.0159 |
| Adequate Relief of IBS symptoms [ 2] | | | |
| Success | 95 (30.6%) | 124 (39.9%) | |
| Failure | 215 (69.4%) | 187 (60.1%) | |
| Adequate Relief of IBS symptom of Bloating [ 3] | | | 0.012 |
| Success | 94 (30.3%) | 124 (39.9%) | |
| Failure | 216 (69.7%) | 187 (60.1%) | |

| | | | |
|---|---|---|---|
| Note: Subjects failed to meet inclusion criteria 3, 4, 5, or exclusion criteria 1 or 8 are excluded from this table. Note: Last observation carried forward method (LOCF) was used to handle missing responses. Baseline responses were not carried forward. [1] p-value is obtained from a Logistic regression model with fixed effects treatment arm and analysis center. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 21. Adequate Relief of IBS Symptoms and IBS Symptom of Bloating Based on Daily Measures by Treatment Group Population: ITT**

| | Placebo | Rifaximin 550mg TID | |
|---|---|---|---|
| | (N=320) | (N=315) | p-value |
| | N (%) | N (%) | |
| Adequate Relief of IBS symptoms [ 2] | | | 0.0139 |
| Success | 88 (27.5%) | 115 (36.5%) | |
| Failure | 232 (72.5%) | 200 (63.5%) | |
| Adequate Relief of IBS symptom of Bloating [ 3] | | | 0.0139 |
| Success | 95 (29.7%) | 133 (42.2%) | |
| Failure | 225 (70.3%) | 182 (57.8%) | |

| | | | |
|---|---|---|---|
| Note: [1] p-value is obtained from a Logistic regression model with fixed effects treatment arm and analysis center. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 22. Adequate Relief of IBS Symptoms and IBS Symptom of Bloating Based on Daily Measures by Treatment Group Population: PP**

| | Placebo | Rifaximin 550mg TID | |
|---|---|---|---|
| | (N=310) | (N=311) | p-value |
| | N (%) | N (%) | |
| Adequate Relief of IBS symptoms [ 2] | | | 0.0075 |
| Success | 84 (27.1 %) | 115 (37.0%) | |
| Failure | 226 (72.9%) | 196 (63.0%) | |
| Adequate Relief of IBS symptom of Bloating [ 3] | | | 0.0012 |
| Success | 92 (29.7%) | 131 (42.1%) | |
| Failure | 218 (70.3%) | 180 (57.9%) | |

| | | | |
|---|---|---|---|
| Note: [1] p-value is obtained from a Logistic regression model with fixed effects treatment arm and analysis center. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

The results of each trial indicated that each of the daily measures of IBS-related symptoms, bloating and abdominal pain demonstrates significant relief with in the primary evaluation period (weeks 3-6) as well as consistent results across all time periods. This finding adds several key observations to what is known about the durable effect of rifaximin on IBS. Namely, that daily questioning, which reduces recall bias presumed to be part of the primary and key secondary endpoints, demonstrates significant and robust finding. Secondly, these finding correlate significantly with the results of the primary and key secondary, yielding interclass correlations which are very strong indicating construct validity (*e.g*., daily measures show strong relationship and validated measure of disease activity for IBS, weekly SGA). Thirdly, the daily measures were all highly correlated with each other (correlation coefficient of at least 80%). Taken in totality, the results of the primary, key secondary, daily measures of symptoms, bloating and abdominal pain strongly support the reliability, validity, responsiveness, and utility of these outcomes used as endpoints in the studies suggests that each of these questionnaires validates the results from the other.

In addition, using the primary endpoint to assess effect over the entire 3 months of the trial, adequate relief of global IBS symptoms was superior in rifaximin treated as compared to placebo treated patients in each of the trials respectively. This endpoint was tested previously and accepted by the review division, specifically with lotronex, that is, the number of months with adequate relief of IBS symptoms during the entire study duration (typical responses include 0 months, 1 month, 2 months, or 3 months with adequate relief). This approach uses all of the data across the period (12 week/3 months) and demonstrates that 2 weeks of treatment provides 3 months of relief.

The two studies, independently, demonstrated that rifaximin 550 mg TID for 14 days provides statistically significant relief of IBS symptoms during the primary evaluation period (Days 15 - 42) as measured in:
Weekly IBS Global Symptoms (Primary Endpoint);
Weekly IBS Symptom of Bloating (Key Secondary Endpoint);
IBS Daily Assessment of Symptoms;
Daily IBS Global Symptoms;
Daily IBS Symptom of Bloating; and
Daily IBS Symptom of Abdominal Pain.

In addition, the two studies, independently, demonstrated that rifaximin 550 mg TID for 14 days provides statistically significant relief of IBS symptoms during all 3 months as demonstrated by:
Weekly Global IBS Symptoms; and
Daily Global IBS symptoms.

**Table 23. Subgroup Analysis: Adequate Relief of IBS Symptoms and IBS Symptom of Bloating by Treatment Group and Gender**

| Gender: Male | | | |
|---|---|---|---|
| | Placebo | Rifaximin 550mg TID | |
| | (N=91) | (N=85) | p-value |
| | N (%) | N (%) | |
| Adequate Relief of IBS symptoms [ 2] | | | 0.2636 |
| Success | 23 (25.3%) | 28 (32.9%) | |
| Failure | 68 (74.7%) | 57 (67.1%) | |
| Adequate Relief of IBS symptom of Bloating [ 3] | | | 0.7695 |
| Success | 26 (28.6%) | 26 (30.6%) | |
| Failure | 65 (71.4%) | 59 (69.4%) | |

### Gender: Female

| | Placebo | Rifaximin 550mg TID | |
|---|---|---|---|
| | (N=219) | (N=226) | p-value |
| | N (%) | N (%) | |
| Adequate Relief of IBS symptoms [ 2] | | | 0.0371 |
| Success | 72 (32.9%) | 96 (42.5%) | |
| Failure | 147 (67.1%) | 130 (57.5%) | |
| Adequate Relief of IBS symptom of Bloating [ 3] | | | 0.0075 |
| Success | 68 (31.1%) | 98 (43.4%) | |
| Failure | 151 (68.9%) | 128 (56.6%) | |

| | | | |
|---|---|---|---|
| Note: Last observation carried forward method (LOCF) was used to handle missing responses. Baseline responses were not carried forward. [1] p-value is obtained from a Logistic regression model with fixed effects treatment arm. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 24. Subgroup Analysis: Adequate Relief of IBS Symptoms and IBS Symptom of Bloating by Treatment Group and Age Group**

| Age Group: <65 | | | |
|---|---|---|---|
| | Placebo | Rifaximin 550mg TID | |
| | (N=274) | (N=281) | p-value |
| | N (%) | N (%) | |
| Adequate Relief of IBS symptoms [ 2] | | | 0.0228 |
| Success | 82 (29.9%) | 110 (39.1%) | |
| Failure | 192 (70.1%) | 171 (60.9%) | |
| Adequate Relief of IBS symptom of Bloating [ 3] | | | 0.0106 |
| Success | 79 (28.8%) | 110 (39.1%) | |
| Failure | 195 (71.2%) | 171 (60.9%) | |

### Age Group: >=65

| | Placebo | Rifaximin 550mg TID | |
|---|---|---|---|
| | (N=274) | (N=281) | p-value |
| | N (%) | N (%) | |
| Adequate Relief of IBS symptoms [ 2] | | | 0.3862 |
| Success | 13 (36.1%) | 14 (46.7%) | |
| Failure | 23 (63.9%) | 16 (53.3%) | |
| Adequate Relief of IBS symptom of Bloating [ 3] | | | 0.6838 |
| Success | 15 (41.7%) | 14 (46.7%) | |
| Failure | 21 (58.3%) | 16 (53.3%) | |

| | | | |
|---|---|---|---|
| Note: Last observation carried forward method (LOCF) was used to handle missing responses. Baseline responses were not carried forward. [1] p-value is obtained from a Logistic regression model with fixed effects treatment arm. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 25. Subgroup Analysis: Adequate Relief of IBS Symptoms and IBS Symptom of Bloating by Treatment Group and Race**

| Race: White | | | |
|---|---|---|---|
| | Placebo | Rifaximin 550mg TID | |
| | (N=292) | (N=280) | p-value |
| | N (%) | N (%) | |
| Adequate Relief of IBS symptoms [ 2] | | | 0.0341 |
| Success | 89 (30.5%) | 109 (38.9%) | |
| Failure | 203 (69.5%) | 171 (61.1%) | |
| Adequate Relief of IBS symptom of Bloating [ 3] | | | 0.0172 |
| Success | 87 (29.8%) | 110 (39.3%) | |
| Failure | 205 (70.2%) | 170 (60.7%) | |

### Race: Non-White

| | Placebo | Rifaximin 550mg TID | |
|---|---|---|---|
| | (N=18) | (N=31) | p-value |
| | N (%) | N (%) | |
| Adequate Relief of IBS symptoms [ 2] | | | 0.3074 |
| Success | 6 (33.3%) | 15 (48.4%) | |
| Failure | 12 (66.7%) | 16 (51.6%) | |
| Adequate Relief of IBS symptom of Bloating [ 3] | | | 0.6691 |
| Success | 7 (38.9%) | 14 (45.2%) | |
| Failure | 11 (61.1%) | 17 (54.8%) | |

| | | | |
|---|---|---|---|
| Note: Last observation carried forward method (LOCF) was used to handle missing responses. Baseline responses were not carried forward. [1] p-value is obtained from a Logistic regression model with fixed effects treatment arm and analysis center. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

**Table 26. Subgroup Analysis: Adequate Relief of IBS Symptoms and IBS Symptom of Bloating by Treatment Group and IBS Sub-type IBS Subtype: Diarrhoea-predominant**

| IBS Subtype: Diarrhoea-predominant | | | |
|---|---|---|---|
| | Placebo | Rifaximin 550mg TID | |
| | (N=292) | (N=280) | p-value |
| | N (%) | N (%) | |
| Adequate Relief of IBS symptoms [ 2] | | | 0.0337 |
| Success | 87 (31.4%) | 111 (40.1%) | |
| Failure | 190 (68.6%) | 166 (59.9%) | |
| Adequate Relief of IBS symptom of Bloating [ 3] | | | 0.0928 |
| Success | 90 (32.5%) | 109 (39.4%) | |
| Failure | 187 (67.5%) | 168 (60.6%) | |

### IBS Subtype: Alternating-predominant

| | Placebo | Rifaximin 550mg TID | |
|---|---|---|---|
| | (N=18) | (N=31) | p-value |
| | N (%) | N (%) | |
| Adequate Relief of IBS symptoms [ 2] | | | 0.2202 |
| Success | 8 (24.2%) | 13 (38.2%) | |
| Failure | 25 (75.8%) | 21 (61.8%) | |
| Adequate Relief of IBS symptom of Bloating [ 3] | | | 0.006 |
| Success | 4 (12.1%) | 15 (44.1%) | |
| Failure | 29 (87.9%) | 19 (55.9%) | |

| | | | |
|---|---|---|---|
| Note: Last observation carried forward method (LOCF) was used to handle missing responses. Baseline responses were not carried forward. [1] p-value is obtained from a Logistic regression model with fixed effects treatment arm. [2] Subjects achieved success if they answered `Yes' to the weekly SGA question, `In the past 7 days, have you had adequate relief of your IBS symptoms', for at least 2 of the first 4 weeks during the follow-up phase (ie, Weeks 3 through 6). [3] Subjects achieved success if they answered `Yes' to the weekly SGA question, `In the past 7 days, have you had adequate relief of your IBS symptom of bloating', for at least 2 of the first 4 weeks during the follow-up phase (ie, Weeks 3 through 6). | | | |

### EXAMPLE 6. (reference example)

### Analysis of Two Studies After 3 Months

Studies were designed to evaluate the efficacy of oral rifaximin at 550 mg TID in providing adequate relief from diarrhea-predominant IBS (dIBS) symptoms over three months. A measure of efficacy is based on subjects' answers to the Weekly Subject Global Assessment (SGA) questions over the study duration in relation to their IBS symptoms.

The two studies, independently, demonstrated that rifaximin 550 mg TID for 14 days provides statistically significant relief of IBS symptoms during the primary evaluation period and the 10 weeks of monitoring after the administration period ends. The results are presented in Table 27 below.

**Table 27. Endpoints**

| | | | | |
|---|---|---|---|---|
| Global IBS symptoms | 31% vs 41% (p=0.0125) | 32% vs 41% (p=0.0263) | 32% vs 41% (p=0.0008) | At least 2 out of 4 weeks adequate relief of IBS symptoms during weeks 3 through 6 (weekly question) |
| AUC in abdominal pain during PEP | P=0.0128 | P=0.0328 | P=0.0017 | |

**Table 28: Endpoints for Abdominal Pain (Change from baseline)**

| **Endpoint** | **Study'** | **Study 2** | **Combined** | **Description** |
|---|---|---|---|---|
| Abdominal pain reduction of 1 point in median | 52% vs 63% (p=0.0039) | 53% vs 62% (p=0.0382) | 53% vs 63% (p=0.0005) | Responder is defined as who had reduction of 1 point in the weekly median score of abdominal pain compared to baseline for at least 2 weeks during PEP. |
| | | | | -Baseline median is based on the last three diary entries prior to the first dose date. |
| | | | | - Post-baseline weekly median is based on all diary entries in that week. |
| Abdominal Pain reduction of 2 point in median | 33% vs 37% (p=0.3155) | 30% vs 41% (p=0.0049) | 32% vs 39% (p=0.0064) | See above |
| Abdominal Pain reduction of 3 point in median | 19% vs 22% (p=0.3443) | 17% vs 21% (p=0.2329) | 18% vs 21% (p=0.1333) | See above |
| Daily abdominal pain < 2 | 32% vs 40% (p=0.0373) | 31% vs 39% (p=0.0383) | 31% vs 39% (p=0.0036) | Responder is defined as who had daily abdominal pain <2 for at least 50% of days in a given week for at least 2 weeks during PEP. |
| Overall reduction of median weekly abdominal pain by >=25% | 52% vs 63% (p=0.0051) | 52% vs 62% (p=0.0170) | 52% vs 62% (p=0.0003) | Responder is defined as whose weekly median abdominal pain score dropped by at least 25% comparing to baseline median pain score for at least 2 weeks during PEP. |
| | | | | -Baseline median is based on the last three diary entries prior to the first dose date. |
| | | | | - Post-baseline weekly median is based on all diary entries in that week. |
| Overall reduction of median weekly abdominal pain by >=50% | 34% vs 44% (p=0.0080) | 35% vs 44% (p=0.0117) | 34% vs 44% (p=0.0003) | See above |
| Overall reduction of median weekly abdominal pain by >=75% | 19% vs 23% (p=0.2169) | 17% vs 20% (p=0.3648) | 18% vs 21% (p=0.1334) | See above |
| Abdominal pain reduction of 1 point in mean | 41% vs 52% (p=0.0065) | 43% vs 52% (p=0.0156) | 42% vs 52% (p=0.0003) | Responder is defined as who had reduction of 1 point in the weekly mean score of abdominal pain compared to baseline for at least 2 weeks during PEP. |
| Abdominal Pain reduction of 2 point in mean | 18% vs 26% (p=0.0181) | 18% vs 25% (p=0.0198) | 18% vs 25% (p=0.001) | See above |
| Abdominal Pain reduction of 3 point in mean | 6% vs 7% (p=0.4475) | 6% vs 7% (p=0.4828) | 6% vs 7% (p=0.3676) | See above |
| Daily abdominal pain < 2 | 32% vs 40% (p=0.0373) | 31% vs 39% (p=0.0383) | 31% vs 39% (p=0.0036) | Responder is defined as who had daily abdominal pain <2 for at least 50% of days in a given week for at least 2 weeks during PEP. |
| Overall reduction of mean weekly abdominal pain by >=25% | 47% vs 56% (p=0.0125) | 47% vs 58% (p=0.0036) | 47% vs 57% (p=0.0001) | Responder is defined as whose weekly mean abdominal pain score dropped by at least 25% comparing to baseline mean pain score for at least 2 weeks during PEP. |
| Overall reduction of mean weekly abdominal pain by >=50% | 28% vs 36% (p=0.0280) | 29% vs 35% (p=0.1101) | 28% vs 35% (p=0.0075) | See above |
| Overall reduction of mean weekly abdominal pain by >=75% | 10% vs 14% (p=0.1045) | 11% vs 14% (p=0.3617) | 11% vs 14% (p=0.0780) | See above |
| IBS symptom of Bloating | 29% vs 40% (p=0.0045) | 32% vs 41% (p=0.0167) | 30% vs 40% (p=0.0002) | At least 2 out of 4 weeks adequate relief of IBS symptom of bloating during weeks 3 through 6 (weekly question) |
| Durable response during the entire 3-month study period | | | | |
| - Global IBS symptoms (weekly) | P=0.0477 (See Table 14.2.5a) | P=0.0053 (See Table 14.2.5a) | P=0.0007 (See Table 3.05a) | Number of months that subjects are monthly responders during the 3-month study period. Monthly responders are defined as at least 2 out of 4 weeks adequate relief. |
| - IBS symptom of bloating (weekly) | P=0.1042 (See Table 14.2.5a) | P=0.0031 (See Table 14.2.5a) | P=0.0011 (See Table 3.05a) | See above |
| - Abdominal pain (daily) | P=0.0495 (See Table 14.2.6a) | P=0.0435 (See Table 14.2.6a) | P=0.0118 (See Table 3.06a) | Number of months that subjects are monthly responders during the 3-month study period. Monthly responders are defined as at least 2 out of 4 weeks relief of abdominal pain. Weekly relief is defined as subjects who had |
| | | | | - 0 (not at all) or 1 (hardly) 50% of days within a given week, OR |
| | | | | 0, 1 or 2(somewhat) 100% of days within a given week. |

**Table 29. Definitions**

| **Study Population** | **Study 1** | **Study 2** | **Definition** | |
|---|---|---|---|---|
| Intent to Treat | 623 | 635 | Randomized subjects who took at least one dose of the study drug. | |
| Modified Intent to Treat | 461 (73%) | 501 (78%) | Randomized subjects who took at least one dose of the study drug and met the following criteria: | |
| | | | | ○ Compliance rate is at least 90% |
| | | | | ○ Had at least 4 weeks follow-up after the end of dosing. |

The study endpoints of the studies were Global IBS symptoms and AUC in abdominal pain during the study.

Other endpoints measured were:
Reduction of abdominal pain by 1 point from baseline in mean;
Reduction of abdominal pain by 2 point from baseline in mean;
Reduction of abdominal pain by 3 point from baseline in mean;
Daily abdominal pain of <2 from baseline in mean;
Overall reduction of median weekly abdominal pain by >=25% from baseline in mean;
Overall reduction of median weekly abdominal pain by >=50% from baseline in mean;
Overall reduction of median weekly abdominal pain by >=75% from baseline in mean;
Reduction of abdominal pain by 1 point from baseline in mean;
Reduction of abdominal pain by 2 point from baseline in mean;
Reduction of abdominal pain by 3 point from baseline in mean;
Daily abdominal pain of <2 from baseline in mean;
Overall reduction of median weekly abdominal pain by >=25% from baseline in mean;
Overall reduction of median weekly abdominal pain by >=50% from baseline in mean;
Overall reduction of median weekly abdominal pain by >=75% from baseline in mean;
IBS Symptom of bloating;
Durable response during the three month study
   Global IBS symptoms (weekly);
   IBS symptom of bloating (weekly);
   Abdominal pain (daily).

The proportions of subjects with adequate relief are set forth in Tables 27-29.

The two studies demonstrated that rifaximin 550 mg TID for 14 days provides statistically significant relief of IBS symptoms over a three month period as demonstrated by evaluating the primary and secondary endpoints.

The primary and secondary endpoints evaluated in this study were the effect of treatment on the percentage of subjects who reported adequate relief of Global IBS symptoms, reduction in abdominal pain and the adequate relief of IBS symptom of bloating. These results are shown in Appendices. The data demonstrates that more subjects taking rifaximin had adequate relief of Global IBS symptoms, abdominal pain and of bloating.

### EXAMPLE 7. (reference example)

### Relief of Abdominal Pain and Reduction in IBS Symptoms daily Average Score

Studies were designed to evaluate the efficacy of oral rifaximin at 550 mg TID in providing adequate relief from IBS symptoms over three months. A measure of efficacy is based on subjects' answers to the Weekly Subject Global Assessment (SGA) questions over the study duration in relation to their IBS symptoms.

An analysis was performed to determine the patients that had a decrease in IBS-related abdominal pain and discomfort as a function of time. Additionally subjects having a stool consistency score of <4 and at least a 1 point reduction in average daily IBS score were identified.

Results are presented in Tables 30-33 and depict the subjects having Stool Consistency scores of <4, at least a 30% decrease in abdominal pain and IBS Symptoms score decreased by at least 1.

Accordingly, described is a method of treating Irritable Bowel Syndrome (IBS), wherein the method includes administering 550 mg of rifaximin TID to a subject in need thereof, wherein there is at least a 25% decrease in IBS-related abdominal pain and a stool consistency score of <4, thereby treating IBS. In some embodiments, administration of 550 mg rifaximin TID results in at least a 1 point decrease in average daily IBS score. In some embodiments, administration of 550 mg rifaximin TID results in a 30% decrease in IBS-related abdominal pain. In some embodiments, administration of 550 mg rifaximin TID results in a 35% decrease in IBS-related abdominal pain.

In some aspects, the IBS is diarrhea-predominant IBS. In some embodiments, the IBS is alternating-predominant IBS.

In some aspects, the subject is administered rifaximin for between about 14 days and about 24 months.

In some aspects, baseline symptoms are established prior to treatment.

In some aspects, the subject being treated is white.

In some aspects, the at least 25% decrease in IBS-related abdominal pain and a stool consistency score of <4 is at a time point of 1 month after the treatment with rifaximin.

In some aspects, the at least 25% decrease in IBS-related abdominal pain and a stool consistency score of <4 is at a time point of 2 months after the treatment.

In some aspects, the at least 25% decrease in IBS-related abdominal pain and a stool consistency score of <4 is at a time point of 3 months after the treatment.

In some aspects, the method further includes determining the gender of a subject and administering the therapeutically effective amount of rifaximin to a female subject.

In some aspects, the method includes administering 550 mg of rifaximin TID to the subject for 14 days.

In some aspects, administration of 550 mg rifaximin TID results in at least 25% of subjects treated with rifaximin having at least a 30% decrease in IBS-related pain, a stool consistency score of <4 and at least a 1 point decrease in average daily IBS score.

In some aspects, administration of 550 mg rifaximin TID results in at least 30% of subjects treated with rifaximin having at least a 30% decrease in IBS-related pain, a stool consistency score of <4 and at least a 1 point decrease in average daily IBS score.

In some aspects, administration of 550 mg rifaximin TID results in at least 35% of subjects treated with rifaximin having at least a 30% decrease in IBS-related pain, a stool consistency score of <4 and at least a 1 point decrease in average daily IBS score.

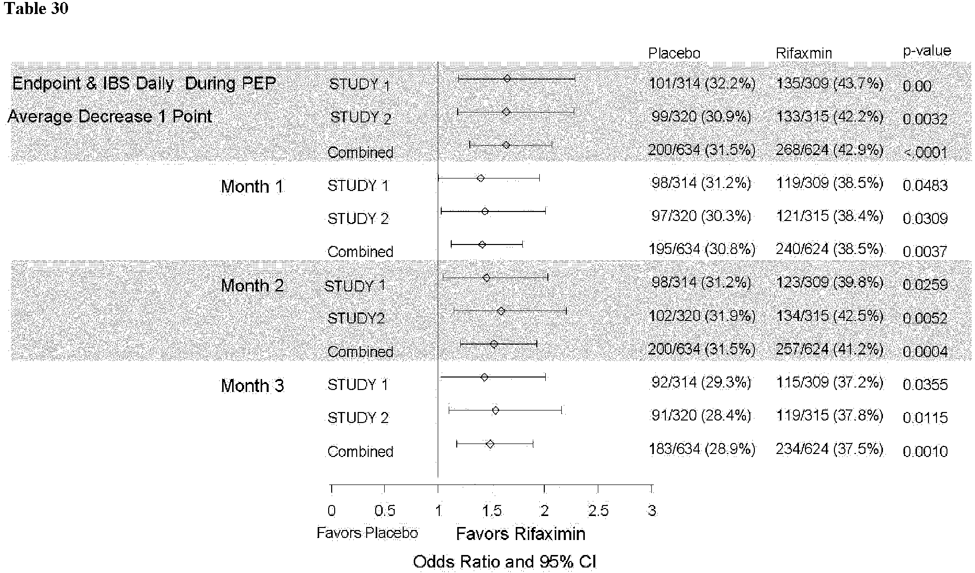

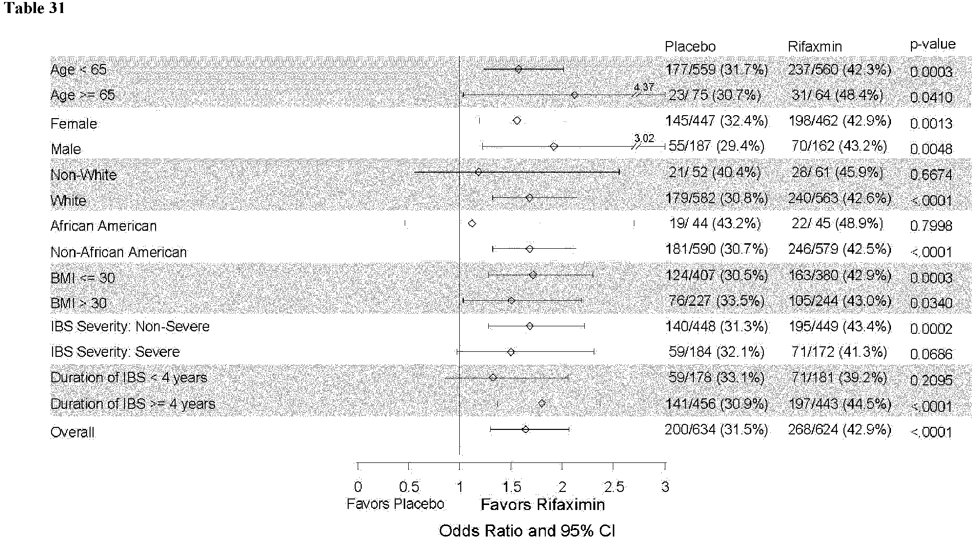

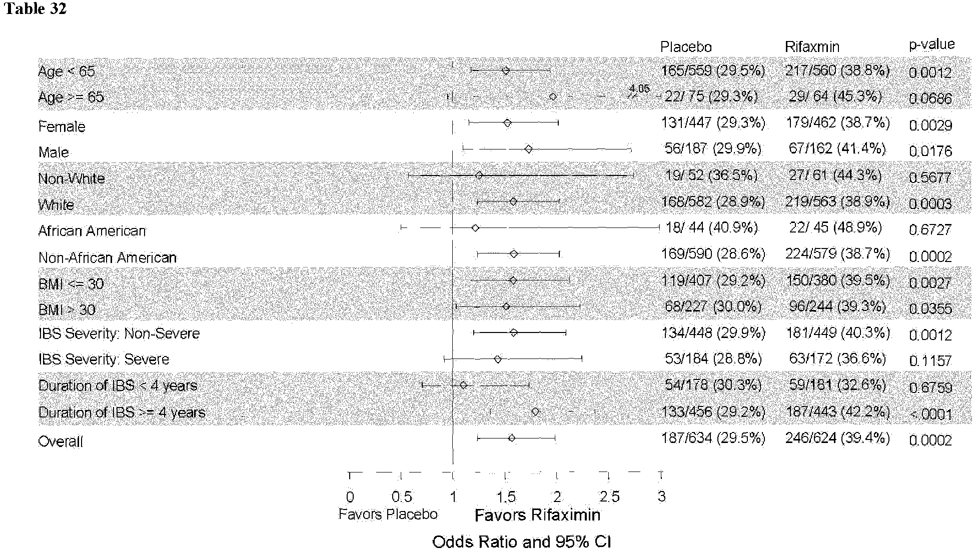

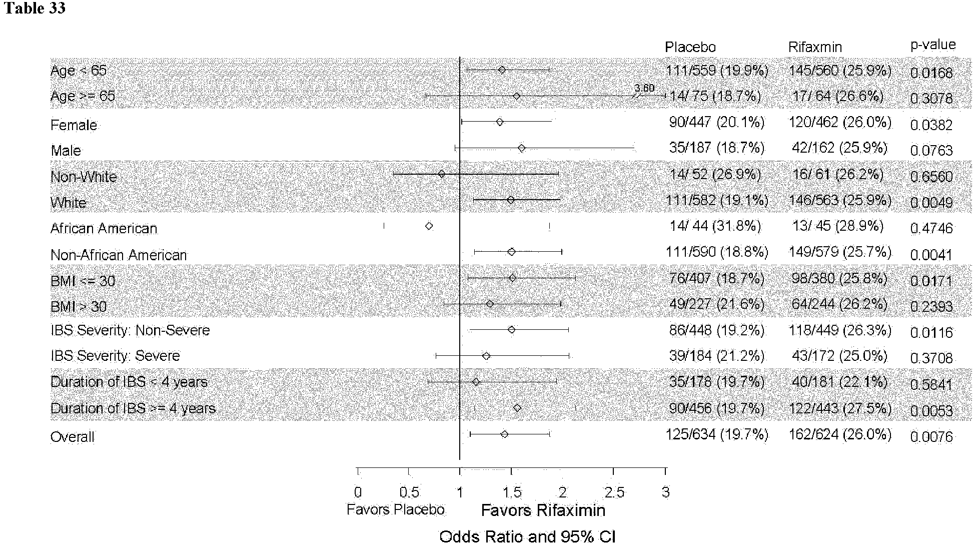

### EXAMPLE 8.

### Re-treatment of IBS-D in patients with rifaximin

The inventors developed a repeat treatment method. The data in Table 34 (below) were taken into account by the inventors when developing the novel and inventive repeat treatment methods described herein. The study is a multi-center, randomized, double-blind, placebo-controlled trial in adult subjects with non-C IBS confirmed using Rome III diagnostic criteria. The primary study objective is to evaluate the efficacy of repeat treatment with rifaximin 550 mg TID in subjects who responded to initial treatment with rifaximin. An exemplary study design is illustrated in Figure 14.

**Table 34. Existing Data for Repeat Rifaximin Use in IBS**

| **Study (Duration) Population** | **RFX Dose and Duration** | **Number of Re-treatments** | **Results** |
|---|---|---|---|
| Pimentel, et al. (> 6 yr) | • 400-550 mg TID for 14 days | 1 to 6 re-treatments | - Initial treatment response: 75% (111/148) |
| 169 Non-C IBS Patients (Rome III) | | | |
| | | | - Re-treatment response (at least 1): > 75% |
| | | | - First: 54/65 Second 38/40: Third: 17/18 |
| | | | - Duration of benefit is ~4 m |
| Weinstock (> 6 yr) | • 1200-1650 mg/day for 10 days | 1 to 5 re-treatments | - Initial treatment response: 75% (74/99) |
| 99 Non-C IBS Patients (Rome II) | | | - 27% did not require re-treatment |
| | | | - 41% maintained response for mean 1.6 y |
| | | | - 51% only 1-2 retreatment in 2 y |
| Jolley (∼1 yr) | • 1200 mg/day for 10 days | 2400 mg/day for 10 days (if no response in 2-4 weeks) | For IBS -D patients: |
| 162 IBS Patients (Rome III; 28% IBS-D) | | | - Initial treatment response: 56% (25/45) |
| | | | - Re-treatment response (at least 1): 54% (13/24) |
| | | | - Complete(>90%) relief: 11% (5/45) |
| | | | - Complete (>90%) relief upon re-treatment: 13% (3/24) |
| Yang, et al (1.25 yr) | • 1200 mg/day for 10 days | 1200 mg/day for 10 days | - Initial treatment response: 69% (58/84) |
| 84 IBS Patients (Rome I) | | | - Re-treatment response (at least 1): 100% |
| | | | - First: 16/16 Second 4/4 |
| | | | - Initial response to antibiotic other than rifaximin: 38% (27/61) |
| | | | - Retreatment response to antibiotic other than rifaximin: 25% (2/8) |

| | | | |
|---|---|---|---|
| Abbreviations: IBS = irritable bowel syndrome; non-C IBS = non-constipation IBS; IBS-D = diarrhea-predominant IBS; RFX = rifaximin; and TID = 3 times daily. | | | |

This study consists of several treatment phases, and is initiated with a placebo run-in treatment during the Screening/Treatment 1 Phase. The placebo run-in is included in the design to disqualify subjects from enrollment if they no longer meet IBS symptom related entry criteria at the end of the Screening/Treatment 1 Phase (e.g., spontaneous improvement). Patients meeting entry criteria are enrolled into an open-label Treatment 2 Phase where all subject will receive rifaximin 550 mg TID for 2 weeks, and will be followed though a 4 week treatment free follow-up period. Subjects who achieve treatment success in both IBS-related abdominal pain and stool consistency during at least two of the four week follow-up period are classified as responders and enter a treatment free Maintenance Phase 1. Non-responders are withdrawn from the study to provide an enriched population of subjects who respond to treatment with rifaximin. The treatment free Maintenance Phase 1 is variable in time (up to 18 weeks in total) and depends upon recurrence (e.g., absence of treatment success in both IBS related abdominal pain or stool consistency).

The subjects remain blinded to placebo received during Treatment 1. Subjects with recurrence enter the DBR Treatment 3 Phase. Subjects are randomized 1:1 to receive either rifaximin 550 mg TID or placebo TID for 2 weeks with a 4-week treatment-free follow-up, then enter a second treatment free phase for up to 6 weeks (Maintenance Phase 2).

All subjects from Maintenance Phase 2 enter a SRT Treatment 4 Phase where they receive the same treatment as previously assigned (rifaximin 550 mg TID or placebo TID for 2 weeks with a 4-week treatment-free follow-up).

Figure 14 sets forth a specific Repeat Treatment Study.

A "responder" to treatment is defined as a subject who demonstrates at least 2 weeks of improvement in a 4 week treatment free follow up period in both primary symptoms of IBS (e.g., abdominal pain and stool consistency).

Subjects are considered to have met recurrence criteria or have "relapsed" when they experience the recurrence of abdominal pain OR stool consistency for at least 3 weeks during a 4-week follow-up period. Similar "relapse" rates were noted in subjects who met the responder definition during the PEP for previous studies for both definitions of "relapse": e.g., when relapse is defined as an absence of treatment success for abdominal pain for at least three out of four consecutive weeks; or as a loss of stool consistency for at least three out of four consecutive weeks. Therefore a recurrence of both symptoms is not required to define a "relapse".

Treatment 1 Phase: Screening Phase - During Treatment 1, subjects receive single-blind placebo TID for up to 13 days and are required to respond to daily IBS symptom related questions for at least 7 days. Potential subjects may also undergo a colonoscopy, if necessary. This placebo run-in is included in the trial in order to disqualify subjects who experience spontaneous improvement and to decrease the high placebo response typically seen in IBS trials. Periodic safety monitoring is performed during each clinic visit.

Treatment 2 Phase: Initial Treatment Phase - Eligible subjects receive a two-week course of rifaximin 550 mg TID, with four weeks of treatment-free follow-up. At the end of this initial treatment and follow-up phase, subjects are assessed for response. Subjects who are responders enter a treatment- free maintenance phase (*i.e.*, Maintenance Phase 1) whereas non-responders are withdrawn from the study.

Maintenance Phase 1 - This phase is variable in duration for subjects, depending on whether or not there is a recurrence of IBS symptoms. Subjects are continually assessed for ongoing response as well as recurrence of IBS symptoms starting after 2 weeks in Maintenance Phase 1. Subjects who meet the criteria for recurrence enter the Double-Blind, Randomized (first repeat) Treatment Phase. Subjects who do not meet recurrence criteria by the end of Maintenance Phase 1 are allowed to continue up to an additional 12 weeks until they either experience recurrence; or until enrollment is met in the Double-Blind, Randomized (first repeat) Treatment Phase.

Treatment 3 Phase: Double-Blind, Randomized (first repeat) Treatment Phase and Interim Analysis - In this phase, subjects who experienced recurrence during Maintenance Phase 1 are randomized 1:1 to receive rifaximin 550 mg TID or placebo TID for 2 weeks with a four-week treatment-free follow-up. Primary efficacy analysis is performed at the end of the Treatment 3 (DBR Treatment) Phase.

Maintenance Phase 2 - Responders in the Double-Blind, Randomized (first repeat) Treatment Phase are eligible for Maintenance Phase 2 and continue with an additional treatment-free follow-up period of up to 8 weeks. Subjects who experience recurrence are immediately transitioned into the Second Repeat Treatment Phase. Subjects who do not meet recurrence criteria by the end of an 8-week Maintenance Phase 2 are withdrawn from the study.

Treatment 4 Phase: Second Repeat Treatment Phase and End of Study - Subjects with recurrence in Maintenance Phase 2 are eligible to enter the Second Repeat Treatment Phase, and receive a second repeat treatment of rifaximin 550 mg TID or placebo TID for 2 weeks with a four-week treatment-free follow up. The treatment assignment from the Double-Blind, Randomized (first repeat) Treatment Phase is maintained in this phase (*i.e*. subjects entering the Treatment 4 Phase will continue to receive the same treatment as in the Treatment 3). At the end of this phase, subjects undergo end of study assessments.

Patients selected for inclusion meet the Rome III diagnostic criteria for IBS-D. The Rome III criteria are the accepted current standard for diagnosing IBS in the clinical setting and are consistent with FDA guidance. Table 4 outlines the criteria for diagnosing and subtyping IBS using Rome III.

Additionally, during the diary eligibility period, the following average daily symptom scores for IBS are required in all categories for entry into the proposed study designs:
- An average score ≥ 3 for abdominal pain (Scale: 0-10, with 0 indicating no pain, and 10 indicating the worst imaginable pain).
- An average score ≥ 3 for bloating (Scale: 0-6, ranking how bothersome IBS-related bloating was in the last 24 hours, 0 = not at all; 1 = hardly; 2 = somewhat; 3 = moderately; 4 = a good deal; 5 = a great deal; 6 = a very great deal.")
- A score of 6 or greater for stool consistency using the Bristol Stool form Scale for at least 2 out of 7 days (Note: Subjects will not be eligible for the study if they experience hard or lumpy stools [Bristol Scale Type 1 or 2, consistent with constipation], during the eligibility period.)

Exclusion criteria include the following: a patient history consistent with constipation-predominant IBS; a patient history of inflammatory bowel disease (IBD), diabetes, unstable thyroid disease, previous abdominal surgery, HIV, renal or hepatic disease; and/or current use of at least one of the following medicine/medications: alosetron, tegaserod, lubiprostone, an antipsychotic medicine, an antispasmodic medicine, an antidepressant (except stable dose TCA or SSRI), warfarin, antidiarrheals, probiotics, narcotics, an antibiotic within the previous 14 days, and/or rifaximin within the previous 60 days.

Subjects record IBS symptoms in an IVRS during screening to confirm eligibility and will have had a colonoscopy within the last 2 years to rule out inflammatory bowel diseases or other causes of IBS symptoms. Other confounding medical conditions and medications are excluded by qualified healthcare professionals.

### Endpoints

The objectives of the study are: (1) to evaluate the efficacy of repeat treatment with rifaximin 550mg TID in subjects with IBS-D who responded to initial treatment with rifaximin 550 mg TID, and (2) to evaluate the safety of rifaximin 550 TID in subjects with IBS-D.

The Primary Endpoint is the proportion of subjects who are responders to repeat treatment in both IBS-related abdominal pain AND stool consistency during the 4 week treatment-free follow-up (or Primary Evaluation Period [PEP]) in the Double Blind Repeat (DBR) Treatment Phase.

The Secondary Endpoints include:
- the proportion of subjects who are responders to repeat treatment in both IBS-related abdominal pain and/or stool consistency with at least 1 point improvement in weekly average daily IBS symptoms compared to baseline during PEP in the DBR Treatment Phase;
- the proportion of subjects who are responders to repeat treatment in bloating during PEP in the DBR Treatment Phase;
- The proportion of subjects who are responders during PEP in the DBR Treatment Phase based on:
   - IBS-related abdominal pain
   - Stool consistency
   - IBS symptoms
   - Urgency
- Time to recurrence during the Treatment 2 Phase and through the follow-up Maintenance Phase 1 for the following:
   - IBS-related abdominal pain OR stool consistency
   - IBS-related abdominal pain
   - Stool consistency
- Time to recurrence during the DBR Treatment Phase and through the follow-up Maintenance Phase 2 for the following:
   - IBS-related abdominal pain OR stool consistency
   - IBS-related abdominal pain
   - Stool consistency
- Change from baseline to each week across all study phases for the following:
   - Abdominal pain
   - Stool consistency
   - Bloating
   - IBS symptoms
   - Urgency
- Change from baseline in quality of life based on the IBS quality of life (IBS-QOL) questionnaire
- Proportion of responders on rifaximin during PEP after the DBR Treatment Phase versus their response profile (yes/no) during the 4-week treatment free follow-up period in the Second Repeat Treatment (SRT) Phase.
- The proportion of subjects who are responders during the 4-week treatment free follow-up period in the SRT Phase based on:
   - IBS-related abdominal pain
   - Stool consistency
   - IBS symptoms
   - Urgency
- Total number of Type 1 = Separate hard lumps like nuts (hard to pass) or Type 2 = Sausage shaped but lumpy stools based on BSS (Bristol Stool -form Scale) stools by week

### Efficacy Endpoint Definitions

### Treatment Success

Weekly response for the primary endpoint is defined based on IBS-symptom related questions, as follows:
- Weekly treatment success in IBS-related abdominal pain is defined as a 30% or greater improvement from baseline in the weekly average abdominal pain score, based on subject response to the following daily question:
   "In regards to your specific IBS symptom of abdominal pain, on a scale of 0-10, what was your worst IBS-related abdominal pain in the last 24 hours? 'Zero' means you have no pain at all; `Ten' means the worst possible pain you can imagine."
- Weekly treatment success in stool consistency is achieved when a subject has 50% reduction in the number of stools scored at ≥ 6 over 7 days as compared to baseline based on subject response to the following daily question based on the Bristol Stool Form Scale:
   "On a scale of 1-7, what was the overall form of your bowel movements in the last 24 hours? 1 = Separate hard lumps, like nuts (hard to pass); 2 = Sausage-shaped but lumpy; 3 = Like a sausage but with cracks on its surface; 4 = Like a sausage or snake, smooth and soft; 5 = Soft blobs with clear cut edges (passed easily); 6 = Fluffy pieces with ragged edges, a mushy stool; 7 = Watery stool, no solid pieces; entirely liquid."

Responders are patients who, upon administration of rifaximin, experience (1) a decrease in the weekly average score of "worst pain in the last 24 hours" of ≥ 30% compared with baseline levels and (2) a ≥ 50% reduction in the number of days per week with at least one stool having a consistency of ≥ type 6 Bristol Stool Form Scale compared with baseline.

Weekly treatment success for IBS-related bloating is assessed using the following question: "In regards to your specific IBS symptom of bloating, on a scale of 0-6, how bothersome was your IBS-related bloating in the last 24 hours? 0 = not at all; 1 = hardly; 2 = somewhat; 3 = moderately; 4 = a good deal; 5 = a great deal; 6 = a very great deal." Treatment success for bloating is achieved when a subject rates his/her daily IBS-related bloating as either: 0 (not at all) or 1(hardly) at least 50% of the days in a given week; OR 0 (not at all), 1 (hardly) or 2 (somewhat) 100% of the days in a given week

Weekly treatment success for IBS symptoms (daily reported) is assessed using the following question: "In regards to all of your symptoms of IBS, on a scale of 0-6, how bothersome were your symptoms of IBS in the last 24 hours? 0 = not at all; 1 = hardly; 2 = somewhat; 3 = moderately; 4 = a good deal; 5 = a great deal; 6 = a very great deal." Treatment success for IBS symptoms is achieved when a subject rates his/her daily IBS symptoms as either: 0 (not at all) or 1(hardly) at least 50% of the days in a given week; OR 0 (not at all), 1 (hardly) or 2 (somewhat) 100% of the days in a given week.

Weekly treatment success in urgency is defined as a 30% or greater improvement from baseline in the percentage of days with urgency, based on subject response to the following daily question: "Have you felt or experienced a sense of urgency today with any of your bowel movements? (Yes/No)"

Patients are responders in a given month if they have a positive response during ≥ 2 out of 4 weeks. Patients will be considered to have a recurrence criteria when treatment success of abdominal pain or stool consistency is absent for at least three weeks out of a 4-week assessment period.

Planned Exploratory Endpoints for the study include the following:
- Descriptive characterization of the proportion of responders (yes/no) on rifaximin after the Double-Blind, Randomized (first repeat) Treatment Phase versus their response profile (yes/no) in the Second Repeat Treatment Phase.
- Biomarker assessments

Safety Endpoints will include monitoring and assessment of AEs, clinical laboratory parameters, vital signs, and physical examinations.

### Analysis Populations and Efficacy Endpoints

Three analysis populations are planned for efficacy assessments:
- The ITT population will include all randomized subjects who ingested at least one dose of the study drug.
- The Double-Blind, Randomized (first repeat) Treatment population will include subjects who responded to the initial treatment and who were randomized and received at least one dose of the study drug in the First Re-treatment Phase. This will serve as the primary analysis population.
- The Second Repeat Treatment population will include subjects who responded to the initial repeat treatment and received at least one dose of the study drug in the Second Re-treatment Phase.

The primary efficacy analysis will be conducted on the Double-Blind, Randomized (first repeat) Treatment population and will be conducted at the end of the Double-Blind, Randomized (first repeat) Treatment Phase. The analysis will utilize Cochran-Mantel-Haenszel method adjusting for analysis center (PROC FREQ in SAS/STAT). Weekly response will be set to non-response when the subject completes <4 diary days.

### Treatment of Subjects

### Formulation and Supply

Study drug is supplied as tablets containing either rifaximin or matching placebo. Each rifaximin tablet contains 550 mg rifaximin, and the following inactive ingredients: colloidal silicon dioxide, disodium edetate, glycerol palmitostearate, hypromellose, microcrystalline cellulose, propylene glycol, red iron oxide, sodium starch glycolate, talc, and titanium dioxide. Matching placebo is supplied.

### Dosing and Dosing Schedule

Each subject is provided blinded study drug. All subjects receive:
- Placebo TID for up to 13 days during Treatment 1, and
- Rifaximin 550 mg TID for 2 weeks with a 4-week follow-up.

Subjects who continue to the DBR Treatment Phase are randomized 1:1 to the following arms:
- Rifaximin 550 mg TID for 2 weeks with a 4-week follow-up, or
- Placebo TID for 2 weeks with a 4-week follow-up.

During the DBR Treatment/Treatment 3 and SRT/Treatment 4 Phases each subject is provided study drug at the DBR Treatment Day 1 and SRT Day 1. Subjects entering the SRT Phase continue to receive the same treatment as previously assigned during the DBR Treatment Phase.

### Treatment 2 Day 1/Baseline

The following are to be completed for subjects who have met all eligibility criteria to participate in the study:
- Assess responses to the average daily IBS symptom questions.

The following average daily scores for IBS symptoms are required for entry into the study: (1) an average score of greater than or equal to 3 for abdominal pain, (2) an average score of greater than or equal to 3 for bloating, and (3) at least 2 days in the last week with stool consistency of Type 6 (Fluffy pieces with ragged edges, a mushy stool) or Type 7 (Watery stool, no solid pieces; entirely liquid), using the BSS.
- Collection of a stool sample to identify the presence of enteric infections (e.g. *Yersinia enterocolitica, Campylobacter jejuni, Salmonella, Shigella,* ovum and parasite and/or *Clostridium difficile) may also be done as well as* administration of the IBS-QOL.

### Maintenance Phase I

Maintenance Phase 1 consists of phone calls to assess for non-responders. Assessment of responses to the average daily IBS symptom questions will be done. If the subject is having recurrence, the subject should be scheduled for the Treatment 3.

### Treatment 3 Phase

The following are to be completed:
- Assessment of responses to the average daily IBS symptom questions; collection of stool samples; performance of symptom-directed physical examination; and administration of IBS-QOL.

### End of Treatment 3 Phase

The following assessments are completed: (1) collection of stool samples, and (2) assessment of responses to the daily IBS symptom questions.

### Maintenance Phase 2

Maintenance Phase 2 comprises follow-up and assessment for relapse and administration of the IBS-QOL.

### Treatment 4 Phase

The following are completed: (1) an assessment of compliance and responses to the average daily IBS symptom questions, and (2) administration of IBS-QOL.

### End of Treatment 4 Phase

The following assessments are completed: (1) performance of symptom directed physical examination, and (2) assessment of responses to the daily IBS symptom questions.

### End of Study Follow-up Phase

The EOS Visit consists of the following: perform physical examination; administration of IBS-QOL and collection of stool sample.

### Efficacy Assessments

Daily IBS symptoms are collected and analyzed for efficacy assessments.

IBS daily questions include:
- How many bowel movements did you have in the last 24 hours?
- On a scale of 1-7, what was the score of your least formed bowel movement in the last 24 hours?
   - 1 = Separate hard lumps, like nuts (hard to pass)
   - 2 = Sausage-shaped but lumpy
   - 3 = Like a sausage but with cracks on its surface
   - 4 = Like a sausage or snake, smooth and soft
   - 5 = Soft blobs with clear cut edges (passed easily)
   - 6 = Fluffy pieces with ragged edges, a mushy stool
   - 7 = Watery stool, no solid pieces; entirely liquid.
- Have you felt or experienced a sense of urgency in the last 24 hours with any of your bowel movements? Yes/No
- In regards to your specific IBS symptom of abdominal pain, on a scale of 0-10, what was your worst IBS-related abdominal pain over the last 24 hours? 'Zero' means you have no pain at all; 'Ten' means the worst possible pain you can imagine. In regards to your specific IBS symptom of bloating, on a scale of 0-6, how bothersome was your IBS-related bloating in the last 24 hours?
   - 0 = not at all 4 = a good deal
   - 1 = hardly 5 = a great deal
   - 2 = somewhat 6 = a very great deal
   - 3 = moderately
- In regards to all your symptoms of IBS, on a scale of 0-6, how bothersome were your symptoms of IBS in the last 24 hours?
   - 0 = not at all 4 = a good deal
   - 1 = hardly 5 = a great deal
   - 2 = somewhat 6 = a very great deal
   - 3 = moderately

Accordingly, described herein is a randomized, double-blind, placebo-controlled study to be conducted in approximately 250 sites throughout the United States. The Primary Endpoint is the proportion of subjects who are responders to repeat treatment in both IBS-related abdominal pain AND stool consistency during the 4 week treatment-free follow-up (Primary Evaluation Period, or PEP) in the Double Blind Repeat (or DBR) Treatment Phase. The Key Secondary Endpoints are 1) proportion of subjects who are responders to repeat treatment in both IBS-related abdominal pain AND stool consistency with at least 1 point improvement in weekly average daily IBS symptoms compared to baseline during PEP in the DBR Treatment Phase and 2) proportion of subjects who are responders to repeat treatment in bloating during PEP in the DBR Treatment Phase.

### EXAMPLE 9 (reference example)

### Treatment Effect for Sustained Response from Weeks 7 through 12 Recurrence of Symptoms after Primary Evaluation Period

An evaluation of the enrolled subjects was carried out after the Primary Evaluation Period in the study described in Examples 5-7. Subjects administered rifaximin for treatment of IBS had a sustained response. Specifically, for any four week window of the trial, a subject had a sustained response. Subjects with no recurrence are defined as having a stool consistency score of less than 4, abdominal pain reduced by at least 30 percent or both. The results are set forth in Tables 35 and 36.

**Table 35**

| | | **Placebo (N = 634)** | | **Rifaximin (N = 624)** | |
|---|---|---|---|---|---|
| **Endpoint** | **Rolling 4 Weeks** | **No Recurrence** | **Recurrence** | **No Recurrence** | **Recurrence** |
| **Stool Consistency** | 4 - 7 Weeks | 432 (68.1%) | 8 (1.3%) | 492 (78.8%) | 8 (1.3%) |
| | 5 - 8 Weeks | 424 (66.9%) | 13 (2.1%) | 484 (77.6%) | 13 (2.1%) |
| | 6 - 9 Weeks | 411 (64.8%) | 16 (2.5%) | 471 (75.5%) | 16 (2.6%) |
| | 7 - 10 Weeks | 395 (62.3%) | 9 (1.4%) | 455 (72.9%) | 9 (1.4%) |
| | 8 - 11 Weeks | 386 (60.9%) | 7 (1.1%) | 446 (71.5%) | 3 (0.5%) |
| | 9 - 12 Weeks | 379 (59.8%) | 8 (1.3%) | 443 (71.0%) | 13 (2.1%) |
| | Sustained Durable Response | 371 (58.5%) | | 430 (68.9%) | |
| **Abdominal Pain** | 4 - 7 Weeks | 270 (42.6%) | 22 (3.5%) | 324 (51.9%) | 24 (3.8%) |
| | 5 - 8 Weeks | 248 (39.1%) | 11 (1.7%) | 300 (48.1%) | 19 (3.0%) |
| | 6 - 9 Weeks | 237 (37.4%) | 7 (1.1%) | 281 (45.0%) | 15 (2.4%) |
| | 7 - 10 Weeks | 230 (36.3%) | 12 (1.9%) | 266 (42.6%) | 15 (2.4%) |
| | 8 - 11 Weeks | 218 (34.4%) | 11 (1.7%) | 251 (40.2%) | 14 (2.2%) |
| | 9 - 12 Weeks | 207 (32.6%) | 9 (1.4%) | 237 (38.0%) | 14 (2.2%) |
| | Sustained Durable Response | 198 (31.2%) | | 223 (35.7%) | |
| **Abdominal Pain & Stool Consistency** | 4 - 7 Weeks | 239 (37.7%) | 17 (2.7%) | 301 (48.2%) | 23 (3.7%) |
| | 5 - 8 Weeks | 222 (35.0%) | 10 (1.6%) | 278 (44.6%) | 20 (3.2%) |
| | 6 - 9 Weeks | 212 (33.4%) | 7 (1.1%) | 258 (41.3%) | 14 (2.2%) |
| | 7 - 10 Weeks | 205 (32.3%) | 14 (2.2%) | 244 (39.1%) | 11 (1.8%) |
| | 8 - 11 Weeks | 191 (30.1%) | 10 (1.6%) | 233 (37.3%) | 14 (2.2%) |
| | 9 - 12 Weeks | 181 (28.5%) | 9 (1.4%) | 219 (35.1%) | 12 (1.9%) |
| | Sustained Durable Response | 172 (27.1%) | | 207 (33.2%) | |

**Table 36. Results from First and Second Studies**

| | | **Rifaximin (N = 624)** | |
|---|---|---|---|
| **Endpoint** | **Rolling 4 Weeks** | **No Recurrence** | **Recurrence** |
| **Abdominal Pain or Stool Consistency** | 2 - 5 Weeks | 286 (45.8%) | 12 (1.9%) |
| | 3 - 6 Weeks | 274 (43.9%) | 23 (3.7%) |
| | 4 - 7 Weeks | 251 (40.2%) | 17 (2.7%) |
| | 5 - 8 Weeks | 234 (37.5%) | 16 (2.6%) |
| | 6 - 9 Weeks | 218 (34.9%) | 10 (1.6%) |
| | 7 - 10 Weeks | 208 (33.3%) | 10 (1.6%) |
| | 8 - 11 Weeks | 198 (31.7%) | 8 (1.3%) |
| | 9 - 12 Weeks | 190 (30.4%) | 10 (1.6%) |
| | Sustained Durable Response | 180 (28.8%) | |

### EXAMPLE 10. (reference example)

### Stability or Decline in Rates of Commonly-occurring Infections in Cirrhotic Patients Receiving Long-term Rifaximin Treatment

Cirrhosis patients can have an increased risk of infections and subsequent hospitalizations, leading to increased mortality. Long-term treatment with rifaximin 550 mg BID (RFX) was observed to demonstrate continued protection against hepatic encephalopathy (HE) and to provide a reduced risk of hospitalizations in cirrhotic patients in an open-label maintenance trial (OLM), following a randomized, double-blind, placebo-controlled trial (RCT). A description of the OLM and RCT can be found in WO 2011/005388, "METHODS OF TREATING HEPATIC ENCEPHALOPATHY". In the analysis below, the effect of long term RFX treatment on infection rates and antibiotic use was examined.

Patients with cirrhosis and ≥ 2 overt HE episodes within 12 months were enrolled in the RCT (RFX =140; placebo [PBO] = 159); 170 new patients, in addition to 70 RFX and 82 PBO patients who rolled over from the RCT were enrolled in OLM. The "All RFX group" (n=392) consisted of RFX-treated patients in both studies. Infection rates per person exposure years (PEY) were compared across RCT and All RFX groups, and antibiotic use over time was examined.

In the 6 month RCT, RFX exposure was 50 PEY vs 46 PEY in the PBO group. Long term RFX exposure was for median = 427 (2-1427) days, or 510 PEY. The overall infection rate was found to be lower in patients using RFX long term compared to both, the PBO and RFX RCT groups. The rates of commonly-occurring infections in cirrhotic patients were observed to decline or remain stable in the long term (Table 37). Overall, use of antibiotics (oral and intravenous) remained the same or declined with time.

**Table 37: Area Under the Curve and Time-Weighted Average for Venous Ammonia Concentrations (ITT Population)**

| | RCT Patients | | All RFX Patients |
|---|---|---|---|
| Term, N (rate*) | PBO (n=159) | RFX (n=140) | (n=392) |
| | PEY=46 | PEY=50 | PEY=510 |
| Any infection | 49 (0.132) | 46 (0.112) | 214 (0.072) |
| Cellulitis | 3 (0.066) | 3 (0.006) | 34 (0.071) |
| *C. difficile* infection | 0 | 2 (0.040) | 6 (0.012) |
| Peritonitis | 6 (0.131) | 3 (0.060) | 22 (0.044) |
| Pneumonia | 1 (0.022) | 4 (0.080) | 42 (0.084) |
| Sepsis / septic shock | 5 (0.109) | 2 (0.040) | 31 (0.062) |
| Urinary tract/kidney | 14 (0.320) | 9 (0.187) | 83 (0.193) |

| | | | |
|---|---|---|---|
| * Rate is calculated as number of subjects /PEY. | | | |

The results illustrate that long-term treatment with rifaximin 550 mg BID did not adversely affect infection rates or increase antibiotics use in cirrhotic patients with HE.

### EXAMPLE 11. (reference example)

### Treatment of C. difficile infections

A double-blind, randomized, 10-day treatment of Rifaximin 400 mg TID vs. Vancomycin 125 mg QID, non-inferiority trial for treatment of *C*. *difficile* was conducted.

### Enrollment Criteria

Criteria for entry into the trial were:
- Age >=18 years old;
- Had acute diarrhea at screening defined as >=3 unformed stools in the last 24 hours and at least one sign of enteric infection (fever, nausea, lose of appetite, vomiting, severe abdominal pain/discomfort); and
- a positive *C. diff.* stool toxin assay

### Endpoint:

The primary efficacy endpoint of the study was defined as the proportion of subjects achieving clinical success. Specifically, clinical success was defined as the absence of severe abdominal pain at Test of Cure (TOC), absence of fever (< 38°C) at TOC, and < 3 unformed stools for two consecutive days at TOC.

The non-inferiority margin for the primary endpoint was defined as lower bound of 95% CI of delta above -15%.

The study enrolled 238 subjects, half of which were administered rifaximin (400 mg TID) and half of which were administered vancomycin (125 mg QID). Table 38 sets forth the demographic statistics of the enrolled subjects.

**Table 38**

| | | **Rifaximin (N=117)** | **Vancomycin (N=115)** | **Total (N=232)** |
|---|---|---|---|---|
| Age (Mean, SD) | | 58.9 (16.2) | 60.0 (18.1) | 59.5 (17.1) |

| Age Group | | | | |
|---|---|---|---|---|
| | <65 | 74 (63%) | 65 (57%) | 139 (60%) |
| | >=65 | 43 (37%) | 50 (43%) | 93 (40) |

| Gender | | | | |
|---|---|---|---|---|
| | Male | 43 (37%) | 48 (42%) | 91 (39%) |
| | Female | 74 (63%) | 67 (58%) | 141 (61%) |

| Race | | | | |
|---|---|---|---|---|
| | White | 103 (88%) | 102 (89%) | 205 (88%) |
| | Non-White | 14 (12%) | 13 (11%) | 27(12%) |

The baseline characteristics of the enrolled population are set forth in Table 39.

**Table 39**

| | | **Rifaximin (N=117)** | **Vancomycin (N=115)** | **Total (N=232)** |
|---|---|---|---|---|
| Fever | | | | |
| | Yes | 7 (6%) | 5 (4%) | 12 (5%) |
| | No | 110 (94%) | 110 (96%) | 220 (95%) |

| Pre-Treated w/ C Diff | | | | |
|---|---|---|---|---|
| | Yes | 23 (20%) | 25 (22%) | 48 (21%) |
| | No | 94 (80%) | 90 (78%) | 184 (79%) |

| Pre-Treated Medication | | | | |
|---|---|---|---|---|
| | Metronidazole | 22 (19%) | 22 (19%) | 44 (19%) |
| | Vancomycin | 2 (2%) | 3 (3%) | 5 (2%) |
| | Other | 3 (3%) | 0 | 3 (1%) |

As illustrated in Tables 38 and 39, demographics and baseline characteristics were comparable between groups; the majority of subjects (92.7%) had mild CDI.

The clinical success of CDI treatment with rifaximin is set forth in Table 40. Clinical success was defined as the: Absence of severe abdominal pain at TOC, absence of fever (< 38°C) at TOC, and <3 unformed stools for two consecutive days at TOC. Test of cure defined as Day 14+/- 1 day.

**Table 40**

| | | **Rifaximin (N=117)** | **Vancomycin (N=115)** | **Treatment Difference (95% CI)** |
|---|---|---|---|---|
| Clinical Success [1] | | | | |
| | Yes | 67 (57%) | 73 (64%) | -6.2% |
| | No | 50 (43%) | 42 (37%) | (-18.8%, 6.4%) |

Table 41 sets forth a summary of the enteric symptoms at TOC.

**Table 41**

| | | **Rifaximin (N=117)** | **Vancomycin (N=115)** | **Treatment Difference (95% CI)** |
|---|---|---|---|---|
| Severe Abdominal Pain/Discomfort | | N=108 | N=111 | -11.5% |
| | No | 80 (74%) | 95 (86%) | (-22.1%, -0.98%) |
| | Yes | 28 (26%) | 16 (14%) | |
| Fever | | N=108 | N=111 | -7.5% |
| | No | 98 (91%) | 109 (98%) | (-13.5%, -1.5%) |
| | Yes | 10 (9%) | 2 (2%) | |
| Diarrhea | | N=108 | N=111 | -1.5% |
| | No | 86 (80%) | 90 (81%) | (-12.0%, 9.1%) |
| | Yes | 22 (20%) | 21 (19%) | |

Tables 42 and 43 set forth a subgroup analysis of diarrhea at TOC.

**Table 42**

| | | | **Rifaximin** | **Vancomycin** | **Treatment Difference (95% CI)** |
|---|---|---|---|---|---|
| Age Group | | | | | |
| | <65 | | N=69 | N=62 | -3.8% |
| | | No Diarrhea | 53 (77%) | 50 (81%) | (-17.8%, 10.2%) |
| | | Diarrhea | 16 (23%) | 12 (19%) | |
| | >=65 | | N=39 | N=49 | 3.0% |
| | | No Diarrhea | 33 (85%) | 40 (82%) | (-12.7%, 18.7%) |
| | | Diarrhea | 6 (15%) | 9 (18%) | |

| Gender | | | | | |
|---|---|---|---|---|---|
| | Male | | N=40 | N=47 | 1.3% |
| | | No Diarrhea | 32 (80%) | 37 (79%) | (-15.8%, 18.3%) |
| | | Diarrhea | 8 (20%) | 10 (21%) | |
| | Female | | N=68 | N=64 | -3.4% |
| | | No Diarrhea | 54 (79%) | 53 (83%) | (-16.7%, 9.9%) |
| | | Diarrhea | 14 (21%) | 11 (17%) | |

**Table 43**

| | | | **Rifaximin** | **Vancomycin** | **Treatment Difference (95% CI)** |
|---|---|---|---|---|---|
| Age Group | | | | | |
| | <65 | | N=69 | N=62 | -3.8% |
| | | No Diarrhea | 53 (77%) | 50 (81%) | (-17.8%, 10.2%) |
| | | Diarrhea | 16 (23%) | 12 (19%) | |
| | >=65 | | N=39 | N=49 | 3.0% |
| | | No Diarrhea | 33 (85%) | 40 (82%) | (-12.7%, 18.7%) |
| | | Diarrhea | 6 (15%) | 9 (18%) | |

| Gender | | | | | |
|---|---|---|---|---|---|
| | Male | | N=40 | N=47 | 1.3% |
| | | No Diarrhea | 32 (80%) | 37 (79%) | (-15.8%, 18.3%) |
| | | Diarrhea | 8 (20%) | 10 (21%) | |
| | Female | | N=68 | N=64 | -3.4% |
| | | No Diarrhea | 54 (79%) | 53 (83%) | (-16.7%, 9.9%) |
| | | Diarrhea | 14 (21%) | 11 (17%) | |

Table 44 sets for the results of an analysis to determine the percent of subjects having a recurrence of CDI. Recurrence was defined to be a diarrhea and a positive *C*. *diff.* stool toxin assay that occurred after initial clinical success.

**Table 44**

| | **Rifaximin (N=67)** | **Vancomycin (N=73)** | **Treatment Difference (95% CI)** |
|---|---|---|---|
| No Recurrence | 61 (91%) | 63 (86%) | 4.7% |
| Recurrence | 6 (9%) | 10 (14%) | (-5.7%, 15.2%) |

Subjects having a global cure of diarrhea are set for the in Table 45. Global cure of diarrhea was defined as subjects who were cured of diahrrea at TOC without recurrence at follow up.

**Table 45**

| | | **Rifaximin (N=117)** | **Vancomycin (N=115)** | **Treatment Difference (95% CI)** |
|---|---|---|---|---|
| Global Cure of Diarrhea | | | | |
| | Yes | | | |
| | No | 83 (71%) | 85 (74%) | -3.0% |
| | | 34 (29%) | 30 (26%) | (-14.5%, 8.5%) |

Table 46 sets forth the recurrence rate of CDI per person-year.

**Table 46**

| | | **Rifaximin (N=117)** | **Vancomycin (N=115)** | **Treatment Difference (95% CI)** |
|---|---|---|---|---|
| Recurrence | | | | |
| | Yes | 6 (5%) | 10 (7%) | |
| | No | 111 (95%) | 105 (93%) | |
| Recurrence Rate per | | 0.613 | 0.912 | 0.67 (Ratio) |
| Person-Year Exposure | | | | (0.24, 1.86) |

Subjects having continuing illnesses are set forth in Table 47:

**Table 47**

| | | **Rifaximin (N=117)** | **Vancomycin (N=115)** | **Treatment Difference (95% CI)** |
|---|---|---|---|---|
| Continuing Illness [1] | | | | |
| | No | 90 (77%) | 99 (86%) | -9.2% |
| | Yes | 27 (23%) | 16 (14%) | (-19.1%, 0.8%) |

Table 48 sets forth the efficacy adjusting for concomitant medication usage.

**Table 48**

| | | **Rifaximin (N=117)** | **Vancomycin (N=115)** | **Treatment Difference (95% CI)** |
|---|---|---|---|---|
| Clinical Success | | | | |
| | Yes | 58 (50%) | 69 (60%) | -10.4% |
| | No | 59 (50%) | 46 (40%) | (-23.2%, 2.3%) |
| Diarrhea | | N=109 | N=112 | -8.1% |
| | No | 71 (65%) | 82 (73%) | (-20.2%, 4.1%) |
| | Yes | 38 (35%) | 30 (27%) | |

Table 49 sets forth the number of unformed stools during the treatment period.

**Table 49.**

| | **Rifaximin (N=117)** | **Vancomycin (N=115)** | **Treatment Difference (95% CI)** |
|---|---|---|---|
| | | | **Ratio (95% CI)** |
| Adjusted mean number of unformed stools - ANOVA [1] | 2.36 | 1.90 | 0.46 (-0.08, 1.00) |
| | | | 1.20 (0.90, 1.59) |
| Overall treatment difference in number of unformed stools - Mixed Model [2] | | | 0.32 (-0.08, 0.72) |
| | | | 1.22 (0.96, 1.56) |

| | | | |
|---|---|---|---|
| [1] ANOVA model included treatment and center as main effects. [2] The mixed effects model included treatment, center, and study day as fixed effects. | | | |

### Time to Last Unformed Stool Analysis

Figure 16 shows the number of days between the start of double-blind treatment and the last unformed stool prior to the achievement of clinical success. Subjects who completed the study without achieving clinical success were censored at Day 14

### Time to Resolution of Diarrhea Analysis

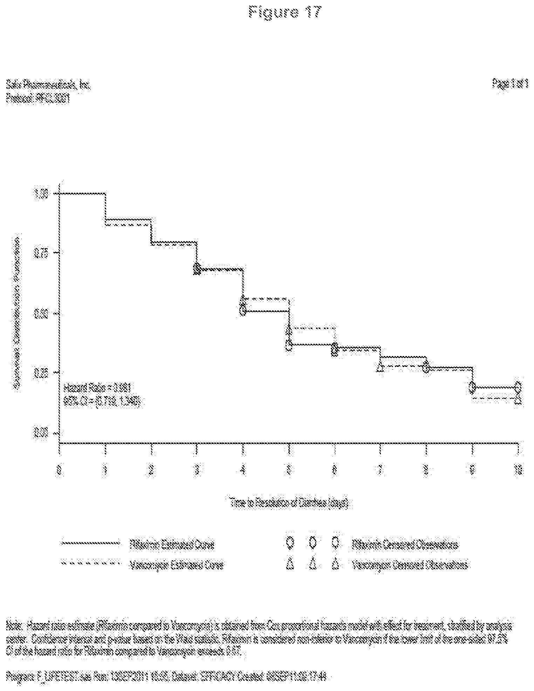

The number of days between the start of double-blind treatment and resolution of diarrhea that was defined as no unformed stools for at least 48 hours prior to Day 10 are shown in Figure 17.

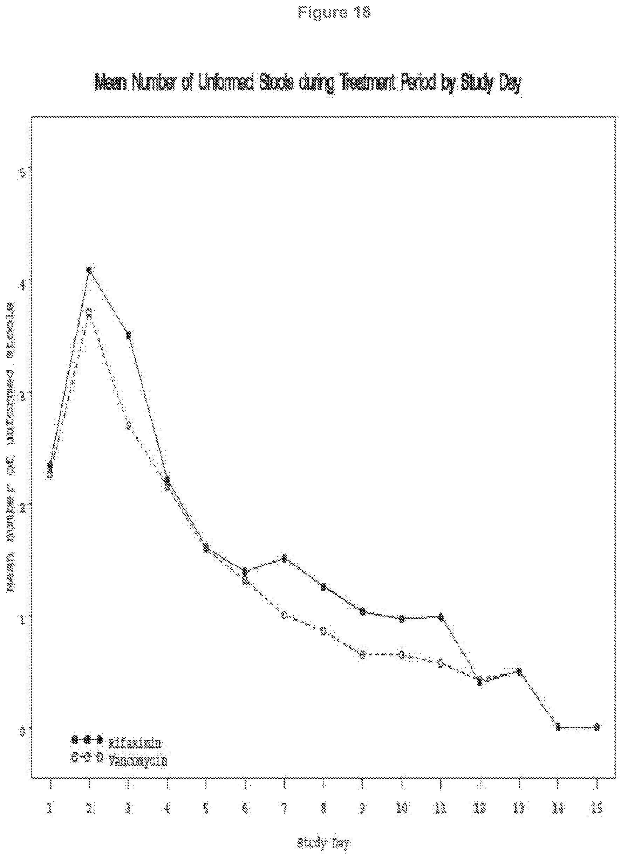

Figure 18 sets for the average number of unformed stools per day of the study.

The results of this study demonstrate that rifaximin is effective for treating CDI and CDI recurrence. Additionally, the results demonstrate that rifaximin is comparable to vancomycin for treating CDI. Supporting evidence for this effect was also observed in studies that were carried out to determine the safety and efficacy of administration of rifaximin to treat patients suffering from hepatic encephalopathy (HE). In the HE study, it was observed that the incidence of *C*. *difficile* infection was significantly lower in patients treated with rifaximin relative to patients treated with lactulose (p < 0.007).

## Claims

1. Rifaximin for use in retreating a subject having diarrhea-predominant IBS (d-IBS), being a responder to an initial treatment of d-IBS with rifaximin and having a recurrence of abdominal pain or stool consistency for at least 3 weeks during a 4-week assessment period, comprising
- an initial treatment phase comprising a 2 weeks administration of 550 mg of rifaximin three times daily (TID) and a 2 weeks of treatment-free follow-up,
- a first treatment-free maintenance phase of up to 8 to an additional 12 weeks,
- a first repeat treatment phase comprising a 2 weeks administration of 550 mg of rifaximin TID and a 2 weeks of treatment-free follow-up,
- a second treatment-free maintenance phase of up to 8 weeks,
- a second repeat treatment phase comprising a 2 weeks administration of 550 mg of rifaximin TID and a 2 weeks treatment-free follow-up.

2. Rifaximin for the use of claim 1, wherein the first and the second treatment-free maintenance phase each have a duration of 8 weeks.

3. Rifaximin for the use of any of claims 1 to 2, wherein IBS symptoms are
- abdominal pain with an average score ≥3 (Scale 0-10, of Rome III diagnostic Criteria for IBS-D),
- bloating with an average ≥3 (Scale 0-6 of Rome III diagnostic Criteria for IBS-D),
- stool consistency with a score ≥ 6 (Bristol Stool form scale).

4. Rifaximin for the use of any of claims 1 to 3, wherein the 550 mg of rifaximin is administered in tablet form for oral use.

## Patentansprüche

1. Rifaximin zur Verwendung bei der erneuten Behandlung eines Patienten mit diarrhöisch dominiertem IBS (d-IBS), der auf eine Erstbehandlung von d-IBS mit Rifaximin angesprochen hat, und bei dem während eines vierwöchigen Beurteilungszeitraums mindestens drei Wochen lang ein Wiederauftreten von Bauchschmerzen oder Stuhlkonsistenz auftrat, umfassend
- eine erste Behandlungsphase, die eine 2-wöchige Verabreichung von 550 mg Rifaximin dreimal täglich (TID) und eine 2-wöchige behandlungsfreie Nachbeobachtungsphase umfasst,
- eine erste behandlungsfreie Erhaltungsphase von bis zu 8 bis zu weiteren 12 Wochen,
- eine erste Wiederholungsbehandlungsphase, die eine zweiwöchige Verabreichung von 550 mg Rifaximin TID und eine zweiwöchige behandlungsfreie Nachbeobachtungsphase umfasst,
- eine zweite behandlungsfreie Erhaltungsphase von bis zu 8 Wochen,
- eine zweite Wiederholungsbehandlungsphase, die eine zweiwöchige Verabreichung von 550 mg Rifaximin TID und eine zweiwöchige behandlungsfreie Nachbeobachtungsphase umfasst.

2. Rifaximin zur Verwendung nach Anspruch 1, wobei die erste und die zweite behandlungsfreie Erhaltungsphase jeweils eine Dauer von 8 Wochen haben.

3. Rifaximin zur Verwendung nach einem der Ansprüche 1 bis 2, wobei die IBS-Symptome sind:
- Bauchschmerzen mit einem durchschnittlichen Score ≥3 (Skala 0-10, der Rom-III-Diagnosekriterien für IBS-D),
- Blähungen mit einem Durchschnitt ≥3 (Skala 0-6 der Rom-III-Diagnosekriterien für IBS-D),
- Stuhlkonsistenz mit einem Score ≥ 6 (Bristol Stool form scale).

4. Rifaximin zur Verwendung nach einem der Ansprüche 1 bis 3, wobei die 550 mg Rifaximin in Tablettenform zur oralen Anwendung verabreicht werden.

## Revendications

1. Rifaximine pour le traitement d'un sujet souffrant d'un syndrome de l'intestin irritable à prédominance de diarrhée (SII-D), ayant répondu à un traitement initial du SII-D par la rifaximine et présentant une récurrence des douleurs abdominales ou de la consistance des selles pendant au moins 3 semaines au cours d'une période d'évaluation de 4 semaines, et qui comprend
- une phase de traitement initial comprenant l'administration pendant 2 semaines de 550 mg de rifaximine trois fois par jour (TID) et un suivi de 2 semaines sans traitement,
- une première phase d'entretien sans traitement d'une durée de 8 à 12 semaines supplémentaires,
- une première phase de traitement répété comprenant une administration de 550 mg de rifaximine TID pendant 2 semaines et un suivi de 2 semaines sans traitement,
- une deuxième phase d'entretien sans traitement d'une durée maximale de 8 semaines,
- une deuxième phase de traitement répété comprenant une administration de 550 mg de rifaximine TID pendant 2 semaines et un suivi de 2 semaines sans traitement.

2. Rifaximine pour l'utilisation selon la revendication 1, dans laquelle la première et la deuxième phase d'entretien sans traitement ont chacune une durée de 8 semaines.

3. Rifaximine pour l'utilisation selon l'une des revendications 1 à 2, dans laquelle les symptômes du syndrome de l'intestin irritable sont les suivants
- douleur abdominale avec un score moyen ≥3 (échelle 0-10, des critères diagnostiques de Rome III pour le SII-D),
- ballonnements avec une moyenne ≥3 (échelle 0-6 des critères diagnostiques de Rome III pour le SII-D),
- consistance des selles avec un score ≥ 6 (échelle de Bristol pour forme de selles).

4. Rifaximine pour l'utilisation selon l'une des revendications 1 à 3, dans laquelle les 550 mg de rifaximine sont administrés sous forme de comprimés à usage oral.
